(19) Europäisches Patentamt
European Patent Office
Office européen des brevets

(11) Publication number: **0 540 074 A1**

# (12) EUROPEAN PATENT APPLICATION

(21) Application number: **92203037.4**

(22) Date of filing: **03.10.92**

(51) Int. Cl.5: **C12N 15/52**, C07K 7/06, C12P 21/04, //(C12N15/52, C12R1:125)

(30) Priority: **09.10.91 IT MI912683**
**02.09.92 IT MI922044**

(43) Date of publication of application:
**05.05.93 Bulletin 93/18**

(84) Designated Contracting States:
**AT BE CH DE DK ES FR GB GR IE LI LU MC NL PT SE**

(71) Applicant: **ENIRICERCHE S.p.A.**
**Corso Venezia 16**
**I-20121 Milan(IT)**

(72) Inventor: **Grandi, Guido**
**Nona Strada 4**
**I-20099 San Felice, Segrate, Milan(IT)**
Inventor: **Cosmina, Paola**
**Viale Misurata 14**
**I-20146 Milan(IT)**
Inventor: **Rodriguez, Francesco**
**Via Kennedy 17**
**I-20097 San Donato Milanese, Milan(IT)**
Inventor: **Perego, Marta**
**Piazza San Pietro 6**
**I-23022 Chiavenna-Sondrio(IT)**
Inventor: **Carrera, Paolo**
**Via Zuretti 5**
**I-20125 Milan(IT)**
Inventor: **De Ferra, Francesca**
**Corso Mazzini 45**
**I-20075 Lodi, Milan(IT)**

(74) Representative: **Roggero, Sergio**
**Ing. Barzanò & Zanardo Milano S.p.A. Via Borgonuovo 10**
**I-20121 Milano (IT)**

## (54) Cloning and sequencing of that chromosomal DNA region of bacillus subtilis comprising the SRFA operon which encodes the multienzymatic complex surfactin synthetase.

(57) The cloning and sequencing of a Bacillus subtilis chromosomal DNA region comprising the srfA operon which encodes the multienzymatic complex surfactin synthetase responsible for surfactin production and induction of the state of competence of B.subtilis

Fig.1

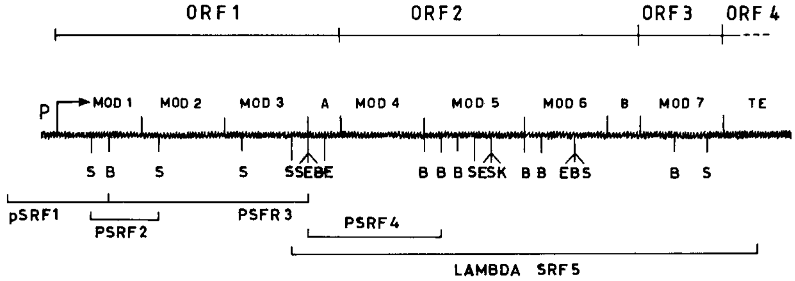


E = EcoRI; S = SacI; B = BamHI; K = KpnI

EP 0 540 074 A1

This invention relates to the cloning and sequencing of that chromosomal DNA region of Bacillus subtilis (B.subtilis) comprising the srfA operon which encodes the multienzymatic complex surfactin synthetase responsible for surfactin production and for inducing competence in this micro-organism.

Surfactin, a cyclic lipopeptide produced from B.subtilis, consists of a heptapeptide plus a lipid portion represented by a mixture of $\beta$-hydroxy-fatty acids with chains of between 13 and 15 carbon atoms.

The structure of surfactin is schematically as follows:

```
L-Leu ----- β-OH-C13-15 ---- L-Glu ---- L-Leu
  :                                       :
  :                                       :
D-Leu ----- L-Asp ---------- L-Val ---- D-Leu
```

Because of its multiple activity, surfactin finds wide application in the pharmaceutical, energy and environmental fields and is hence of particular interest.

In this respect, surfactin is a powerful surfactant with anti-cholesterase, fungicidal and antibiotic properties.

In addition, surfactin can be used as an anticoagulant in the prophylaxis of thrombosis, and generally for the prevention of illnesses such as myocardium infarct and pulmonary emboly, as it inhibits the fibrinogen-thrombin reaction by slowing fibrin formation.

Currently available methods for producing surfactin are based essentially on fermentation of the strain B.subtilis ATCC 21332 isolated by Arima K. (US 3,687,926) or its mutants (Mulligan C. et al. Appl. Microbiol. Biotech., 31: 486-489, (1989); IT-20738 A/90 and IT-22176 A/90).

However, none of these methods has proved sufficiently economical to allow its development on an industrial scale, mainly because of the low productivity of the currently available micro-organisms.

The application of molecular cloning to surfactin producing micro-organisms could enable the productivity of this lipopeptide to be improved and new surfactin analogues to be created with characteristics better than the natural product.

To allow such an approach, the gene or genes involved in surfactin synthesis have obviously to be isolated, sequenced and possibly modified.

It is known that surfactin biosynthesis takes place with a non-ribosomal mechanism analogous to that of other antibiotic peptides of the tyrocidin and gramicidin type (Kleinkauf H. and von Doren H., (1987), Ann. Rev. Microbiol., 41: 259-289; Mittenhuber G. et al. (1989), J. Bacteriology, 171: 4881-4887).

These antibiotic peptides are synthesized from particular proteins (synthetase) with various associated enzymatic activity, such as:

- activation of amino acid residues;
- racemization of amino acid residues from L to D form;
- polymerization and possibly cyclization of the final product.

Recent genetic and molecular biology studies on the chromosome of B.subtilis have identified three genetic loci (sfp, srfA and comA) involved in the biosynthesis of surfactin (Nakano M.M. et al. (1988), J. Bacteriology, 170: 5662-5668; Nakano M.M. and Zuber P. (1989), J. Bacteriology, 171: 5347-5353).

With regard to the srfA locus, it has been reported (Nakano M. et al., J. of Bacteriol. 173: 1770-1778 (1991)) that this is a wide operon of about 25 Kb involved in surfactin production and competence induction. For the srfA operon the authors give only a partial characterisation ad a short nucleotide sequence (about 1000 bases).

The genetic characterisation of the entire srfA operon and the identification and sequencing of the promoter and genes responsible for surfactin synthesis are reported hereinafter for the first time.

One aspect of the present invention is that chromosomal DNA region of B.subtilis comprising the srfA operon which encodes the multienzymatic complex surfactin synthetase.

A further aspect of the present invention is a DNA sequence comprising at least one coding sequence (ORF) for the srfA operon.

A further aspect of the present invention is a cloning vector containing at least one coding sequence (ORF) for the srfA operon.

A further aspect of the present invention is a method for producing surfactin by means of a culture of host cells transformed with an expression cloning vector containing the B.subtilis srfA operon.

A further aspect of the present invention is a method for producing at least one enzymatic protein of the surfactin synthetase complex by means of a culture of host cells transformed with an expression cloning vector containing a DNA sequence (ORF) coding for the srfA operon.

A further aspect of the present invention is the use of at least one enzymatic protein of the surfactin synthetase complex for the in vitro or in vivo preparation of peptides.

A further aspect of the present invention is the use of regions of the srfA operon for preparing specific antibodies for surfactin synthesis.

A further aspect of the present invention is the use of said antibodies for purifying surfactin synthetase.

Further aspects of the present invention will be more apparent from the ensuing description.

Brief description of the terminology used:

Locus: a genetically defined region of the chromosomal DNA involved in a phenotypic expression.

Operon: a DNA region comprising a complex of near genes transcribed together, the expression of which is hence controlled simultaneously, and a control region (regulon) which regulates this expression.

Promoter: a region within the regulon involved in the transcription of a gene to which the RNA polymerase is bound for commencing transcription.

Open Reading Frame (ORF): sequence containing a continuous open reading phase.

Auxotrophy: a phenotypic condition which occurs when a mutation causes the inactivation of a biosynthetic path, making the cell dependent on the presence of one or more layers in the growth culture medium.

Competence: the physiological state in which the bacterial cells bind and internalize extraneous DNA. The final product of this process depends on the type of DNA. If the incorporated DNA contains regions of homology with the receiving chromosome or with a plasmid present in the cell, an integration can take place, whereas if it is a non-homologous covalently closed plasmid with an origin of replication it stabilizes as a independent replication entity. Some of the genes implicated in competence are known as com genes.

Description of the figures:

Figure 1

Functional and restriction map of the srfA operon. The seven structural domains (MOD 1-7) present in ORF1, ORF2 and ORF3 are indicated. Isolated recombinant phages and plasmids are also shown, containing DNA fragments of the operon. The restriction sites indicated are: EcoRI (E), SacI (S), BamHI (B) and KpnI (K).

Figure 2:

Comparative analysis of the seven structural domains (MOD 1-7) found in ORF1, ORF2 and ORF3. The asterisks indicate the identical residues present in all seven modules. The dot indicates the presence of similar amino acids. The three amino acid sequences found in all seven domains are underlined.

Figure 3

Functional and restriction map of the region downstream of the srfA operon.

Detailed description of the invention

In isolating the entire region responsible for surfactin synthesis, the first step was to transfer the capacity to produce surfactin from a B.subtilis surfactin producer strain (donor) such as B.subtilis ATCC 21332, to a genetically manipulable non-producer (receiving) strain such as B.subtilis JH642 ($phe^-$, $trp^-$) (M.M. Nakano and P. Zuber (1989), J. Bacteriol., 171: 5347-5353).

For this purpose the chromosomal DNA extracted from the donor strain was transferred into a receiving strain and, after isolating the transformants by an auxotrophic marker (present only on the donor) the surfactin producing colonies were analyzed.

By operating in this manner some surfactin producer strains (JH642 $srf^+$) were isolated.

After this, the map position on the bacterial chromosome of the JH642 srf$^+$ strains of the DNA responsible for the surfactin production was determined.

Genetic mapping was achieved by transduction with the PBS-1 bacteriophage (Hoch J.A. et al. (1967) J. Bacteriology, 93: 1925-1937).

This method is based on the fact that when following induction the phagic particles leave the host strain (phagic lysate), they carry inserted into their gene makeup portions of B.subtilis chromosomal DNA which can have a size of up to 5-6% of the host genome.

The phagic lysate constructed on a surfactin producer strain and able for example to use mannitol as carbon source, is used for infecting a B.subtilis strain incapable of producing surfactin and using mannitol.

As these two markers are fairly close on the chromosome they can be co-transduced onto the DNA of the same phagic particle. When the DNA of the transducing phage comes into contact with that of the receiving strain, the high percentage of homology existing between the two DNA zones favours their recombination following one or more cross-overs.

By selecting on a suitable culture medium containing mannitol only those bacterial colonies capable of using this sugar (mtl$^+$) as the only carbon source, they will be able to grow. If at this point the mtl$^+$ colonies are transferred onto plates containing blood and they are selected by appearance of the hemolysis halo, some colonies will be srf$^+$ whereas others will be srf$^-$. The percentages with which the two markers are co-transferred indicate their distance apart on the chromosome.

Using this method it is possible to determine, by means of a three factor cross-over, the map position of the DNA sequence of interest relative to the genetic markers aroI, mtlB and dalI.

The results showed that the srf (surfactin) marker is positioned on the chromosomal DNA between the markers aroI ad mtlB.

In order to clone the DNA sequence responsible for surfactin production, a gene bank was constructed from chromosomal DNA isolated from the surfactin non-producer strain JM642 using a plasmid integration vector.

The choice of this strategy is based on the principle that B.subtilis integration vectors such as pJM7145, pJM7146, pJM103 and pJM102, replicate in Escherichia coli (E.coli), but are unable to replicate in B.subtilis as they do not contain that DNA portion responsible for the replication of the plasmid in this microorganism. These plasmid vectors are however able to integrate into the B.subtilis chromosome via homologous recombination and hence to express the resistance marker which they contain.

This mechanism can occur only if a fragment of B.subtilis chromosomal DNA is present on the plasmid to provide the homology zone necessary for the cross-over and hence the integration to take place.

In this case a gene bank was constructed in E.coli by digesting the chromosomal DNA isolated from B.subtilis JH642 with PstI and binding it to the integrating vector pJM103.

This gene bank was analyzed for the presence of colonies containing a plasmid which by integrating into the chromosome of a B.subtilis JH642 srf$^+$ surfactin producer strain would inactivate the production of this lipopeptide.

The clones which were srf$^-$ by integration of the plasmid DNA of the gene bank were further analyzed. Specifically, their chromosomal DNA was purified, digested with a restriction enzyme chosen so as not to have an insertion site within the pJM103 plasmid, and finally bound onto itself.

The ligase mixture was then used to transform competent E.coli cells, selecting by resistance to the ampicillin carried by the pJM103 plasmid.

The ampicillin resistant clones were those containing the plasmids formed from pJM103 and a portion of B.subtilis chromosomal DNA.

These plasmids were able to circularize during the ligase reaction.

Specifically, the isolated plasmids (p2E1, p8E9, p17E17, p26E25, p27E33 and p29E41) were formed from the initial integrating plasmid and those chromosomal DNA fragments of B.subtilis JH642 srf$^+$ adjacent to the plasmid insertion site.

The dimensions of these fragments (inserts) in the various plasmids are given in Table 2.

From a subsequent analysis of the restriction and cross-hybridization maps it was found that the plasmids p2E1 and p26E25 contained part of the fragments carried by p17E17 and p27E33 respectively. All these plasmids, when integrated into the chromosome of a B.subtilis surfactin producer strain, inactivated surfactin production and mapped in the region between aroI and mtlB.

As the chromosomal DNA fragments originating from the plasmids p8E9, p17E17, p27E33 and p29E41 do not represent the entire srfA operon, in order to extend the cloned gene zone an analysis was made of mini-gene banks from chromosomal DNA of B.subtilis JH642-4 srf$^+$ obtained by chromosome walking (Strauss E.C. et al. (1987) Anal. Biochem. 154: 353).

In practice, the p27E33, p17E17 and p8E9 inserts were isolated by enzymatic digestion of the plasmid DNA, radioactively labelled and then used as probes for identifying the clones of B.subtilis gene banks in the plasmid integration vector and in the phagic vector lambda FIXII which should cover increasingly large zones of the operon.

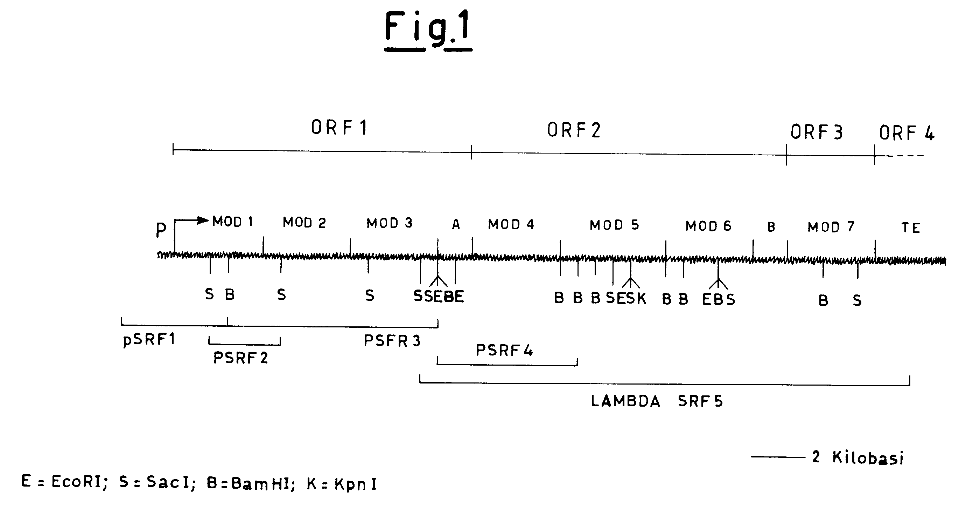

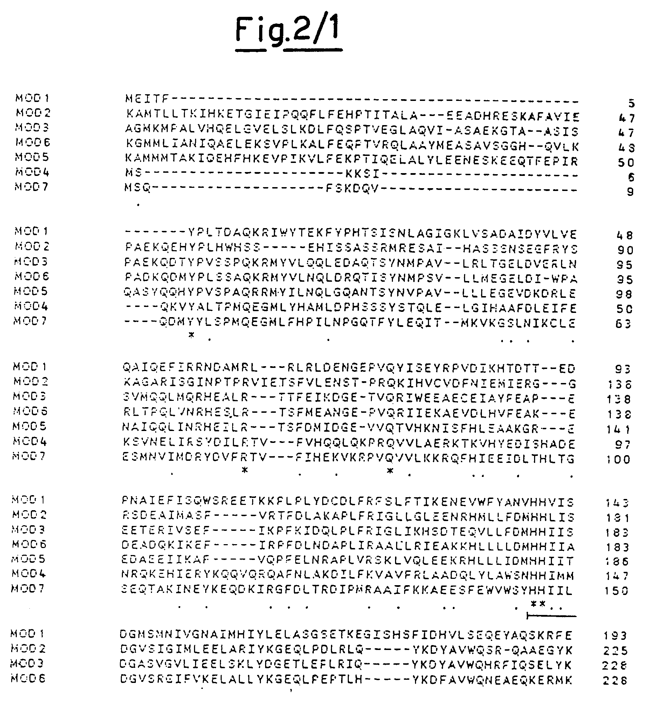

Operating in this manner the plasmids pSRF3, pSRF2, pSRF1, pSRF4 and lambda SRF5 were isolated, containing the DNA fragments of the srfA operon shown in Figure 1.

The pSRF1, pSRF2, pSRF3, pSRF4 and lambda SRF5 inserts were then sequenced by the method of Sanger et al. ((1977), PNAS, 74: 5463 - 5467) using the sequence strategy described by Strauss et al. (-(1986), Analytical Biochem., 154: 353 - 360).

The results of these experiments led to the construction of the restriction map of the entire srfA operon, to the definition of the precise limits of the gene complex of surfactin synthetase including the regulation sequences, and the cloning of nearly the entire coding zone (Figure 1).

The srfA operon consists of 27 kilobases (Kb) and comprises four regions (Open Reading Frames or ORFs) potentially coding for different enzymatic proteins, a zone upstream of the first ORf containing the srfA operon promoter (from - 610 to + 1) and a presumed terminator downstream of the stop codon of the fourth ORF.

The nucleotide sequence of the srfA operon is reported in Table 3 below. The start and stop codons of the four identified ORFs are indicated. The possible attachment sites of the ribosomes (SD) responsible for activating the translation of the four ORFs are also indicated.

TABLE 3

```
-609                                                   ATCGACAAA
-600    AATGTCATGA AAGAATCGTT GTAAGACGCT CTTCGCAAGG GTGTCTTTTT
-550    TTGCCTTTTT TTCGGTTTTT GCGCGGCACA CATAGTCATG TAAAGATTGT
-500    AAATTGCATT CAGCAATAAA AAAAGATTGA ACGCAGCAGT TGATTGGTTT
-450    AAAAATTTTT ATTTTTCTGT AAATAATGTT TAGTGGAAAT GATTGCGGCA
-400    TCCCGCAAAA AATATTGCTG TAAATAAACT GGAATCTTTC GGCATCCCGC
-350    ATGAAACTTT TCACCCATTT TTCGGTGATA AAAACATTTT TTTCATTTAA
-300    ACTGAACGGT AGAAAGATAA AAAATATTGA AAACAATGAA TAAATAGCCA
-250    AAATTGGTTT CTTATTAGGG TGGGGTCTTG CGGTCTTTAT CCGCTTATGT
-200    TAAACGCCGC AATGCTGACT GACGGCAGCC TGCTTTAATA GCGGCCATCT
-150    GTTTTTTGAT TGGAAGCACT GCTTTTTAAG TGTAGTACTT TGGGCTATTT
-100    CGGCTGTTAG TTCATAAGAA TTAAAAGCTG ATATGGATAA GAAAGAGAAA
                                              SD1
- 50    ATGCGTTGCA CATGTTCACT GCTTATAAAG ATTAGGGGAG GTATGACAAT
ORF1
----->
ATGGAAATAA CTTTTTACCC TTTAACGGAT GCACAAAAAC GAATTTGGTA     50
CACAGAAAAA TTTTATCCTC ACACGAGCAT TTCAAATCTT GCGGGGATTG    100
GTAAGCTGGT TTCAGCTGAT GCGATTGATT ATGTGCTTGT TGAGCAGGCG    150
ATTCAAGAGT TTATTCGCAG AAATGACGCC ATGCGCCTTC GGTTGCGGCT    200
AGATGAAAAC GGGGAGCCTG TTCAATATAT TAGCGAGTAT CGGCCTGTTG    250
ATATAAAACA TACTGACACT ACTGAAGATC CGAATGCGAT AGAGTTTATT    300
TCACAATGGA GCCGGGAGGA AACGAAGAAA CCTTTGCCGC TATACGATTG    350
TGATTTGTTC CGTTTTTCCT TGTTCACCAT AAAGGAAAAT GAAGTGTGGT    400
TTTACGCAAA TGTTCATCAC GTGATTTCTG ATGGTATGTC CATGAATATT    450
GTCGGGAATG CGATCATGCA CATTTATTTA GAATTAGCCA GCGGCTCAGA    500
GACAAAAGAA GGAATCTCGC ATTCATTTAT CGATCATGTT TTATCTGAAC    550
AGGAATATGC TCAATCGAAG CGGTTTGAAA AGGACAAGGC GTTTTGGAAC    600
AAACAATTTG AATCGGTGCC TGAACTTGTT TCCTTGAAAC GGAATGCATC    650
CGCAGGGGGA AGTTTAGATG CTGAGAGGTT CTCTAAAGAT GTGCCTGAAG    700
CGCTTCATCA GCAGATTCTG TCGTTTTGTG AGGCGAATAA AGTCAGTGTT    750
```

(Table 3 cont.)

| | | | | | |
|---|---|---|---|---|---|
| CTTTCGGTAT | TTCAATCGCT | GCTCGCCGCC | TATTTGTACA | GGGTCAGCGG | 800 |
| CCAGAATGAT | GTTGTGACGG | GAACATTTAT | GGGCAACCGG | CAAAATGCGA | 850 |
| AAGAGAAGCA | GATGCTTGGC | ATGTTTGTTT | CTACGGTTCC | GCTTCGGACA | 900 |
| AACATTGACG | GCGGGCAGGC | GTTTTCAGAA | TTTGTCAAAG | ACCGGATGAA | 950 |
| GGATCTGATG | AAGACACTTC | GCCACCAAAA | GTATCCGTAT | AATCTCCTAA | 1000 |
| TCAACGATTT | GCGTGAAACA | AAGAGCTCTC | TGACCAAGCT | GTTCACGGTT | 1050 |
| TCTCTTGAAT | ATCAAGTGAT | GCAGTGGCAG | AAAGAAGAGG | ATCTTGCCTT | 1100 |
| TTTGACTGAG | CCGATTTTCA | GCGGCAGCGG | ATTAAATGAT | GTCTCAATTC | 1150 |
| ATGTAAAGGA | TCGATGGGAT | ACTGGGAAAC | TCACCATAGA | TTTTGATTAC | 1200 |
| CGCACTGATT | TATTTTCACG | TGAAGAAATC | AACATGATTT | GTGAGCGCAT | 1250 |
| GATTACCATG | CTGGAGAACG | CGTTAACGCA | TCCAGAACAT | ACAATTGATG | 1300 |
| AATTAACACT | GATTTCTGAT | GCGGAGAAAG | AGAAGCTGCT | TGCGAGGGCC | 1350 |
| GGCGGTAAAT | CTGTGAGCTA | CCGTAAGGAC | ATGACGATAC | CAGAGCTGTT | 1400 |
| CCAAGAAAAG | GCTGAACTGC | TTTCTGATCA | TCCAGCGGTT | GTATTTGAAG | 1450 |
| ATCGCACATT | GTCCTATCGA | ACGTTACATG | AGCAATCTGC | ACGCATCGCC | 1500 |
| AATGTGCTGA | AACAGAAAGG | GGTTGGCCCG | GACAGTCCTG | TCGCGGTTTT | 1550 |
| GATTGAACGC | TCTGAACGGA | TGATTACAGC | TATCATGGGA | ATTTTAAAAG | 1600 |
| CCGGCGGAGC | CTATGTGGCG | ATTGATCCGG | GTTTTCCTGC | TGAGCGCATT | 1650 |
| CAATATATTT | TGGAGGACTG | CGGGGCGGAT | TTCTACCTGA | CTGAATCGAA | 1700 |
| GGTTGCGGCG | CCTGAAGCCG | ATGCTGAGCT | GATTGACTTA | GATCAGGCGA | 1750 |
| TTGAGGAAGG | TGCAGAAGAA | AGCCTGAATG | CAGATGTGAA | CGCTCGGAAC | 1800 |
| CTTGCCTACA | TTATTTACAC | ATCGGGAACA | ACCGGACGCC | CGAAAGGCGT | 1850 |
| TATGATCGAG | CATCGCCAGG | TTCATCATTT | GGTTGAATCT | CTGCAGCAGA | 1900 |
| CGATTTATCA | AAGCCCCACC | CAAACCCTCC | CCATGGCATT | CCTTCCGCCG | 1950 |
| TTCCACTTTG | ATGCGTCAGT | GAAGCAGATC | TTCGCGTCGC | TTCTTTTGGG | 2000 |
| CCAAACCCTT | TATATCGTAC | CGAAGAAAAC | AGTGACGAAC | GGGGCCGCCC | 2050 |
| TTACTGCATA | TTATCGGAAG | AACAGCATTG | AGGCGACGGA | CGGAACACCG | 2100 |
| GCTCATTTGC | AAATGCTGGC | AGCAGCAGGC | GATTTTGAAG | GCCTAAAACT | 2150 |
| GAAGCACATG | CTGATCGGAG | GAGAAGGCCT | GTCATCTGTT | GTTGCGGACA | 2200 |
| AGCTGCTGAA | GCTGTTTAAA | GAAGCCGGCA | CAGCGCCGCG | TTTGACTAAT | 2250 |
| GTGTACGGGC | CGACTGAAAC | GTGCGTTGAC | GCGTCTGTTC | ATCCGGTTAT | 2300 |
| CCCTGAGAAT | GCAGTTCAAT | CAGCGTATGT | GCCGATCGGG | AAAGCGCTGG | 2350 |

(Table 3 cont.)

| | | | | | |
|---|---|---|---|---|---|
| GGAATAACCG | CTTATATATT | TTGGATCAAA | AAGGCCGGCT | GCAGCCTGAA | 2400 |
| GGCGTGGCGG | GTGAGCTTTA | TATCGCGGGA | GACGGTGTGG | GCCGAGGCTA | 2450 |
| TTTACATTTG | CCTGAATTAA | CGGAAGAGAA | GTTTTTACAA | GATCCATTCG | 2500 |
| TGCCGGGCGA | TCGCATGTAC | CGGACCGGGG | ACGTGGTGCG | CTGGCTTCCA | 2550 |
| GATGGAACAA | TCGAATATTT | AGGCAGAGAG | GATGACCAGG | TCAAAGTCCG | 2600 |
| CGGATACCGG | ATTGAGCTTG | GGGAAATTGA | AGCCGTGATT | CAGCAGGCGC | 2650 |
| CAGACGTTGC | AAAAGCCGTT | GTTTTGGCAC | GCCCTGACGA | ACAGGGAAAT | 2700 |
| CTTGAGGTTT | GCGCATATGT | TGTGCAGAAG | CCTGGAAGCG | AATTTGCGCC | 2750 |
| AGCCGGTTTG | AGGGAGCATG | CGGCCAGACA | GCTTCCTGAC | TATATGGTGC | 2800 |
| CGGCTTACTT | TACAGAAGTG | ACAGAAATTC | CGCTTACACC | AAGCGGCAAA | 2850 |
| GTCGACCGCC | GCAAGCTGTT | TGCACTAGAG | GTGAAGGCTG | TCAGCGGCAC | 2900 |
| TGCCTATACA | GCGCCGCGAA | ATGAGACTGA | AAAAGCAATC | GCAGCCATTT | 2950 |
| GGCAGGACGT | GCTGAACGTT | GAGAAGGCGG | GGATCTTTGA | CAATTTCTTT | 3000 |
| GAAACTGGCG | GACATTCATT | AAAAGCCATG | ACCCTTTTAA | CAAAGATTCA | 3050 |
| TAAGGAAACA | GGCATTGAGA | TTCCGCAACA | ATTTTTGTTT | GAGCATCCGA | 3100 |
| CGATTACGGC | TCTTGCAGAG | GAAGCTGATC | ACAGAGAAAG | CAAAGCTTTT | 3150 |
| GCGGTGATTG | AACCTGCTGA | AAAACAGGAG | CATTACCCGC | TTCATTGGCA | 3200 |
| CAGCAGCGAA | CATATATCGT | CAGCCAGTTC | GAGGATGCGG | GAGTCGGCTA | 3250 |
| TACATGCCAG | CAGCAGCAAT | TCTGAAGGCT | TTAGATATTC | AAAAGCTGGA | 3300 |
| GCGCGCATTT | CAGGGATTAA | TCCGACGCCA | CGAGTCATTG | AGACATCATT | 3350 |
| TGTTCTTGAA | AACAGCACGC | CGAGACAGAA | AATTCACGTA | TGCGTTGATT | 3400 |
| TCAACATCGA | AATGATTGAA | AGAGGCGGCC | GCTCAGATGA | GGCAATTATG | 3450 |
| GCTTCATTCG | TTCGGACATT | TGATTTGGCG | AAAGCTCCGC | TGTTCAGAAT | 3500 |
| CGGTTTGCTG | GGGCTTGAAG | AGAACCGTCA | TATGCTGCTG | TTTGACATGC | 3550 |
| ACCATTTGAT | TTCTGACGGT | GTATCCATTG | GCATTATGCT | GGAGGAGTTA | 3600 |
| GCACGCATTT | ATAAAGGCGA | ACAGCTTCCT | GATCTTCGTC | TCCAGTATAA | 3650 |
| GGACTACGCT | GTATGGCAAA | GCAGACAGGC | TGCTGAAGGG | TACAAGAAGG | 3700 |
| ACCAGGCTTA | TTGGAAGGAA | GTCTTTGCAG | GCGAGCTCCC | GGTGCTTCAG | 3750 |
| CTTCTGTCCG | ATTACCCAAG | ACCACCTGTT | CAAAGCTTTG | AAGGGGATCG | 3800 |
| GGTGTCAATC | AAGCTGGATG | CGGGGGTAAA | GGATCGCCTC | AATCGTTTGG | 3850 |
| CTGAACAAAA | CGGCGCCACT | TTATATATGG | TGATGCTTTC | CGCTTACTAT | 3900 |
| ACGCTTTTGT | CAAAGTATAC | GGGGCAGGAT | GACATCATTG | TCGGGACACC | 3950 |

(Table 3 cont.)

| | | | | | |
|---|---|---|---|---|---|
| GTCAGCGGGC | AGAAATCACT | CCGATACAGA | GGGCATTATC | GGGATGTTCG | 4000 |
| TCAATACGCT | TGCGATTCGC | AGTGAGGTGA | AGCAGAATGA | GACGTTTACC | 4050 |
| CAATTGATCT | CGCGTGTCCG | CAAACGGGTG | CTGGATGCCT | TTTCTCATCA | 4100 |
| GGACTATCCG | TTTGAGTGGC | TTGTTGAAGA | TTTGAACATC | CCGCGTGATG | 4150 |
| TTAGCAGGCA | TCCGCTGTTT | GACACGATGT | TCAGCCTTCA | AAACGCGACA | 4200 |
| GAGGGCATTC | CGGCTGTCGG | CGATCTTTCC | TTGTCTGTTC | AAGAGACCAA | 4250 |
| TTTCAAGATT | GCCAAATTTG | ATTTGACGGT | GCAGGCGAGA | GAAACCGATG | 4300 |
| AAGGCATTGA | GATTGATGTG | GATTACAGCA | CAAAGCTGTT | TAAACAAAGC | 4350 |
| ACGGCAGACA | GGCTGCTTAC | GCATTTTGCG | CGTTTGCTTG | AAGATGCTGC | 4400 |
| GGCTGATCCA | GAGAAGCCGA | TTTCTGAGTA | TAAGCTTCTT | TCTGAAGAGG | 4450 |
| AGGCTGCTTC | GCAAATTCAG | CAGTTTAACC | CGGGCAGAAC | ACCTTATCCG | 4500 |
| AAAGATAAAA | CAATTGTTCA | GCTGTTTGAG | GAGCAAGCGG | CGAATACGCC | 4550 |
| AGACCACACT | GCGCTTCAAT | ATGAAGGCGA | ATCACTCACT | TATCGTGAAC | 4600 |
| TGAATGAACG | GGCCAATCGT | TTAGCCCGCG | GCATTCTTTC | TCTTGGAGCT | 4650 |
| GGCGAAGGCA | GAACTGCGGC | TGTCTTATGC | GAGCGGTCAA | TGGATATGAT | 4700 |
| TGTGTCGATC | TTGGCAGTAT | TAAAATCAGG | TTCGGCTTAT | GTTCCGATCG | 4750 |
| ATCCGGAACA | TCCGATTCAG | CGGATGCAGC | ATTTCTTCCG | TGACAGCGGA | 4800 |
| GCAAAGGTGC | TTCTCACTCA | GAGGAAATTA | AAGGCTTTGG | CGGAAGAAGC | 4850 |
| GGAATTTAAG | GGCGTTATCG | TGCTAGCCGA | TGAGGAAGAA | AGCTATCATG | 4900 |
| CCGATGCGCG | AAATCTCGCA | CTGCCTCTTG | ATTCTGCAGC | AATGGCCAAC | 4950 |
| CTGACGTATA | CTTCCGGAAC | GACTGGAACA | CCTAAGGGGA | ATATCGTGAC | 5000 |
| ACATGCCAAT | ATTCTCCGCA | CGGTGAAGGA | AACGAATTAT | CTCAGCATTA | 5050 |
| CAGAACAGGA | TACGATTCTC | GGTCTTTCCA | ATTACGTGTT | TGACGCGTTT | 5100 |
| ATGTTCGATA | TGTTCGGCTC | TTTGTTAAAC | GGAGCCAAGC | TGGTGCTGAT | 5150 |
| ACCGAAGGAA | ACCGTTTTGG | ACATGGCTCG | CCTGTCCCGC | GTCATTGAAC | 5200 |
| GGGAGAACAT | CAGCATTCTC | ATGATTACAA | CCGCTTTGTT | CCACTTGCTT | 5250 |
| GTGGACTTGA | ATCCGGCATG | CTTGTCAACG | CTTCGCAAGA | TTATGTTTGG | 5300 |
| CGGGGAACGG | GCTTCGGTTG | AGCATGTCAG | AAAAGCTTTG | CAAACGGTTG | 5350 |
| GAAAGGGCAA | GCTCCTTCAT | ATGTATGGAC | CGTCTGAAAG | CACGGTTTTC | 5400 |
| GCGACGTATC | ATCCGGTTGA | TGAATTGGAG | GAGCACACGC | TGTCTGTTCC | 5450 |
| GATTGGAAAA | CCGGTCAGCA | ATACGGAAGT | ATACATTCTT | GACCGTACGG | 5500 |

(Table 3 cont.)

| | | | | | |
|---|---|---|---|---|---|
| GACACGTGCA | GCCTGCCGGG | ATTGCCGGAG | AGCTTTGCGT | CACGGGCGAA | 5550 |
| GGACTCGTGA | AAGGCTATTA | CAACCGTCCA | GAACTGACTG | AGGAGAAATT | 5600 |
| TGTTCCCCAT | CCGTTTACAT | CCGGCGAACG | CATGTATAAA | ACGGGAGACC | 5650 |
| TTGCGAGATG | GCTGCCGAAT | GGGACCATCG | AATTTATCGG | GCGAATCGAC | 5700 |
| CATCAGGTGA | AGATTCGCGG | ACAGGCCATC | GAGCTTGGAG | AAATCGAACA | 5750 |
| TCAGCTGCAA | ACCCATGATC | GTGTTCAGGA | AAGTGTTGTG | CTTGCCGTTG | 5800 |
| ATCAAGGAGC | GGGAGATAAA | CTGTTGTGTG | CTTACTATGT | CGGAGAAGGA | 5850 |
| GACATCTCAT | CACAAGAGAT | GAGAGAGCAT | GCTGCGAAGG | ACTTGCCGGC | 5900 |
| ATATATGGTT | CCTGCGGTGT | TTATCCAAAT | GGACGAGCTG | CCGCTGACAG | 5950 |
| GGAACGGAAA | AATTGACCGG | AGAGCGCTGC | CGATTCCTGA | TGCCAACGTT | 6000 |
| TCAAGAGGTG | TTTCATATGT | TGCGCCACGC | AATGGAACGG | AACAAAAAGT | 6050 |
| CGCGGACATT | TGGGCACAGG | TACTTCAGGC | AGAACAAGTC | GGCGCTTATG | 6100 |
| ACCACTTCTT | TGACATTGGC | GGACATTCAT | TAGCAGGCAT | GAAGATGCCT | 6150 |
| GCCTTGGTTC | ATCAGGAACT | GGGCGTTGAG | CTGTCACTCA | AGGATCTCTT | 6200 |
| CCAGTCACCG | ACGGTTGAGG | GCTTGGCACA | GGTGATTGCC | TCTGCTGAAA | 6250 |
| AAGGGACAGC | CGCAAGTATC | AGCCCGGCAG | AGAAACAAGA | TACGTATCCT | 6300 |
| GTTTCTTCAC | CGCAAAAACG | GATGTACGTG | CTTCAGCAGC | TTGAGGATGC | 6350 |
| GCAAACGAGC | TATAACATGC | CGGCGGTTCT | GCGCCTGACA | GGTGAGCTTG | 6400 |
| ATGTTGAAAG | GCTTAACAGC | GTCATGCAGC | AGTTAATGCA | GCGTCATGAA | 6450 |
| GCCTTGAGAA | CCACGTTTGA | AATAAAAGAT | GGAGAAACGG | TGCAGCGGAT | 6500 |
| CTGGGAAGAG | GCTGAGTGCG | AGATAGCCTA | TTTCGAAGCC | CCGGAAGAAG | 6550 |
| AGACAGAGCG | GATCGTTTCT | GAGTTTATTA | AGCCTTTCAA | AATCGACCAA | 6600 |
| CTTCCACTGT | TCAGAATAGG | GCTTATCAAG | CATTCAGACA | CTGAGCAGGT | 6650 |
| GCTGCTGTTC | GATATGCATC | ATATTATTTC | TGATGGTGCA | TCTGTCGGTG | 6700 |
| TGCTGATTGA | GGAGCTTTCA | AAGCTGTACG | ACGGAGAAAC | CCTTGAGCCG | 6750 |
| CTCCGTATTC | AATATAAGGA | TTATGCCGTG | TGGCAGCACA | GGTTCATTCA | 6800 |
| GTCTGAGCTT | TACAAGAAGC | AAGAAGAGCA | TTGGCTGAAG | GAGCTCGACG | 6850 |
| GAGAGCTGCC | GGTGCTGACG | CTTCCGACTG | ATTACAGTCG | GCCTGCCGTT | 6900 |
| CAAACATTTG | AGGGAGACCG | CATTGCATTT | TCATTAGAAG | CAGGCAAAGC | 6950 |
| TGATGCTCTG | CGCAGGCTTG | CAAAAGAAAC | GGATTCCACG | CTTTACATGG | 7000 |
| TGCTTCTGGC | TTCATACAGT | GCGTTTTTAT | CAAAAATTTG | CGGGCAAGAC | 7050 |
| GATATCATCG | TCGGTTCACC | TGTGGCCGGA | CGATCTCAAG | CGGACGTCAG | 7100 |

(Table 3 cont.)

CCGCGTCATC GGAATGTTCG TCAATACATT GGCGCTGCGC ACGTATCCGA 7150
AGGGTGAAAA GACGTTTGCT GACTATCTTA ACGAAGTGAA AGAAACTGCA 7200
CTCAGCGCTT TTGATGCGCA GGATTACCCA CTTGAGGATT TGATCGGAAA 7250
TGTTCAGGTT CAGCGTGACA CAAGCAGCAA TCCGTTATTC GATGCAGTTT 7300
TTTCAATGCA AAATGCGAAT ATAAAGGATT TAACAATGAA AGGGATTCAG 7350
CTTGAGCCGC ATCCGTTTGA ACGGAAAACA GCCAAGTTTG ACCTCACGCT 7400
GACGGCTGAC GAAACCGACG GAGGGCTTAC ATTCGTACTC GAATACAATA 7450
CAGCTCTGTT TAAGCAGGAA ACGATTGAAC GATGGAAGCA ATATTGGATG 7500
GAGCTTTTAG ATGCAGTTAC TGGGAACCCG AACCAGCCGC TTTCCAGCCT 7550
GTCACTGGTC ACCGAGACAG AAAAGCAAGC GCTTCTTGAG GCTTGGAAGG 7600
GCAAAGCGCT GCCTGTGCCG ACAGACAAAA CGGTTCATCA GCTATTCGAA 7650
GAGACTGCCC AGCGCCACAA AGACCGCCCG GCTGTCACAT ACAACGGCCA 7700
GTCTTGGACG TACGGCGAGC TGAATGCGAA GGCAAACCGT CTCGCGCGGA 7750
TTCTGATGGA CTGCGGCATC AGCCCGGATG ACCGCGTCGG CGTTCTCACG 7800
AAGCCGTCGC TTGAAATGTC CGCCGCGGTG CTCGGCGTCT TGAAAGCCGG 7850
AGCGGCGTTT GTGCCGATTG ATCCTGACTA TCCGGATCAG CGGATTGAGT 7900
ATATTTTACA GGACAGCGGC GCGAAGCTTC TCTTGAAACA GGAAGGCATT 7950
TCAGTGCCGG ACAGCTATAC GGGAGATGTC ATTCTTCTCG ACGGAAGCCG 8000
CACGATTCTA AGCCTGCCGC TTGATGAAAA CGACGAGGAA AATCCAGAAA 8050
ATCCAGAAAC CGCTGTAACC GCGGAGAACT TGGCGTACAT GATTTACACG 8100
TCTGGAACGA CCGGACAGCC GAAGGGTGTC ATGGTCGAGC ACCATGCGCT 8150
TGTGAACCTG TGCTTCTGGC ACCACGACGC GTTCAGCATG ACAGCGGAGG 8200
ACCGCAGTGC GAAGTACGCG GGCTTTGGGT TCGACGCTTC CATTTGGGAG 8250
ATGTTCCCGA CCTGGAGCAT CGGTGCCGAA CTTCACGTCA TTGAGGAAGC 8300
GATCCGCCTC GATATCGTCC GCCTGAACGA TTATTTTGAA ACGAACGGCG 8350
TAACGATCAC GTTCCTGCCG ACACAGCTTG CGGAACAGTT CATGGAGCTT 8400
GAGAACACAT CACTTCGCGT ATTGCTTACT GGAGGAGACA AGCTGAAGCG 8450
CGCAGTGAAA AAGCCGTACA CACTCGTCAA TAACTACGGG CCGACAGAGA 8500
ATACGGTCGT TGCCACAAGC GCAGAAATCC ATCCGGAGGA AGGCTCGCTT 8550
TCCATCGGAA GGGCCATTGC CAATACGAGA GTATACATTC TCGGCGAGGG 8600
CAATCAGGTG CAGCCGGAAG GCGTAGCCGG AGAGCTTTGC GTGGCGGGGC 8650
GCGGACTGGC ACGCGGCTAT CTGAATCGAG AAGACGAAAC CGCGAAGCGG 8700

(Table 3 cont.)

| | | | | | |
|---|---|---|---|---|---|
| TTTGTCGCTG | ATCCGTTTGT | GCCGGGTGAG | CGCATGTACC | GCACCGGCGA | 8750 |
| TTTGGTGAAA | TGGACGGGCG | GCGGCATCGA | ATACATCGGG | CGGATCGACC | 8800 |
| AGCAGGTCAA | GGTCCGCGGC | TACCGGATCG | AGCTCTCAGA | AATTGAAGTC | 8850 |
| CAGCTCGCCC | AGCTTTCTGA | GGTGCAGGAT | GCGGCGGTGA | CAGCTGTCAA | 8900 |
| AGATAAAGGC | GGCAACACAG | CGATCGCGGC | GTATGTCACA | CCGGAATCAG | 8950 |
| CTGACATAGA | AGCACTGAAA | TCAGCACTGA | AGGAAACCCT | GCCGGATTAC | 9000 |
| ATGATCCCGG | CGTTCTGGGT | GACGCTGAAC | GAGCTTCCGG | TTACGGCAAA | 9050 |
| CGGCAAAGTG | GACCGCAAAG | CCTTGAATGA | GCCGGACATC | GAAGCGGGAA | 9100 |
| GCGGAGAATA | CAAAGCGCCG | ACGACCGACA | TGGAAGAGCT | GCTTGCCGGC | 9150 |
| ATCTGGCAGG | ACGTGCTTGG | AATGTCTGAA | GTCGGTGTCA | CCGACAATTT | 9200 |
| CTTCTCGCTT | GGCGGAGATT | CCATCAAAGG | AATTCAAATG | GCGAGCCGCT | 9250 |
| TGAACCAGCA | CGGCTGGAAG | CTGGAAATGA | AAGATCTCAA | CCAGCACCCG | 9300 |
| ACGATCGAAG | AGCTCACCCA | GTACGTAGAG | CGTGCCGAAG | GCAAACAGGC | 9350 |
| AGACCAAGGC | CCGGTGGAGG | GCGAAGTCAT | CCTGACGCCG | ATCCAGCGCT | 9400 |
| GGTTCTTTGA | AAAGAACTTC | ACGAACAAGC | ACCACTGGAA | CCAATCTGTG | 9450 |
| ATGCTTCACG | CCAAGAAGGG | CTTTGATCCT | GAACGGGTGG | AGAAAACATT | 9500 |
| GCAGGCGCTG | ATCGAGCATC | ATGACGCGCT | CCGCATGGTC | TATCGTGAGG | 9550 |
| GACAGGAAGA | CGTCATTCAA | TACAACAGAG | GTCTTGAAGC | TGCTTCAGCT | 9600 |
| CAATTGGAGG | TCATCCAGAT | TGAGGGCCAA | GCTGCAGATT | ACGAAGACCG | 9650 |
| AATAGAGAGA | GAAGCGGAGC | GTTTGCAAAG | CAGCATCGAC | TTGCAGGAAG | 9700 |
| GCGGCTTGTT | AAAAGCAGGC | TTGTTCCAAG | CGGAAGACGG | AGATCACTTG | 9750 |
| CTTCTTGCCA | TTCACCACTT | AGTGGTTGAC | GGCGTGTCGT | GGCGGATTTT | 9800 |
| ACTGGAGGAT | TTCTCCGCGG | TATATACACA | GCTTGAGCAA | GGCAATGAAC | 9850 |
| CGGTTCTCCC | GCAGAAAACA | CATTCATTTG | CAGAGTATGC | AGAGAGATTG | 9900 |
| CAAGACTTCG | CGAACTCCAA | AGCCTTTTTG | AAAGAAAAAG | AGTATTGGTC | 9950 |
| ACAGCTTGAA | GAACAAGCTG | TTGCGGCAAA | GCTTCCGAAA | GACCGCGAAT | 10000 |
| CTGGTGATCA | GCGAATGAAA | CATACAAAGA | CAATTGAATT | CTCGCTGACT | 10050 |
| GCTGAAGAGA | CAGAACAGCT | CACCACAAAG | GTGCATGAGG | CATATCACAC | 10100 |
| AGAAATGAAT | GATATTTTGC | TGACGGCATT | CGGATTGGCA | ATGAAGGAGT | 10150 |
| GGACGGGTCA | AGATCGAGTA | AGTGTTCATT | TAGAGGGGCA | TGGACGTGAA | 10200 |
| GAAATCATAG | AAGACCTGAC | CATTTCTCGC | ACAGTCGGCT | GGTTTACAAG | 10250 |
| TATGTACCCA | ATGGTGCTCG | ATATGAAGCA | TGCGGATGAT | CTGGGCTACC | 10300 |
| AGCTGAAGCA | AATGAAAGAA | GATATCAGAC | ATGTGCCGAA | TAAGGGAGTC | 10350 |

(Table 3 cont.)

```
GGATCAGGCA TTCTGCGCTA TCTGACGGCA CCGGAACATA AAGAAGATGT   10400
GGCGTTCTCG ATTCAGCCGG ATGTCAGCTT CAACTATTTA GGTCAGTTTC   10450
ACCAAATGTC CCATCCACCT TTCTTTACCA CATCACACCT CCCATCACCA   10500
CACTCATTAA GCCCTGAAAC AGAAAAACCG AATGCGCTGG ATGTTGTCGG   10550
ATATATCGAA AACGGAAAAC TGACGATGTC ACTGGCCTAT CATTCTCTTG   10600
AATTTCATGA AAAAACAGTA CAAACATTCA GTGACAGCTT TAAAGCGCAT   10650
CTTCTCAGAA TCATTGAACA TTGCCTATCT CAAGATGGTA CGGAACTGAC   10700
CCCGAGTGAT CTTGGTGACG ACGATTTGAC GCTGGATGAG CTGGATAAAT   10750
                STOP ORF1  SD2          ---> ORF2
TAATGGAAAT TTTCTAATAG AGGTGGCATA TGAGCAAAAA ATCGATTCAA   10800
AAGGTGTACG CACTGACACC AATGCAGGAG GGAATGCTGT ATCATGCGAT   10850
GCTTGATCCG CATTCTTCCT CGTACTCCAC ACAATTAGAG CTTGGGATTC   10900
ACGCCGCTTT TGATCTTGAA ATCTTTGAGA AAAGCGTCAA TGAACTGATT   10950
CGGTCATACG ATATTCTCCG TACGGTATTT GTGCATCAGC AGCTGCAAAA   11000
ACCGCGTCAG GTCGTGTTAG CGGAACGCAA AACAAAAGTG CATTATGAGG   11050
ATATCAGTCA TGCAGACGAA AACCGCCAGA AAGAGCACAT TGAGCGTTAC   11100
AAACAACAGG TTCAGCGCCA AGCCTTTAAC CTGGCAAAAG ACATATTGTT   11150
CAAGGTAGCG GTTTTCCGCC TTGCTGCAGA TCAGCTGTAT CTTGCTTGGA   11200
GCAATCATCA TATTATGATG GACGGCTGGA GCATGGGCGT CCTCATGAAA   11250
AGCCTGTTCC AAAACTATGA AGCGCTCAGA GCAGGCAGGA CACCGGCAAA   11300
CGGTCAGGGC AAGCCTTATT CCGATTACAT CAAGTGGCTT GGAAAACAGG   11350
ACAATGAAGA AGCGGAGAGC TACTGGAGCG AGCGCTTGGC GGGTTTTGAA   11400
CAGCCAAGCG TGCTCCCGGG CCGCCTGCCT GTGAAAAAAG ACGAATACGT   11450
CAATAAAGAA TATTCATTTA CATGGGACGA AACACTGGTT GCCCGTATTC   11500
AGCAAACCGC AAATCTCCAT CAAGTGACAG GGCCTAACCT ATTTCAGGCC   11550
GTTTTGGGCA TTGTTCTCAG CAAATACAAC TTTACGGATG ATGTGATCTT   11600
CGGAACGGTC GTCTCGGGAC GACCGTCTGA AATCAACGGA ATCGAAACGA   11650
TGGCGGGGCT GTTTATCAAC ACCATTCCAG TGCGGGTGAA AGTTGAACGA   11700
GATCGTGCAT TCGCTGATAT TTTCACAGCT GTTCAGCAGC ATGCAGTAGA   11750
GGCAGAGCGT TACGATTACG TGCCGCTCTA TGAGATTCAA AAACGCTCAG   11800
CTCTTGATGG CAATCTCTTA AACCATCTGG TCGCGTTTGA AAATTATCCG   11850
CTTGATCAAG AGCTTGAAAA CGGCAGCATG GAAGACCGCC TCGGGTTTTC   11900
```

(Table 3 cont.)

| | | | | | |
|---|---|---|---|---|---|
| GATTAAGGTA | GAAAGCGCAT | TTGAACAAAC | GAGCTTCGAT | TTCAACCTGA | 11950 |
| TTGTGTATCC | GGGCAAAACG | TGGACCGTCA | AAATTAAATA | TAACGGAGCC | 12000 |
| GCCTTTGATT | CCGCTTTTAT | CGAACGGACA | GCGGAGCACC | TTACCCGCAT | 12050 |
| GATGGAAGCA | GCTGTCGATC | AGCCGGCCGC | TTTTGTGCGT | GAGTACGGGC | 12100 |
| TTGTGGGAGA | CGAAGAGCAG | CGGCAAATTG | TCGAGGTGTT | TAACAGCACG | 12150 |
| AAAGCCGAAC | TCCCTGAAGG | AATGGCTGTT | CACCAAGTGT | TTGAAGAGCA | 12200 |
| AGCGAAACGG | ACGCCGGCGA | GCACCGCCGT | CGTATATGAA | GGAACCAAGC | 12250 |
| TGACGTACCG | CGAGCTGAAT | GCAGCGGCTA | ACCGTCTGGC | GAGAAAGCTT | 12300 |
| GTCGAACATG | GGCTTCAAAA | AGGGGAAACA | GCAGCGATTA | TGAACGACCG | 12350 |
| CTCAGTAGAA | ACCGTTGTCG | GCATGCTGGC | TGTTTTAAAA | GCAGGCGCCG | 12400 |
| CATATGTGCC | GCTTGATCCA | GCGCTTCCTG | GGGATCGTCT | TCGTTTCATG | 12450 |
| GCAGAGGACA | GCTCCGTTCG | AATGGTTTTG | ATCGGAAATT | CTTATACAGG | 12500 |
| GCAGGCACAT | CAGCTGCAAG | TGCCGGTTCT | TACACTGGAC | ATAGGCTTTG | 12550 |
| AAGAGAGTGA | AGCGGCTGAC | AATCTCAACC | TGCCATCCGC | CCCGTCTGAT | 12600 |
| TTGGCGTACA | TCATGTACAC | ATCCGGTTCA | ACGGGCAAAC | CGAAAGGCGT | 12650 |
| CATGATCGAA | CATAAAAGCA | TTCTGCGCCT | CGTCAAAAAT | GCCGGGTACG | 12700 |
| TTCCTGTTAC | TGAAGAAGAC | GCAATGGCGC | AAACGGGAGC | TGTCAGCTTT | 12750 |
| GATGCCGGAA | CGTTTGAGGT | ATTCGGCGCA | CTGCTGAATG | GCGCAGCACT | 12800 |
| TTATCCGGTC | AAAAAGAGAC | ACGTGCTTGA | TGCGAAACAA | TTTGCTGCAT | 12850 |
| TCCTGCGTGA | GCAAAGCATC | ACAACTATGT | GGCTGACATC | ACCTTTATTC | 12900 |
| AACCAGCTTG | CTGCAAAGGA | TGCGGGTATG | TTCGGCACAC | TGCGCCATTT | 12950 |
| AATCATCGGC | GGAGATGCCC | TTGTCCCGCA | TATTGTCAGC | AAAGTAAAAC | 13000 |
| AGGCATCGCC | GTCGCTTTCA | TTGTGGAACG | GATACGGCCC | GACAGAAAAC | 13050 |
| ACGACGTTTT | CAACCAGTTT | TCTGATCGAC | CGCGAGTATG | GCGGCTCTAT | 13100 |
| CCCAATCGGG | AAGCCGATCG | GAAATTCCAC | TGCCTATATT | ATGGATGAGC | 13150 |
| AGCAATGCCT | GCAGCCAATC | GGCGCGCCTG | GTGAGCTTTG | CGTAGGCGGA | 13200 |
| ATCGGTGTAG | CGCGTGGGTA | TGTCAATCTC | CCTGAGCTGA | CGGAGAAGCA | 13250 |
| ATTTCTGGAA | GACCCGTTCA | GACCGGGTGA | GAGAATTTAC | CGCACTGGTG | 13300 |
| ACTTGGCAAG | ATGGCTGCCG | GACGGCAATA | TCGAATTTTT | AGGCAGAATC | 13350 |
| GACAACCAAG | TGAAGGTGCG | CGGCTTCCGA | ATTGAGCTTG | GCGAAATTGA | 13400 |
| AACAAAACTG | AACATGGCTG | AGCATGTGAC | AGAAGCCGCG | GTGATCATAC | 13450 |
| GCAAGAACAA | AGCAGACGAA | AATGAAATTT | GCGCGTACTT | CACGGCGGAT | 13500 |

(Table 3 cont.)

| | | | | | |
|---|---|---|---|---|---|
| CGTGAAGTGG | CTGTGAGTGA | GCTGAGAAAA | ACACTGTCTC | AGTCTTTGCC | 13550 |
| TGACTATATG | GTCCCTGCCC | ACCTGATTCA | AATGGACAGC | CTGCCGCTCA | 13600 |
| CGCCAAACGG | GAAAATCAAC | AAAAAGGAAC | TGCCTGCACC | GCAATCAGAA | 13650 |
| GCCGTGCAGC | CGGAATACGC | AGCACCAAAA | ACAGAGAGTG | AAAAGAAATT | 13700 |
| AGCGGAGATC | TGGGAAGGAA | TACTCGGCGT | CAAAGCAGGC | GTTACCGATA | 13750 |
| ACTTCTTTAT | GATCGGCGGC | CATTCTTTGA | AAGCGATGAT | GATGACGGCG | 13800 |
| AAAATTCAAG | AGCATTTTCA | TAAGGAAGTT | CCGATAAAAG | TGCTTTTTGA | 13850 |
| AAAGCCGACT | ATTCAAGAAC | TGGCACTGTA | TTTGGAAGAG | AACGAAAGCA | 13900 |
| AGGAGGAGCA | GACGTTTGAA | CCGATCAGGC | AAGCATCTTA | TCAGCAGCAT | 13950 |
| TATCCTGTAT | CCCCGGCCCA | GCGGAGAATG | TATATCCTCA | ATCAGCTTGG | 14000 |
| ACAAGCAAAC | ACAAGCTACA | ACGTCCCCGC | TGTACTTCTG | CTGGAGGGAG | 14050 |
| AAGTAGATAA | AGACCGGCTT | GAAAACGCAA | TTCAGCAATT | AATCAACCGG | 14100 |
| CACGAAATCC | TCCGTACATC | GTTTGACATG | ATCGACGGAG | AGGTTGTGCA | 14150 |
| AACCGTTCAT | AAAAACATAT | CCTTCCACCT | GGAGGCTGCC | AAGGGACGGG | 14200 |
| AAGAAGACGC | GGAAGAGATA | ATCAAAGCAT | TTGTTCAGCC | GTTTGAATTA | 14250 |
| AACCGCGCGC | CGCTCGTCCG | TTCGAAGCTT | GTCCAGCTGG | AAGAAAAACG | 14300 |
| CCACCTGCTG | CTCATTGATA | TGCATCATAT | TATTACTGAC | GGAAGTTCAA | 14350 |
| CAGGCATTCT | AATCGGTGAT | CTTGCCAAAA | TATATCAAGG | CGCAGATCTG | 14400 |
| GAACTGCCAC | AAATTCACTA | TAAAGATTAC | GCAGTTTGGC | ACAAAGAACA | 14450 |
| AACTAATTAT | CAAAAAGATG | AGGAATACTG | GCTCGATGTC | TTTAAAGGCG | 14500 |
| AACTGCCAAT | ACTGGATCTT | CCCGCGGATT | TCGAGCGGCC | AGCTGAACGG | 14550 |
| AGCTTTGCGG | GAGAGCGCGT | GATGTTTGGG | CTTGATAAGC | AAATCACGGC | 14600 |
| TCAAATCAAA | TCGCTCATGG | CAGAAACAGA | TACGACAATG | TACATGTTTT | 14650 |
| TGCTGGCGGC | GTTCAATGTA | CTCCTTTCCA | AGTACGCGTC | ACAGGATGAT | 14700 |
| ATCATTGTCG | GCTCGCCGAC | AGCTGGCAGA | ACACATCCTG | ATCTGCAAGG | 14750 |
| TGTGCCGGGT | ATGTTTGTCA | ACACGGGGGC | ACTAAGAACG | GCACCAGCGG | 14800 |
| GAGATAAAAC | CTTCGCGCAA | TTCCTTGAAG | AGGTCAAAAC | AGCCAGCCTT | 14850 |
| CAAGCATTCG | AGCACCAGAG | CTATCCGCTT | GAGGAGCTGA | TTGAAAAGCT | 14900 |
| TCCGCTTACA | AGGGATACAA | GCAGAAGTCC | GCTGTTCAGC | GTGATGTTCA | 14950 |
| ACATGCAGAA | TATGGAGATT | CCTTCATTAA | GATTAGGAGA | TTTGAAGATT | 15000 |
| TCCTCGTATT | CCATGCTTCA | TCATGTTGCG | AAATTTGATC | TTTCCTTGGA | 15050 |
| AGCGGTCGAG | CGTGAAGAGG | ATATCGGCCT | AAGCTTTGAC | TATGCGACTG | 15100 |

(Table 3 cont.)

| | | | | | |
|---|---|---|---|---|---|
| CCTTGTTTAA | GGACGAGACG | ATCCGCCGCT | GGAGCCGCCA | CTTTGTCAAT | 15150 |
| ATCATCAAAG | CGGCCGCGGC | TAATCCGAAC | GTTCGGCTGT | CTGATGTAGA | 15200 |
| TCTGCTTTCA | TCTGCAGAAA | CGGCTGCTTT | GCTAGAAGAA | AGACATATGA | 15250 |
| CTCAAATTAC | CGAAGCAACC | TTTGCAGCAC | TTTTTGAAAA | ACAGGCCCAG | 15300 |
| CAAACACCTG | ACCATTCTGC | GGTGAAGGCT | GGCGGAAATC | TGTTGACCTA | 15350 |
| TCGTGAGCTT | GATGAACAGG | CGAACCAGCT | GGCGCATCAT | CTTCGTGCCC | 15400 |
| AAGGGGCAGG | AAATGAAGAC | ATCGTCGCGA | TTGTTATGGA | CCGGTCAGCT | 15450 |
| GAAGTCATGG | TATCCATTCT | CGGTGTCATG | AAGGCGGGGG | CAGCTTTCCT | 15500 |
| TCCGATTGAT | CCTGATACAC | CTGGAGAACG | AATCCGTTAT | TCATTAGAGG | 15550 |
| ACAGCGGAGC | AAAATTTGCG | GTCGTGAATG | AAAGAAACAT | GACGGCTATT | 15600 |
| GGGCAATATG | AAGGGATAAT | TGTCAGCCTT | GATGACGGTA | AATGGAGAAA | 15650 |
| TGAAAGCAAG | GAGCGCCCAT | CATCCATTTC | CGGGTCTCGC | AATCTTGCAT | 15700 |
| ACGTCATTTA | TACGTCCGGT | ACGACCGGAA | AGCCAAAGGG | CGTGCAGATT | 15750 |
| GAGCATCGTA | ATCTGACAAA | CTATGTCTCT | TGGTTTAGTG | AAGAGGCGGG | 15800 |
| CCTGACGAAG | AGGCGGGCTG | ACGGAAATGA | TAAGACTGTA | TTGCTTTCAT | 15850 |
| CTTACGCATT | TGACCTTGGC | TATACGTGCA | TGTTCCCTGT | ACTTCTGGGC | 15900 |
| GGGGGCGAGC | TCCATATCGT | CCAGAAGGAA | ACCTATACGG | CGCCGGATGA | 15950 |
| AATAGCGCAC | TATATCAAGG | AGCATGGGAT | CACTTATATC | AAGCTGACAC | 16000 |
| CGTCTCTGTT | CCATACAATA | GTGAACACCG | CCAGTTTTGC | TTTTGATGCG | 16050 |
| AACTTTGAAT | CCTTGCGCTT | GATCGTCTTG | GGAGGAGAAA | AAATCATCCC | 16100 |
| GACTGATGTT | ATCGCCTTCC | GTAAGATGTA | TGGACATACC | GAATTTATCA | 16150 |
| ATCACTACGG | CCCGACAGAA | GCAACGATCG | GCGCCATCGC | CGGGCGGGTT | 16200 |
| GATCTGTATG | AGCCGGATGC | ATTTGCGAAA | CGCCCGACAA | TCGGACGCCC | 16250 |
| GATTGCGAAT | GCCGGTGCGC | TTGTCTTAAA | TGAAGCATTG | AAGCTTGTGC | 16300 |
| CGCCTGGAGC | GAGCGGACAG | CTCTATATCA | CGGGACAGGG | GCTCGCGAGA | 16350 |
| GGGTATCTCA | ACAGGCCTCA | GCTGACAGCC | GAGAGATTTG | TAGAAAATCC | 16400 |
| ATATTCGCCG | GGAAGCCTCA | TGTACAAAAC | CGGAGATGTC | GTACGAAGAC | 16450 |
| TTTCTGACGG | TACGCTTGCA | TTTATCGGCC | GGGCTGATGA | TCAGGTGAAA | 16500 |
| ATCCGAGGCT | ACCGCATTGA | GTTGGGAGAA | ATTGAAACGG | TCATGCTCAG | 16550 |
| CCTCAGCGGA | ATTCAAGAAG | CGGTTGTACT | AGCGGTTTCC | GAGGGCGGTC | 16600 |
| TTCAAGAGCT | TTGCGCGTAT | TATACGTCGG | ATCAAGATAT | TGAAAAAGCA | 16650 |
| GAGCTCCGGT | ACCAGCTTTC | CCTAACACTG | CCGTCTCATA | TGATTCCTGC | 16700 |

(Table 3 cont.)

| | | | | | |
|---|---|---|---|---|---|
| TTTCTTTGTG | CAGGTTGACG | CGATTCCGCT | GACGGCAAAC | GGAAAAACCG | 16750 |
| ACAGAAACGC | TCTGCCGAAG | CCTAACGCGG | CACAATCCGG | AGGCAAGGCC | 16800 |
| TTGGCCGCAC | CGGAGACAGC | GCTTGAAGAA | AGTTTATGCC | GCATTTGGCA | 16850 |
| GAAAACGCTT | GGCATAGAAG | CCATCGGCAT | TGATGACAAT | TTTTTCGATT | 16900 |
| TAGGCGGCCA | TTCATTAAAA | GGGATGATGC | TGATTGCCAA | CATTCAGGCG | 16950 |
| GAATTGGAGA | AAAGCGTACC | GCTTAAAGCA | CTGTTCGAGC | AGCCGACAGT | 17000 |
| TCGCCAGCTG | GCGGCTTATA | TGGAGGCGTC | TGCTGTTTCA | GGCGGCCATC | 17050 |
| AAGTACTCAA | ACCGGCTGAC | AAGCAGGATA | TGTATCCATT | GTCATCCGCA | 17100 |
| CAGAAACGAA | TGTACGTGCT | CAATCAGCTT | GACCGCCAGA | CGATAAGCTA | 17150 |
| CAACATGCCA | TCTGTTCTTC | TAATGGAAGG | AGAGCTTGAT | ATTTGGCCTG | 17200 |
| CGCGACTCAC | TCCTCAGCTT | GTGAACCGTC | ACGAATCATT | GCGGACGTCA | 17250 |
| TTTATGGAAG | CTAATGGTGA | GCCTGTTCAG | CGCATCATTG | AGAAGGCGGA | 17300 |
| GGTTGATCTT | CATGTGTTTG | AAGCCAAAGA | AGACGAAGCG | GACCAAAAGA | 17350 |
| TTAAGGAATT | TATCCGGCCA | TTCGACTTAA | ACGACGCACC | GCTCATTCGC | 17400 |
| GCAGCTTTGC | TTCGAATAGA | AGCGAAAAAA | CATTTGCTGC | TTTTAGATAT | 17450 |
| GCATCATATC | ATCGCAGACG | GCGTCTCAAG | AGGCATCTTT | GTAAAAGAAT | 17500 |
| TGGCGCTGCT | TTACAAAGGA | GAGCAGCTTC | CGGAGCCGAC | GCTTCATTAT | 17550 |
| AAAGATTTCG | CCGTTTGGCA | AAATGAAGCT | GAGCAAAAAG | AACGGATGAA | 17600 |
| GGAGCATGAG | GCGTACTGGA | TGTCAGTTCT | TTCAGGCGAG | CTGCCAGAGC | 17650 |
| TTGATCTCCC | GCTCGATTAT | GCCCGTCCGC | CTGTGCAAAG | CTTTAAAGGA | 17700 |
| GATACGATCC | GTTTCCGTAC | GGGAAGTGAG | ACGGCAAAGG | CGGTAGAAAA | 17750 |
| ACTGCTTGCC | GAAACCGGAA | CGACCTTGCA | CATGGTGCTC | CATGCTGTTT | 17800 |
| TCCACGTCTT | TTTAAGCAAA | ATTTCCGGAC | AGCGGGATAT | CGTCATCGGC | 17850 |
| TCCGTTACTG | CCGGCCGGAC | GAATGCTGAT | GTTCAGGACA | TGCCGGGAAT | 17900 |
| GTTCGTCAAT | ACACTTGCCC | TGAGAATGGA | AGCGAAAGAA | CAGCAAACAT | 17950 |
| TTGCGGAGCT | TTTGGAGCTA | GCAAAGCAGA | CGAACCTGTC | AGCCCTTGAG | 18000 |
| CATCAGGAGT | ATCCGTTTGA | AGATCTGGTT | AATCAGCTTG | ATCTCCCTCG | 18050 |
| GGATATGAGC | CGAAACCCAT | TGTTTAATGT | GATGGTGACG | ACAGAAAACC | 18100 |
| CTGATAAAGA | AGAGCTTACA | TTGCAAAATC | TGAGCATTTC | ACCTTATGAG | 18150 |
| GCTCATCAGG | GAACTTCTAA | GTTTGATCTG | ACACTGGGCG | GATTTACTGA | 18200 |
| TGAAAATGGC | ATTGGCTTGC | AGCTCGAATA | TGCGACAGAT | CTGTTCGCAA | 18250 |
| AAGAAACAGC | TGAAAAATGG | AGCGAATACG | TTCTGCGGCT | ACTAAAGGCT | 18300 |

(Table 3 cont.)

| | | | | | |
|---|---|---|---|---|---|
| GTTGCGGATA | ACCCGAACCA | GCCGCTTTCC | AGTCTGTTAC | TGGTCACCGA | 18350 |
| GACAGAAAAG | CAAGCGCTTC | TTGAGGCATG | GAAGGGCAAA | GCGCTGCCTG | 18400 |
| TGCCGACAGA | CAAAACGGTT | CATCAGCTAT | TCGAAGAGAC | TGTCCAGCGC | 18450 |
| CACAAAGACC | GCCCGGCTGT | CACATACAAC | GGCCAATCTT | GGACGTACGG | 18500 |
| CGAGCTGAAC | GCGAAGGCAA | ACCGCCTCGC | CCGGATTCTG | ATGGACTGCG | 18550 |
| GCATCAGCCC | GGATGACCGC | GTCGGCGTTC | TCACGAAGCC | GTCGCTTGAA | 18600 |
| ATGTCCGCCG | CGGTGCTCGG | CGTCTTGAAA | GCCGGAGCGG | CGTTTGTGCC | 18650 |
| GATTGATCCT | GACTACCCGG | ATCAGCGGAT | TGAGTATATT | TTACAGGACA | 18700 |
| GCGGCGCGAA | GCTTCTCTTG | AAACAGGAAG | GCATTTCAGT | GCCGGACAGC | 18750 |
| TATACGGGAG | ATGTCATTCT | TCTCGACGGA | AGCCGCACGA | TTCTAAGCCT | 18800 |
| GCCGCTTGAT | GAAAACGACG | AGGGAAATCC | AGAAACCGCT | GTAACCGCGG | 18850 |
| AGAACTTGGC | GTACATGATT | TACACGTCTG | GAACGACCGG | ACAGCCGAAG | 18900 |
| GGTGTCATGG | TCGAGGACCA | TGCGCTTGTG | AACCTGTGCT | TCTGGGACCA | 18950 |
| CGACGCGTTC | AGCATGACAG | CGGAGGACCG | CAGTGCGAAG | TACGCGGGCT | 19000 |
| TCGGGTTCGA | CGCTTCCATT | TGGGAGATGT | TCCCGACCTG | GACGATCGGC | 19050 |
| GCTGAACTTC | ACGTCATTGA | TGAAGCGATC | CGCCTCGATA | TCGTCCGCCT | 19100 |
| GAACGATTAT | TTTGAAACGA | ACGGCGTAAC | GATCACGTTC | CTGCCGACAC | 19150 |
| AGCTTGCGGA | ACAGTTCATG | GAGCTTGAGA | ACACATCACT | TCGCGTCCTC | 19200 |
| TTGACCGGAG | GAGACAAGCT | GAAGCGGGCA | GTGAAAAAGC | CGTACACACT | 19250 |
| CGTCAACAAC | TACGGGCCGA | CAGAAAATAC | GGTCGTTGCC | ACAAGCGCAG | 19300 |
| AAATCCATCC | GGAGGAAGGC | TCGCTTTCCA | TCGGACGGGC | CATTGCCAAT | 19350 |
| ACGAGAGTAT | ACATTCTCGG | CGAGGGCAAT | CAGGTGCAGC | CGGAAGGCGT | 19400 |
| AGCCGGAGAG | CTTTGCGTGG | CGGGGCGCGG | ACTGGCACGA | GGCTATCTGA | 19450 |
| ATCGAGAAGA | CGAAACCGCG | AAGCGGTTTG | TCGCTGATCC | GTTTGTGCCG | 19500 |
| GGTGAACGCA | TGTACCGCAC | CGGCGACTTG | GTGAAGTGGG | TGAACGGCGG | 19550 |
| CATCGAATAC | ATCGGCCGGA | TCGACCAGCA | GGTCAAGGTC | CGCGGCTACC | 19600 |
| GGATCGAGCT | CTCAGAAATT | GAAGTCCAGC | TCGCCCAGCT | TTCTGAGGTC | 19650 |
| GAGGATCGGG | CGGTGACACG | TGTCAAAGAT | AAAGGCGGCA | ATACAGCGAT | 19700 |
| CGCGGCGTAT | GTCACACCGG | AAACAGCTGA | CATAGAAGCA | CTAAAATCAA | 19750 |
| CACTAAAGGA | AACCCTGCCG | GATTACATGA | TCCCGGCATT | CTGGGTGACG | 19800 |
| CTGAACGAGC | TTCCGGTTAC | GGCAAACGGC | AAAGTCGACC | GCAAAGCCTT | 19850 |
| GCCTGAGCCG | GACATCGAAG | CGGGAAGCGG | AGAATACAAA | GCGCCGACGA | 19900 |

(Table 3 cont.)

| | | | | | |
|---|---|---|---|---|---|
| CCGACATGGA | AGAGCTGCTT | GCCGGCATCT | GGCAGGACGT | GCTTGGAATG | 19950 |
| TCTGAAGTCG | GTGTCACCGA | CAATTTCTTC | TCGCTTGGCG | GAGATTCCAT | 20000 |
| CAAAGGAATT | CAAATGGCGA | GCCGCTTGAA | TCAGCACGGC | TGGAAGCTGG | 20050 |
| AAATGAAAGA | TCTCTTCCAG | CATCCGACGA | TCGAAGAGCT | CACCCAGTAC | 20100 |
| GTAGAGCGTG | CCGAAGGCAA | ACAGGCAGAC | CAAGGCCCGG | TGGAGGGCGA | 20150 |
| AGTCATCCTG | ACGCCGATCC | AGCGCTGGTT | CTTTGAAAAG | AACTTCACGA | 20200 |
| ACAAGCACCA | CTGGAACCAA | TCGGTGATGC | TTCACGCCAA | AAAGGGCTTT | 20250 |
| GATCCTGAAC | GGGTGGAGAA | AACATTGCAG | GCGCTGATCG | AGCATCATGA | 20300 |
| CGCGCTCCGC | ATGGTCTACC | GCGAGGAAAA | CGGGGACATC | GTTCAGGTGT | 20350 |
| ATAAACCGAT | CGGTGAGAGC | AAGGTCAGCT | TCGAAATCGT | GGATCTGTAC | 20400 |
| GGCTCCGATG | AAGAGATGCT | GAGAAGCCAG | ATTAAGCTTC | TCGCGAACAA | 20450 |
| GCTGCAAAGC | AGTCTCGATC | TGCGAAACGG | GCCGCTTTTA | AAGGCGGCAG | 20500 |
| AATATCGCAC | AGAAGCTGGG | GATCACCTGC | TCATTGCTGT | ACACCATCTC | 20550 |
| GTGGTCGACG | GTGTGTCATG | GCGGATTTTG | CTTGAAGACT | TTGCTTCAGG | 20600 |
| CTACATGCAG | GCTGAGAAAG | AAGAGAGCCT | TGTCTTCCCG | CAAAAAACAA | 20650 |
| ACTCCTTCAA | GGATTGGGCG | GAAGAACTGG | CGGCATTCAG | CCAATCAGCG | 20700 |
| CATCTTTTAC | AGCAGGCTGA | ATACTGGTCG | CAAATTGCCG | CTGAACAGGT | 20750 |
| TTCTCCTTTA | CCTAAGGATT | GTGAAACAGA | GCAGCGGATC | GTCAAGGATA | 20800 |
| CATCATCTGT | CCTATGTGAA | TTAACGGCAG | AAGACACTAA | GCATCTTTTA | 20850 |
| ACAGATGTTC | ATCAGCCATA | TGGAACTGAA | ATCAACGATA | TTCTTCTCAG | 20900 |
| CGCGCTCGGT | TTGACAATGA | AAGAATGGAC | AAAGGGGGCC | AAAATTGGCA | 20950 |
| TTAACCTTGA | GGGACACGGC | CGGGAGGACA | TTATCCCGAA | TGTGAATATC | 21000 |
| TCCAGAACGG | TCGGCTGGTT | TACGGCACAA | TACCCTGTTG | TGCTCGACAT | 21050 |
| ATCTGACGCA | GATGCCTCAG | CTGTGATCAA | AACAGTCAAA | GAAAACCTGC | 21100 |
| GCCGCATTCC | GGACAAAGGT | GTTGGCTACG | GCATTCTTCG | TTATTTCACA | 21150 |
| GAAACAGCGG | AAACAAAGGG | CTTCACACCG | GAGATCAGCT | TCAACTATTT | 21200 |
| GGGCCAATTC | GACAGTGAAG | TCAAAACCGA | TTTCTTTGAA | CCGTCCGCTT | 21250 |
| TCGATATGGG | GCGCCAAGTA | AGCGGAGAAT | CAGAGGCGCT | GTACGCATTA | 21300 |
| AGCTTCAGCG | GCATGATCAG | AAACGGCCGG | TTTGTGCTTT | CCTGCTCCTA | 21350 |
| CAATGAGAAG | GAGTTTGAAA | GAGCTACAGT | CGAGGAGGAA | ATGGAACGGT | 21400 |
| TTAAAGAAAA | CCTCCTGATG | CTAATCCGCC | ATTGCACGGA | AAAAGAAGAC | 21450 |
| AAGGAATTCA | CACCAAGCGA | CTTCAGCGCC | GAAGACCTTG | AAATGGACGA | 21500 |

(Table 3 cont.)

```
                                                STOP ORF2
GATGGGAGAT ATCTTTGACA TGCTTGAGGA GAATTTAAAA TAAAACGCAA   21550
                 SD3          ---> ORF3
GGGAATTACA GAAGGCGGGA GCGAAACATA TGAGTCAATT TAGCAAGGAT   21600
CAGGTTCAAG ATATGTATTA CCTATCGCCG ATGCAGGAAG GGATGCTTTT   21650
TCATCCGATC CTGAATCCCG GCCAAACGTT TTACCTTGAA CAAATCACGA   21700
TGAAAGTAAA AGGCAGCTTG AATATCAAAT GTCTTGAAGA AAGCATGAAT   21750
GTGATCATGG ACCGGTACGA TGTATTTCGT ACCGTGTTCA TTCACGAAAA   21800
AGTAAAAAGG CCTGTCCAAG TCGTATTGAA AAAACGGCAG TTCCATATAG   21850
AAGAAATCGA TCTGACACAC TTAACGGGCA GCGAGCAAAC AGCCAAAATC   21900
AATGAGTACA AAGAACAGGA TAAGATCAGG GGTTTTGATT TGACGCGGGA   21950
TATTCCGATG CGGGCAGCCA TTTTCAAGAA AGCTGAAGAA AGCTTTGAAT   22000
GGGTGTGGAG CTACCACCAC ATTATTTTGG ACGGATGGTG CTTCGGCATC   22050
GTCGTACAGG ATCTATTTAA GGTATACAAT GCTCTGCGCG AACAAAAGCC   22100
GTACAGCCTG CCGCCCGTCA AACCGTATAA AGACTACATC AAGTGGCTTG   22150
AAAAGCAGGA TAAACAAGCA TCACTGCGTT ACTGGCGCGA ATATTTAGAG   22200
GGCTTTGAAG GACAAACGAC GTTTGCGGAG CAAAGAAAGA AACAAAAGGA   22250
CGGCTATGAG CCGAAAGAGC TGCTCTTTTC ACTGTCGGAG GCGGAAACAA   22300
AGGCCTTTAC CGAGCTTGCA AAATCGCAGC ATACCACTTT GAGTACGGCG   22350
CTGCAGGCAG TCTGGAGCGT ATTGATCAGC CGCTATCAGC AGTCTGGCGA   22400
TTTGGCCTTC GGTACAGTTG TTTCAGGGCG TCCCGCGGAA ATCAAAGGCG   22450
TTGAACATAT GGTCGGGCTG TTTATCAACG TTGTCCCGAG ACGTGTGAAG   22500
CTGTCTGAGG GTATCACATT TAACGGCTTG CTCAAGCGGC TGCAGGAGCA   22550
ATCGCTGCAG TCCGAGCCGC ATCAATATGT GCCGCTTTAT GACATCCAAA   22600
GCCAGGCCGA TCAGCCGAAA CTGATTGACC ACATCATTGT TTTTGAGAAC   22650
TATCCGCTTC AGGATGCGAA AAATGAAGAA AGCAGTGAAA ACGGCTTTGA   22700
TATGGTGGAT GTTCATGTTT TTGAGAAGTC GAATTATGAT CTCAACCTGA   22750
TGGCTTCCCC GGGAGATGAG ATGCTGATTA AGCTTGCCTA TAATGAGAAT   22800
GTGTTTGATG AGGCGTTTAT CCTGCGCTTG AAATCTCAGC TTCTTACAGC   22850
AATTCAGCAG CTCATCCAGA ATCCTGATCA GCCTGTCAGC ACGATCAACC   22900
TCGTTGACGA CAGGGAGAGA GAATTTTTGC TAACCGGCTT AAACCCGCCG   22950
GCTCAAGCTC ATGAAACAAA GCCTCTGACG TATTGGTTCA AGGAAGCAGT   23000
```

(Table 3 cont.)

| | | | | | |
|---|---|---|---|---|---|
| GAACGCCAAT | CCGGATGCAC | CGGCGCTTAC | GTATTCCGGC | CAGACCCTGT | 23050 |
| CTTATCGCGA | ATTAGATGAG | GAAGCGAACC | GCATTGCACG | CCGGCTGCAA | 23100 |
| AAACACGGTG | CGGGCAAAGG | CTCTGTTGTA | GCGCTGTACA | CGAAGCGCTC | 23150 |
| ACTTGAACTG | GTGATCGGCA | TTCTCGGTGT | ATTAAAGGCG | GGAGCAGCCT | 23200 |
| ATCTGCCGGT | TGATCCGAAG | CTGCCAGAGG | ACCGAATCTC | GTATATGCTG | 23250 |
| GCTGACAGTG | CGGCAGCCTG | TCTTCTGACG | CATCAGGAGA | TGAAAGAACA | 23300 |
| AGCGGCTGAG | CTGCCGTATA | CAGGCACAAC | GCTCTTCATT | GATGATCAAA | 23350 |
| CACGGTTTGA | AGAACAGGCA | AGCGATCCTG | CAACCGCGAT | TGATCCTAAT | 23400 |
| GATCCGGCAT | ATATCATGTA | CACGTCCGGC | ACAACCGGAA | AGCCAAAGGG | 23450 |
| CAATATCACC | ACTCATGCCA | ATATTCAAGG | ATTGGTCAAG | CATGTAGACT | 23500 |
| ACATGGCATT | TTCTGATCAG | GATACGTTCT | TGTCTGTTTC | GAATTACGCC | 23550 |
| TTTGATGCAT | TTACCTTTGA | TTTCTATGCT | TCTATGCTGA | ATGCGGCACG | 23600 |
| GCTCATTATC | GCAGATGAAC | ATACGCTGCT | TGATACAGAA | CGGCTCACAG | 23650 |
| ATCTGATCCT | GCAAGAGAAT | GTCAATGTCA | TGTTTGCGAC | AACCGCACTA | 23700 |
| TTTAATCTTC | TCACAGATGC | GGGAGAGGAT | TGGATGAAGG | GGCTTCGCTG | 23750 |
| TATATTATTC | GGCGGAGAGC | GCGCGTCAGT | GCCTCATGTC | AGAAAAGCGC | 23800 |
| TGCGGATCAT | GGGGCCGGGC | AAGCTGATTA | ACTGCTACGG | GCCGACTGAG | 23850 |
| GGAACAGTGT | TTGCGACAGC | TCACGTCGTG | CATGATCTGC | CGGATTCCAT | 23900 |
| CTCCTCATTG | CCGATCGGAA | AGCCGATCAG | CAATGCCAGT | GTTTATATTC | 23950 |
| TGAATGAGCA | AAGCCAGCTC | CAGCCATTCG | GGGCGGTCGG | TGAACTGTGC | 24000 |
| ATCAGCGGAA | TGGGCGTGTC | AAAAGGGTAT | GTAAATCGTG | CTGACCTCAC | 24050 |
| GAAGGAAAAG | TTTATCGAGA | ACCCGTTCAA | GCCGGGAGAA | ACGCTTTACC | 24100 |
| GTACAGGGGA | TTTAGCGCGC | TGGCTGCCGG | ATGGAACGAT | TGAATACGCC | 24150 |
| GGCCGTATTG | ACGACCAGGT | CAAAATACGC | GGACACCGGA | TTGAGCTTGA | 24200 |
| AGAAATCGAA | AAGCAGCTGC | AGGAATACCC | AGGTGTGAAA | GATGCGGTCG | 24250 |
| TTGTGGCGGA | CCGCCATGAG | TCTGGCGATG | CATCAATCAA | TGCCTACCTT | 24300 |
| GTGAACCGAA | CGCAGCTTTC | AGCTGAAGAC | GTGAAGGCGC | ACCTGAAAAA | 24350 |
| ACAGCTTCCT | GCTTACATGG | TGCCGCAAAC | CTTTACCTTC | TTGGATGAGC | 24400 |
| TTCCTTTAAC | GACGAACGGG | AAAGTCAATA | AACGGCTGCT | CCCAAAACCT | 24450 |
| GATCAGGATC | AGCTGGCGGA | AGAATGGATT | GGACCGCGGA | ACGAGATGGA | 24500 |
| AGAAACAATC | GCACAAATAT | GGTCTGAGGT | TCTCGGCAGA | AAGCAAATTG | 24550 |
| GCATTCATGA | CGATTTCTTT | GCGCTCGGAG | GGCATTCCTT | GAAGGCCATG | 24600 |

(Table 3 cont.)

| | | | | | |
|---|---|---|---|---|---|
| ACCGCCGTCC | CGCATCAACA | AGAGCTCGCG | ATTGATCTTC | CAGTGAAGCT | 24650 |
| TTTGTTTGAA | GCGCCGACGA | TCGCCGGCAT | TTCAGCGTAT | TTGAAAAACG | 24700 |
| GGGGCTCTGA | TGGCTTGCAG | GATGTAACGA | TAATGAATCA | GGATCAGGAG | 24750 |
| CAGATCATTT | TCGCATTTCC | GCCGGTTCTG | GGCTATGGCC | TTATGTACCA | 24800 |
| AAATCTGTCC | AGCCGCTTGC | CGTCATACAA | GCTATGCGCC | TTTGATTTTA | 24850 |
| TTGAGGAGGA | AGACCGGCTT | GACCGCTATG | CGGATTTGAT | CCAGAAGCTG | 24900 |
| CAGCCGGAAG | GGCCTTTAAC | ATTGTTTGGA | TATTCAGCGG | GATGCAGCCT | 24950 |
| GGCGTTTGAA | GCTGCGAAAA | AGCTTGAGGA | ACAAGGCCGT | ATTGTTCAGC | 25000 |
| GGATCATCAT | GGTGGATTCC | TATAAAAAAC | AAGGTGTCAG | TGATCTGGAC | 25050 |
| GGACGCACGG | TTGAAAGTGA | TGTCGAAGCG | TTGATGAATG | TCAATCGGGA | 25100 |
| CAATGAAGCG | CTCAACAGCG | AAGCCGTCAA | ACACGGCCTC | AAGCAAAAAA | 25150 |
| CACATGCCTT | TTACTCATAC | TACGTCAACC | TGATCAGCAC | AGGCCAGGTG | 25200 |
| AAAGCAGATA | TTGATCTGTT | GACTTCCGGC | GCTGATTTTG | ACATGCCGGA | 25250 |
| ATGGCTTGCA | TCATGGGAAG | AAGCTACAAC | AGGTGTTTAC | CGTGTGAAAA | 25300 |
| GAGGCTTCGG | AACACACGCA | GAAATGCTGC | AGGGCGAAAC | GCTAGATAGG | 25350 |
| AATGCGGAGA | TTTTGCTCGA | ATTTCTTAAT | ACACAAACCG | TAACGGTTTC | 25400 |
| STOP ORF3 | SD4 | | ——> ORF4 | | |
| ATAAATGAAG | TGATGAAAGG | AGGAGACAGC | CAATGAGCCA | ACTCTTCAAA | 25450 |
| TCATTTGATG | CGTCGGAAAA | AACACAGCTC | ATCTGTTTTC | CGTTTGCCGG | 25500 |
| CGGCTATTCG | GCGTCGTTTC | GCCCTCTCCA | TGCTTTTTTG | CAGGGGGAGT | 25550 |
| GCGAGATGCT | CGCTGCCGAG | CCGCCGGGAC | ACGGCACGAA | TCAAACGTCA | 25600 |
| GCCATTGAGG | ATCTCGAAGA | GCTGACGGAT | TTGTACAAGC | AAGAACTGAA | 25650 |
| CCTTCGCCCT | GATCGGCCGT | TTGTGCTGTT | CGGACACAGT | ATGGGCGGAA | 25700 |
| TGATCACCTT | CAGGCTGGCG | CAAAAGCTTG | AGCGTGAAGG | CATCTTTCCG | 25750 |
| CAGGCGGTTA | TCATTTCTGC | AATCCAGCCG | CCTCATATTC | AGCGGAAGAA | 25800 |
| AGTGTCCCAC | CTGCCTGATG | ATCAGTTTCT | CGATCATATT | ATCCAATTAG | 25850 |
| GCGGAATGCC | CGCAGAGCTT | GTTGAAAATA | AGGAGGTCAT | GTCCTTTTTC | 25900 |
| CTGCCTTCTT | TCCGATCAGA | TTACCGGGCT | CTTGAACAAT | TTGAGCTTTA | 25950 |
| CGATCTGGCC | CAGATCCAGT | CGCCTGTTCA | TGTCTTTAAC | GGGCTTGATG | 26000 |
| ATAAAAAAATG | CATACGAGAT | GCGGAAGGGT | GGAAGAAGTG | GGCAAAAGAC | 26050 |
| ATCACATTCC | ATCAATTTGA | CGGCGGGCAC | ATGTTCCTGC | TGTCACAAAC | 26100 |
| GGAAGAAGTC | GCAGAACGGA | TTTTTGCGAT | CTTGAATCAG | CATCCGATCA | 26150 |

(Table 3 cont.)

```
        STOP ORF4
TTCAACCGTG ATCAAAAGCG GACAGCTTCG GCTGTTCCGC TTTTTTTGTG   26200
TTGAATGCCA ATTTCTGCAT GGTATAATAG TCGAAATACT CAAATAAAGG   26250
            ----> ORF5                ----> ORFY
CAGGTCGAAA CATGCGCACG TCTCCCAGGT CGAAATGGTT TGTATTCCTG   26300
TTTACGTTTG TTTTCGCCAT CGGAATGAAC TCATTCAGAA ATTCCTTTCA   26350
ATTTTTTATG CTGCCAATGG CAGACGCCTT CCATGCCGAC AGGTCGCTGA   26400
TTTCGGTTTC TGTCAGCATT TTTATGATCA CAACCGGCAT CGTCCAGTTT   26450
TTTGTCGGTT TTTTTATCGA CCGTTTCAGT GTCAGAAAAA TTATGGCGCT   26500
                                            STOP ORFX
GGGAGCTGTT TGCATCAGCG CAAGCTTTTT GGTGCTTCCT TATTCACCGA   26550
ATGTTCATGT GTTTTCCGCC ATTTACGGTG TGCTTGGCGG AATCGGGTAT   26600
                                            STOP ORFY
TCCTGCGCGG TCGGCGTGAC GACCCAGTAT TTCATCAGCT GTTGGTTTGA   26650
CACACACAAA GGTCTGGCGC TTGCTATTTT GACCAATGCC AACTCTGCGG   26700
GCCTCGTCGT CTCTCCGCCG CCCATTTGGG CTGCGGCTCC GTATCATGCC   26750
GGCTGGCAGA GCACCTATAC CATTTTGGGA ATCGTCATGG CGGCTGTTCT   26800
CGTGCCGCTC CTCGTCTTTG GGATGAAGCA CCCGCCACAT GCGCAAGCGG   26850
AAACTGTGAA AAAATCTTAT GATTGGCGAG GGTTTTGGAA CGTGATGAAG   26900
CAATCCCGCC TCATTCATAT CCTGTACTTC GGCGTGTTTA CTTGCGGATT   26950
TACAATGGGA ATTATTGATG CTCACCTCGT CCCGATACTG AAGGATGCGC   27000
 ORFX <----
ATGTCTCTCA TGTCAACGGA ATGATGGCCG CGTTCGGGGC ATTTATCATC   27050
ATTGGCGGAT TATTGGCGGG CTGGCTGTCA GATCTCCTCG GCAGCAGAAG   27100
CGTCATGCTA TCCATCTTAT TTTTCATTCG GCTGCTCAGC CTCATTTGCC   27150
TGCTCATTCC CATTCTCGGA ATTCATCACA GCGACCTTTG GTACTTCGGG   27200
TTTATCCTGT TATTCGGGCT CAGTTACACA GGCGTGATCC CGCTGACCGC   27250
GGCGTCAATT TCGGAAAGCT ATCAAACAGG ATTGATCGGA TCGCTGTTAG   27300
GCATCAATTT CTTTATCCAT CAGGTTGCCG GAGCTCTCAG CGTGTATGCG   27350
GGCGGTTTGT TTTTTGACAT GACTCATGGT TATTTACTGA TTGTCGCTGT   27400
GTGCATCGTG TTTGTGGGTT TATCGGCTGT AATAGAGCTG GTGCCGTTTT   27450
```

(Table 3 cont.)

STOP ORF5

TAGATAAACA GAAGGCAAAA GAAACCCACC ATTCAATATA AAAGGATCAG 27500

STOP ORF6

CACTGTCAAT GCTGATCCTT TTTAAATTTG AGTTTTTTTG TTTCGGTATT 27550

TTTAAGGATA ATCTCCTTGA ATCTGTTCAT CTCCTCTTCG GAGTGAAAAA 27600

AATGTTTGGG GATCGCAATA TATTGGCCAC TTGATGTATT GACCCTGAAG 27650

ATATTTTTAT ATTCGAAAAC AGCTGTAATT TCATCCCAAT TGAAAATGAG 27700

GTCATATTTT TTAGAACATA TACGAATTCC TTCTTGGTTC AGAGTATATG 27750

TTCTTTTGGA TTTCATCCGT TCGTTCTTCT TGTATGCTCT GGAAAACTTC 27800

ACATATAGAA GAAGGAGGAG CAGCAGTGTA AACAGAACGG ACATCACGAT 27850

AATCAACATA CTGTTCATAA ACAAGTTAAG CGCGTCACTT CCATAAACGA 27900

TACGGGGCCA TCCGTTAATG AAGTTAACTG CAGCAAATAT GGCGCAAAAC 27950

AATAGAAAGT AAAAGCATGA TATTTTTGCG ATTTGATAAA AAAAGATTTC 28000

TCTTACATCC TTAAAAGTGA TTTCTCCTGG AAGATTGATA CTTTCACTAG 28050

ORF6 <——

CATATTGAAT CATACGGTAA ACCTACACCT CTAACCATGT TTTTCCTTTT 28100

<—— STOP ORF7

AGTCTAGCAT AATTTCTCAT TTTTTTGCAG GCATACCAGG GCGCTTTGTT 28150

TTTTTCTCCA GATTGATATT GCTCCCCACC ACGCCAATCA TAATACAAAC 28200

AGCCCCGGCA AGATGATACC AGGCCAGGCT TTCGTTGAGA ATCACAAATC 28250

CTGCAATCAT CGTCACAATG GTAGATACAT GATTAAAAGC GCTCATTTTA 28300

AACGCCTCAA TTCGCGACAG CGTATAGTTA GACAGAAATG AAGTGACAAG 28350

TGAAGACAGC ACGCCCAAAT ACACAATGGC GAGAACGAAG CCGGGCTCCC 28400

GGAACGGCAG AAAATAAGTG CCGACTGTTC CCGCCGCTCC GTGACGCACA 28450

AGGGCGATGG CGTTGAAGAC GACAAAGCCG ATGGCTGACA TGATGTAGGT 28500

GAGCTCGGTC AGCTTGAACC GCTGCGTCAT TTTTCTGGCA GCAGTATTGT 28550

ACATCGCTGA AGACAAAGCG GACAATAGGA TCAGCAAAGA CCCTTTTAAG 28600

CTGGCTGATT CCACGTCAAC GCCTTTCATC ACAAAAATAA ACATGACGCC 28650

GGCAACGGAT AAAACCGTGA ACCCCTTTTG CGTCCACGTT GGGCGTTCCT 28700

TTAAAACATA AGCGGCAAAG ACCATCGTGA AAATCGGAAT GGCTGCCTGA 28750

ATAATTCCCG CTTCAGAGGA GGACGAGTAC ACAAGGCCGA ATGCCTGAAA 28800

GCTGAAAAAT AACGCGGGAT ACAGCAGGGC GAGCGGCAAA ATGTCAATGA 28850

(Table 3 cont.)

```
CGTCCTTTAC GAGGATTGAT AGCTTTACCC AGCCGAATAA GATCGGTACA   28900
GTGGCCGCAG CAAACGCAAT GGTGAACCGA TGCGCCAAAA TATCAAACGG   28950
CTCTGCTGTT TGCAGTGCGA TTTTTACGAA TAGAAAGGAT AAACCGATAA   29000
TGAACGAATA TAAAATAGCC GCTATATAAG CGGGGGTTTG CTGATGTTTA   29050
ORF7 <——
ACCATAATGG GAAGGGCTCC TTTACCTGAA TTGCAGCGCC GGTCGCTCCC   29100
TTTATTGTAT GGCCGCGGTC AGAACGGTAC AATGAGAAAA ACAATGAACT   29150
                                 ——> ORF8-
GTACCGGTAC AAAACAGGGG GAGAAGGCAT GGAGAAATAT ATGAGTCTAT   29200
TAACGAGGAT AGAGGAGATG ATGCAAAGCA CCTCATATCA AGAAGGAGAC   29250
AGGCTTCCAT CTATCCGTCA GCTGTCCGCC CGCTACCAAG TCAGCAAAAG   29300
CACAGTGATC CGCGCGCTGC AGGAGCTGGA AAAGCGCCAC CTTATCTATT   29350
CTGTTCCGAA AAGCGGCTAT TATATTGTGA AAAAGTCAGG GAAATCAAAA   29400
AGCGGGCAGC CTGGCCCCAT CGACTTTGCC ACATCTGCGC CGGATCCCGA   29450
TGTGTTTCCG TATCTTGATT TTCAGCACTG CATCAACAAA GCGATTGATA   29500
CATACAAAAA CGATTTGTTT ATTTATGGGA CGCCAAAGGG GCTTCCATCA   29550
CTCATCCGCG TACTCCGAAA GCTCTTGGCC ACTCAACAGG TATTTGCGGA   29600
TGAACGGCAT ATTTTCATTA CATCAGGTGT CCAGCAGGCG TTATCCTTGC   29650
TTTGTGCCAT GCCGTTCCCA AATGGGAAAG AGAAGATCGC CATTGAACAG   29700
CCGGGCTACC ATTTGATGGT CGAACAGCTT GAGACACTTG GGATTCCCGC   29750
CATCGGGGTG AAACGAACGG AAGAAGGGCT TGATATAGCC AAGGTTGAGC   29800
GGTTATTTCA AACAGAATCG ATTAAATTTT TTTATACGAT GCCGCGCTTC   29850
CATAACCCGC TTGGGTGCTC ATTGTCAGAG CGTGATAAAC AGGAGCTTGT   29900
GAGACTGGCA GAAGCGTATG ATGTCTATCT CGTTGAGGAT GATTACCTCG   29950
GTGATCTGGA GGAAAATAAA AAGGCAGATC CGCTGTACGC ATATGATCTG   30000
TCCTCACATG TCATCTATTT GAAAAGCTTC TCGAAAATGA TGTTCCCCGG   30050
CCTCCGCGTG GGGGCGGCTG TTTTGCCCGA AGCGCTGACT GACACGTTCT   30100
ATGCGTACAA AAAGCTGAAC GACATCGACT GTTCGATGAT TTCTCAAGCG   30150
GCATTGGAGA TTTACCTGAA AAGCGGTATG TACGGCAGGC ATAAGGAGAA   30200
AATCAGAGAT TCTTATAAAG AGCGGTCGCT GAGGCTACAT CAAGCCATTC   30250
GAACTCACAG GCAGCTGGGA AGCGGACGCT TTACGTTCTC CAGCGGGCAG   30300
GCACCCTGTA TGCACACCCA TCTGGTGCTT CCTCAGGATC TGCCCGCCTC   30350
```

(Table 3 cont.)

| | | | | | |
|---|---|---|---|---|---|
| AAGAGTGATT | CATAGACTGG | AAAAACAAGG | GGTTCTCCTT | GAGGCGATAG | 30400 |
| ACCGTCATTA | TTTATCAGAT | TATCCGAAAG | AAAATCTATT | AAAAATCAAT | 30450 |
| ATTTCCAATG | TGAAAACGGA | AGATATTGAG | CGCGGTGTCA | AGCTGTTGAT | 30500 |
| | STOP ORF8 | | | | |
| GAGCCATTTA | TAA | | | | 30513 |

It is interesting to note that about one half of the srfA operon is also important in B.subtilis for manifesting the "competence" character, ie the ability of the cell to incorporate DNA from the extracellular medium. The first ORF (ORF1) is formed from 10767 bases (from nucleotide +1 to nucleotide 10767). The ATG start codon, which encodes the first methionine, is preceded by a typical ribosomal attachment sequence (Shine Dalgarno (SD)) with a GGGAGG sequence 9 bases distant from the ATG codon.

ORF1 codes for a protein of 3588 amino acids (with a molecular weight of 402091 daltons) having the amino acid sequence shown in Table 4, in which three repeated amino acid sequences also found in ORF2 and ORF3 are underlined.

TABLE 4

| | | | | | | | | | | | | | | | |
|---|---|---|---|---|---|---|---|---|---|---|---|---|---|---|---|
| Met | Glu | Ile | Thr | Phe | Tyr | Pro | Leu | Thr | Asp | Ala | Gln | Lys | Arg | Ile | 15 |
| Trp | Tyr | Thr | Glu | Lys | Phe | Tyr | Pro | His | Thr | Ser | Ile | Ser | Asn | Leu | 30 |
| Ala | Gly | Ile | Gly | Lys | Leu | Val | Ser | Ala | Asp | Ala | Ile | Asp | Tyr | Val | 45 |
| Leu | Val | Glu | Gln | Ala | Ile | Gln | Glu | Phe | Ile | Arg | Arg | Asn | Asp | Ala | 60 |
| Met | Arg | Leu | Arg | Leu | Arg | Leu | Asp | Glu | Asn | Gly | Glu | Pro | Val | Gln | 75 |
| Tyr | Ile | Ser | Glu | Tyr | Arg | Pro | Val | Asp | Ile | Lys | His | Thr | Asp | Thr | 90 |
| Thr | Glu | Asp | Pro | Asn | Ala | Ile | Glu | Phe | Ile | Ser | Gln | Trp | Ser | Arg | 105 |
| Glu | Glu | Thr | Lys | Lys | Pro | Leu | Pro | Leu | Tyr | Asp | Cys | Asp | Leu | Phe | 120 |
| Arg | Phe | Ser | Leu | Phe | Thr | Ile | Lys | Glu | Asn | Glu | Val | Trp | Phe | Tyr | 135 |
| Ala | Asn | Val | <u>His</u> | <u>His</u> | <u>Val</u> | <u>Ile</u> | <u>Ser</u> | <u>Asp</u> | <u>Gly</u> | Met | Ser | Met | Asn | Ile | 150 |
| Val | Gly | Asn | Ala | Ile | Met | His | Ile | Tyr | Leu | Glu | Leu | Ala | Ser | Gly | 165 |
| Ser | Glu | Thr | Lys | Glu | Gly | Ile | Ser | His | Ser | Phe | Ile | Asp | His | Val | 180 |
| Leu | Ser | Glu | Gln | Glu | Tyr | Ala | Gln | Ser | Lys | Arg | Phe | Glu | Lys | Asp | 195 |
| Lys | Ala | Phe | Trp | Asn | Lys | Gln | Phe | Glu | Ser | Val | Pro | Glu | Leu | Val | 210 |
| Ser | Leu | Lys | Arg | Asn | Ala | Ser | Ala | Gly | Gly | Ser | Leu | Asp | Ala | Glu | 225 |
| Arg | Phe | Ser | Lys | Asp | Val | Pro | Glu | Ala | Leu | His | Gln | Gln | Ile | Leu | 240 |
| Ser | Phe | Cys | Glu | Ala | Asn | Lys | Val | Ser | Val | Leu | Ser | Val | Phe | Gln | 255 |
| Ser | Leu | Leu | Ala | Ala | Tyr | Leu | Tyr | Arg | Val | Ser | Gly | Gln | Asn | Asp | 270 |
| Val | Val | Thr | Gly | Thr | Phe | Met | Gly | Asn | Arg | Gln | Asn | Ala | Lys | Glu | 285 |
| Lys | Gln | Met | Leu | Gly | Met | Phe | Val | Ser | Thr | Val | Pro | Leu | Arg | Thr | 300 |
| Asn | Ile | Asp | Gly | Gly | Gln | Ala | Phe | Ser | Glu | Phe | Val | Lys | Asp | Arg | 315 |
| Met | Lys | Asp | Leu | Met | Lys | Thr | Leu | Arg | His | Gln | Lys | Tyr | Pro | Tyr | 330 |
| Asn | Leu | Leu | Ile | Asn | Asp | Leu | Arg | Glu | Thr | Lys | Ser | Ser | Leu | Thr | 345 |
| Lys | Leu | Phe | Thr | Val | Ser | Leu | Glu | Tyr | Gln | Val | Met | Gln | Trp | Gln | 360 |
| Lys | Glu | Glu | Asp | Leu | Ala | Phe | Leu | Thr | Glu | Pro | Ile | Phe | Ser | Gly | 375 |
| Ser | Gly | Leu | Asn | Asp | Val | Ser | Ile | His | Val | Lys | Asp | Arg | Trp | Asp | 390 |
| Thr | Gly | Lys | Leu | Thr | Ile | Asp | Phe | Asp | Tyr | Arg | Thr | Asp | Leu | Phe | 405 |
| Ser | Arg | Glu | Glu | Ile | Asn | Met | Ile | Cys | Glu | Arg | Met | Ile | Thr | Met | 420 |
| Leu | Glu | Asn | Ala | Leu | Thr | His | Pro | Glu | His | Thr | Ile | Asp | Glu | Leu | 435 |
| Thr | Leu | Ile | Ser | Asp | Ala | Glu | Lys | Glu | Lys | Leu | Leu | Ala | Arg | Ala | 450 |
| Gly | Gly | Lys | Ser | Val | Ser | Tyr | Arg | Lys | Asp | Met | Thr | Ile | Pro | Glu | 465 |
| Leu | Phe | Gln | Glu | Lys | Ala | Glu | Leu | Leu | Ser | Asp | His | Pro | Ala | Val | 480 |

(Table 4 cont.)

Val Phe Glu Asp Arg Thr Leu Ser Tyr Arg Thr Leu His Glu Gln 495
Ser Ala Arg Ile Ala Asn Val Leu Lys Gln Lys Gly Val Gly Pro 510
Asp Ser Pro Val Ala Val Leu Ile Glu Arg Ser Glu Arg Met Ile 525
Thr Ala Ile Met Gly Ile Leu Lys Ala Gly Gly Ala Tyr Val Ala 540
Ile Asp Pro Gly Phe Pro Ala Glu Arg Ile Gln Tyr Ile Leu Glu 555
Asp Cys Gly Ala Asp Phe Tyr Leu Thr Glu Ser Lys Val Ala Ala 570
Pro Glu Ala Asp Ala Glu Leu Ile Asp Leu Asp Gln Ala Ile Glu 585
Glu Gly Ala Glu Glu Ser Leu Asn Ala Asp Val Asn Ala Arg Asn 600
Leu Ala Tyr Ile Ile Tyr Thr Ser Gly Thr Thr Gly Arg Pro Lys 615
Gly Val Met Ile Glu His Arg Gln Val His His Leu Val Glu Ser 630
Leu Gln Gln Thr Ile Tyr Gln Ser Pro Thr Gln Thr Leu Pro Met 645
Ala Phe Leu Pro Pro Phe His Phe Asp Ala Ser Val Lys Gln Ile 660
Phe Ala Ser Leu Leu Leu Gly Gln Thr Leu Tyr Ile Val Pro Lys 675
Lys Thr Val Thr Asn Gly Ala Ala Leu Thr Ala Tyr Tyr Arg Lys 690
Asn Ser Ile Glu Ala Thr Asp Gly Thr Pro Ala His Leu Gln Met 705
Leu Ala Ala Ala Gly Asp Phe Glu Gly Leu Lys Leu Lys His Met 720
Leu Ile Gly Gly Glu Gly Leu Ser Ser Val Val Ala Asp Lys Leu 735
Leu Lys Leu Phe Lys Glu Ala Gly Thr Ala Pro Arg Leu Thr Asn 750
Val Tyr Gly Pro Thr Glu Thr Cys Val Asp Ala Ser Val His Pro 765
Val Ile Pro Glu Asn Ala Val Gln Ser Ala Tyr Val Pro Ile Gly 780
Lys Ala Leu Gly Asn Asn Arg Leu Tyr Ile Leu Asp Gln Lys Gly 795
Arg Leu Gln Pro Glu Gly Val Ala Gly Glu Leu Tyr Ile Ala Gly 810
Asp Gly Val Gly Arg Gly Tyr Leu His Leu Pro Glu Leu Thr Glu 825
Glu Lys Phe Leu Gln Asp Pro Phe Val Pro Gly Asp Arg Met Tyr 840
Arg Thr Gly Asp Val Val Arg Trp Leu Pro Asp Gly Thr Ile Glu 855
Tyr Leu Gly Arg Glu Asp Asp Gln Val Lys Val Arg Gly Tyr Arg 870
Ile Glu Leu Gly Glu Ile Glu Ala Val Ile Gln Gln Ala Pro Asp 885
Val Ala Lys Ala Val Val Leu Ala Arg Pro Asp Glu Gln Gly Asn 900
Leu Glu Val Cys Ala Tyr Val Val Gln Lys Pro Gly Ser Glu Phe 915
Ala Pro Ala Gly Leu Arg Glu His Ala Ala Arg Gln Leu Pro Asp 930
Tyr Met Val Pro Ala Tyr Phe Thr Glu Val Thr Glu Ile Pro Leu 945
Thr Pro Ser Gly Lys Val Asp Arg Arg Lys Leu Phe Ala Leu Glu 960

(Table 4 cont.)

| | |
|---|---|
| Val Lys Ala Val Ser Gly Thr Ala Tyr Thr Ala Pro Arg Asn Glu | 975 |
| Thr Glu Lys Ala Ile Ala Ala Ile Trp Gln Asp Val Leu Asn Val | 990 |
| Glu Lys Ala Gly Ile Phe Asp Asn Phe Phe Glu Thr Gly Gly His | 1005 |
| Ser Leu Lys Ala Met Thr Leu Leu Thr Lys Ile His Lys Glu Thr | 1020 |
| Gly Ile Glu Ile Pro Gln Gln Phe Leu Phe Glu His Pro Thr Ile | 1035 |
| Thr Ala Leu Ala Glu Glu Ala Asp His Arg Glu Ser Lys Ala Phe | 1050 |
| Ala Val Ile Glu Pro Ala Glu Lys Gln Glu His Tyr Pro Leu His | 1065 |
| Trp His Ser Ser Glu His Ile Ser Ser Ala Ser Ser Arg Met Arg | 1080 |
| Glu Ser Ala Ile His Ala Ser Ser Ser Asn Ser Glu Gly Phe Arg | 1095 |
| Tyr Ser Lys Ala Gly Ala Arg Ile Ser Gly Ile Asn Pro Thr Pro | 1110 |
| Arg Val Ile Glu Thr Ser Phe Val Leu Glu Asn Ser Thr Pro Arg | 1125 |
| Gln Lys Ile His Val Cys Val Asp Phe Asn Ile Glu Met Ile Glu | 1140 |
| Arg Gly Gly Arg Ser Asp Glu Ala Ile Met Ala Ser Phe Val Arg | 1155 |
| Thr Phe Asp Leu Ala Lys Ala Pro Leu Phe Arg Ile Gly Leu Leu | 1170 |
| Gly Leu Glu Glu Asn Arg His Met Leu Leu Phe Asp Met His His | 1185 |
| Leu Ile Ser Asp Gly Val Ser Ile Gly Ile Met Leu Glu Glu Leu | 1200 |
| Ala Arg Ile Tyr Lys Gly Glu Gln Leu Pro Asp Leu Arg Leu Gln | 1215 |
| Tyr Lys Asp Tyr Ala Val Trp Gln Ser Arg Gln Ala Ala Glu Gly | 1230 |
| Tyr Lys Lys Asp Gln Ala Tyr Trp Lys Glu Val Phe Ala Gly Glu | 1245 |
| Leu Pro Val Leu Gln Leu Leu Ser Asp Tyr Pro Arg Pro Pro Val | 1260 |
| Gln Ser Phe Glu Gly Asp Arg Val Ser Ile Lys Leu Asp Ala Gly | 1275 |
| Val Lys Asp Arg Leu Asn Arg Leu Ala Glu Gln Asn Gly Ala Thr | 1290 |
| Leu Tyr Met Val Met Leu Ser Ala Tyr Tyr Thr Leu Leu Ser Lys | 1305 |
| Tyr Thr Gly Gln Asp Asp Ile Ile Val Gly Thr Pro Ser Ala Gly | 1320 |
| Arg Asn His Ser Asp Thr Glu Gly Ile Ile Gly Met Phe Val Asn | 1335 |
| Thr Leu Ala Ile Arg Ser Glu Val Lys Gln Asn Glu Thr Phe Thr | 1350 |
| Gln Leu Ile Ser Arg Val Arg Lys Arg Val Leu Asp Ala Phe Ser | 1365 |
| His Gln Asp Tyr Pro Phe Glu Trp Leu Val Glu Asp Leu Asn Ile | 1380 |
| Pro Arg Asp Val Ser Arg His Pro Leu Phe Asp Thr Met Phe Ser | 1395 |
| Leu Gln Asn Ala Thr Glu Gly Ile Pro Ala Val Gly Asp Leu Ser | 1410 |
| Leu Ser Val Gln Glu Thr Asn Phe Lys Ile Ala Lys Phe Asp Leu | 1425 |
| Thr Val Gln Ala Arg Glu Thr Asp Glu Gly Ile Glu Ile Asp Val | 1440 |

(Table 4 cont.)

| | | | | | | | | | | | | | | | |
|---|---|---|---|---|---|---|---|---|---|---|---|---|---|---|---|
| Asp | Tyr | Ser | Thr | Lys | Leu | Phe | Lys | Gln | Ser | Thr | Ala | Asp | Arg | Leu | 1455 |
| Leu | Thr | His | Phe | Ala | Arg | Leu | Leu | Glu | Asp | Ala | Ala | Ala | Asp | Pro | 1470 |
| Glu | Lys | Pro | Ile | Ser | Glu | Tyr | Lys | Leu | Leu | Ser | Glu | Glu | Glu | Ala | 1485 |
| Ala | Ser | Gln | Ile | Gln | Gln | Phe | Asn | Pro | Gly | Arg | Thr | Pro | Tyr | Pro | 1500 |
| Lys | Asp | Lys | Thr | Ile | Val | Gln | Leu | Phe | Glu | Glu | Gln | Ala | Ala | Asn | 1515 |
| Thr | Pro | Asp | His | Thr | Ala | Leu | Gln | Tyr | Glu | Gly | Glu | Ser | Leu | Thr | 1530 |
| Tyr | Arg | Glu | Leu | Asn | Glu | Arg | Ala | Asn | Arg | Leu | Ala | Arg | Gly | Ile | 1545 |
| Leu | Ser | Leu | Gly | Ala | Gly | Glu | Gly | Arg | Thr | Ala | Ala | Val | Leu | Cys | 1560 |
| Glu | Arg | Ser | Met | Asp | Met | Ile | Val | Ser | Ile | Leu | Ala | Val | Leu | Lys | 1575 |
| Ser | Gly | Ser | Ala | Tyr | Val | Pro | Ile | Asp | Pro | Glu | His | Pro | Ile | Gln | 1590 |
| Arg | Met | Gln | His | Phe | Phe | Arg | Asp | Ser | Gly | Ala | Lys | Val | Leu | Leu | 1605 |
| Thr | Gln | Arg | Lys | Leu | Lys | Ala | Leu | Ala | Glu | Glu | Ala | Glu | Phe | Lys | 1620 |
| Gly | Val | Ile | Val | Leu | Ala | Asp | Glu | Glu | Glu | Ser | Tyr | His | Ala | Asp | 1635 |
| Ala | Arg | Asn | Leu | Ala | Leu | Pro | Leu | Asp | Ser | Ala | Ala | Met | Ala | Asn | 1650 |
| Leu | Thr | Tyr | Thr | Ser | Gly | Thr | Thr | Gly | Thr | Pro | Lys | Gly | Asn | Ile | 1665 |
| Val | Thr | His | Ala | Asn | Ile | Leu | Arg | Thr | Val | Lys | Glu | Thr | Asn | Tyr | 1680 |
| Leu | Ser | Ile | Thr | Glu | Gln | Asp | Thr | Ile | Leu | Gly | Leu | Ser | Asn | Tyr | 1695 |
| Val | Phe | Asp | Ala | Phe | Met | Phe | Asp | Met | Phe | Gly | Ser | Leu | Leu | Asn | 1710 |
| Gly | Ala | Lys | Leu | Val | Leu | Ile | Pro | Lys | Glu | Thr | Val | Leu | Asp | Met | 1725 |
| Ala | Arg | Leu | Ser | Arg | Val | Ile | Glu | Arg | Glu | Asn | Ile | Ser | Ile | Leu | 1740 |
| Met | Ile | Thr | Thr | Ala | Leu | Phe | His | Leu | Leu | Val | Asp | Leu | Asn | Pro | 1755 |
| Ala | Cys | Leu | Ser | Thr | Leu | Arg | Lys | Ile | Met | Phe | Gly | Gly | Glu | Arg | 1770 |
| Ala | Ser | Val | Glu | His | Val | Arg | Lys | Ala | Leu | Gln | Thr | Val | Gly | Lys | 1785 |
| Gly | Lys | Leu | Leu | His | Met | Tyr | Gly | Pro | Ser | Glu | Ser | Thr | Val | Phe | 1800 |
| Ala | Thr | Tyr | His | Pro | Val | Asp | Glu | Leu | Glu | Glu | His | Thr | Leu | Ser | 1815 |
| Val | Pro | Ile | Gly | Lys | Pro | Val | Ser | Asn | Thr | Glu | Val | Tyr | Ile | Leu | 1830 |
| Asp | Arg | Thr | Gly | His | Val | Gln | Pro | Ala | Gly | Ile | Ala | Gly | Glu | Leu | 1845 |
| Cys | Val | Thr | Gly | Glu | Gly | Leu | Val | Lys | Gly | Tyr | Tyr | Asn | Arg | Pro | 1860 |
| Glu | Leu | Thr | Glu | Glu | Lys | Phe | Val | Pro | His | Pro | Phe | Thr | Ser | Gly | 1875 |
| Glu | Arg | Met | Tyr | Lys | Thr | Gly | Asp | Leu | Ala | Arg | Trp | Leu | Pro | Asn | 1890 |
| Gly | Thr | Ile | Glu | Phe | Ile | Gly | Arg | Ile | Asp | His | Gln | Val | Lys | Ile | 1905 |
| Arg | Gly | Gln | Ala | Ile | Glu | Leu | Gly | Glu | Ile | Glu | His | Gln | Leu | Gln | 1920 |

(Table 4 cont.)

Thr His Asp Arg Val Gln Glu Ser Val Val Leu Ala Val Asp Gln 1935
Gly Ala Gly Asp Lys Leu Leu Cys Ala Tyr Tyr Val Gly Glu Gly 1950
Asp Ile Ser Ser Gln Glu Met Arg Glu His Ala Ala Lys Asp Leu 1965
Pro Ala Tyr Met Val Pro Ala Val Phe Ile Gln Met Asp Glu Leu 1980
Pro Leu Thr Gly Asn Gly Lys Ile Asp Arg Arg Ala Leu Pro Ile 1995
Pro Asp Ala Asn Val Ser Arg Gly Val Ser Tyr Val Ala Pro Arg 2010
Asn Gly Thr Glu Gln Lys Val Ala Asp Ile Trp Ala Gln Val Leu 2025
Gln Ala Glu Gln Val Gly Ala Tyr Asp His Phe Phe Asp Ile Gly 2040
Gly His Ser Leu Ala Gly Met Lys Met Pro Ala Leu Val His Gln 2055
Glu Leu Gly Val Glu Leu Ser Leu Lys Asp Leu Phe Gln Ser Pro 2070
Thr Val Glu Gly Leu Ala Gln Val Ile Ala Ser Ala Glu Lys Gly 2085
Thr Ala Ala Ser Ile Ser Pro Ala Glu Lys Gln Asp Thr Tyr Pro 2100
Val Ser Ser Pro Gln Lys Arg Met Tyr Val Leu Gln Gln Leu Glu 2115
Asp Ala Gln Thr Ser Tyr Asn Met Pro Ala Val Leu Arg Leu Thr 2130
Gly Glu Leu Asp Val Glu Arg Leu Asn Ser Val Met Gln Gln Leu 2145
Met Gln Arg His Glu Ala Leu Arg Thr Thr Phe Glu Ile Lys Asp 2160
Gly Glu Thr Val Gln Arg Ile Trp Glu Glu Ala Glu Cys Glu Ile 2175
Ala Tyr Phe Glu Ala Pro Glu Glu Glu Thr Glu Arg Ile Val Ser 2190
Glu Phe Ile Lys Pro Phe Lys Ile Asp Gln Leu Pro Leu Phe Arg 2205
Ile Gly Leu Ile Lys His Ser Asp Thr Glu Gln Val Leu Leu Phe 2220
Asp Met His His Ile Ile Ser Asp Gly Ala Ser Val Gly Val Leu 2235
Ile Glu Glu Leu Ser Lys Leu Tyr Asp Gly Glu Thr Leu Glu Pro 2250
Leu Arg Ile Gln Tyr Lys Asp Tyr Ala Val Trp Gln His Arg Phe 2265
Ile Gln Ser Glu Leu Tyr Lys Lys Gln Glu Glu His Trp Leu Lys 2280
Glu Leu Asp Gly Glu Leu Pro Val Leu Thr Leu Pro Thr Asp Tyr 2295
Ser Arg Pro Ala Val Gln Thr Phe Glu Gly Asp Arg Ile Ala Phe 2310
Ser Leu Glu Ala Gly Lys Ala Asp Ala Leu Arg Arg Leu Ala Lys 2325
Glu Thr Asp Ser Thr Leu Tyr Met Val Leu Leu Ala Ser Tyr Ser 2340
Ala Phe Leu Ser Lys Ile Cys Gly Gln Asp Asp Ile Ile Val Gly 2355
Ser Pro Val Ala Gly Arg Ser Gln Ala Asp Val Ser Arg Val Ile 2370
Gly Met Phe Val Asn Thr Leu Ala Leu Arg Thr Tyr Pro Lys Gly 2385
Glu Lys Thr Phe Ala Asp Tyr Leu Asn Glu Val Lys Glu Thr Ala 2400

(Table 4 cont.)

| | | | | | | | | | | | | | | | |
|---|---|---|---|---|---|---|---|---|---|---|---|---|---|---|---|
| Leu | Ser | Ala | Phe | Asp | Ala | Gln | Asp | Tyr | Pro | Leu | Glu | Asp | Leu | Ile | 2415 |
| Gly | Asn | Val | Gln | Val | Gln | Arg | Asp | Thr | Ser | Ser | Asn | Pro | Leu | Phe | 2430 |
| Asp | Ala | Val | Phe | Ser | Met | Gln | Asn | Ala | Asn | Ile | Lys | Asp | Leu | Thr | 2445 |
| Met | Lys | Gly | Ile | Gln | Leu | Glu | Pro | His | Pro | Phe | Glu | Arg | Lys | Thr | 2460 |
| Ala | Lys | Phe | Asp | Leu | Thr | Leu | Thr | Ala | Asp | Glu | Thr | Asp | Gly | Gly | 2475 |
| Leu | Thr | Phe | Val | Leu | Glu | Tyr | Asn | Thr | Ala | Leu | Phe | Lys | Gln | Glu | 2490 |
| Thr | Ile | Glu | Arg | Trp | Lys | Gln | Tyr | Trp | Met | Glu | Leu | Leu | Asp | Ala | 2505 |
| Val | Thr | Gly | Asn | Pro | Asn | Gln | Pro | Leu | Ser | Ser | Leu | Ser | Leu | Val | 2520 |
| Thr | Glu | Thr | Glu | Lys | Gln | Ala | Leu | Leu | Glu | Ala | Trp | Lys | Gly | Lys | 2535 |
| Ala | Leu | Pro | Val | Pro | Thr | Asp | Lys | Thr | Val | His | Gln | Leu | Phe | Glu | 2550 |
| Glu | Thr | Ala | Gln | Arg | His | Lys | Asp | Arg | Pro | Ala | Val | Thr | Tyr | Asn | 2565 |
| Gly | Gln | Ser | Trp | Thr | Tyr | Gly | Glu | Leu | Asn | Ala | Lys | Ala | Asn | Arg | 2580 |
| Leu | Ala | Arg | Ile | Leu | Met | Asp | Cys | Gly | Ile | Ser | Pro | Asp | Asp | Arg | 2595 |
| Val | Gly | Val | Leu | Thr | Lys | Pro | Ser | Leu | Glu | Met | Ser | Ala | Ala | Val | 2610 |
| Leu | Gly | Val | Leu | Lys | Ala | Gly | Ala | Ala | Phe | Val | Pro | Ile | Asp | Pro | 2625 |
| Asp | Tyr | Pro | Asp | Gln | Arg | Ile | Glu | Tyr | Ile | Leu | Gln | Asp | Ser | Gly | 2640 |
| Ala | Lys | Leu | Leu | Leu | Lys | Gln | Glu | Gly | Ile | Ser | Val | Pro | Asp | Ser | 2655 |
| Tyr | Thr | Gly | Asp | Val | Ile | Leu | Leu | Asp | Gly | Ser | Arg | Thr | Ile | Leu | 2670 |
| Ser | Leu | Pro | Leu | Asp | Glu | Asn | Asp | Glu | Glu | Asn | Pro | Glu | Asn | Pro | 2685 |
| Glu | Thr | Ala | Val | Thr | Ala | Glu | Asn | Leu | Ala | Tyr | Met | Ile | Tyr | Thr | 2700 |
| Ser | Gly | Thr | Thr | Gly | Gln | Pro | Lys | Gly | Val | Met | Val | Glu | His | His | 2715 |
| Ala | Leu | Val | Asn | Leu | Cys | Phe | Trp | His | His | Asp | Ala | Phe | Ser | Met | 2730 |
| Thr | Ala | Glu | Asp | Arg | Ser | Ala | Lys | Tyr | Ala | Gly | Phe | Gly | Phe | Asp | 2745 |
| Ala | Ser | Ile | Trp | Glu | Met | Phe | Pro | Thr | Trp | Ser | Ile | Gly | Ala | Glu | 2760 |
| Leu | His | Val | Ile | Glu | Glu | Ala | Ile | Arg | Leu | Asp | Ile | Val | Arg | Leu | 2775 |
| Asn | Asp | Tyr | Phe | Glu | Thr | Asn | Gly | Val | Thr | Ile | Thr | Phe | Leu | Pro | 2790 |
| Thr | Gln | Leu | Ala | Glu | Gln | Phe | Met | Glu | Leu | Glu | Asn | Thr | Ser | Leu | 2805 |
| Arg | Val | Leu | Leu | Thr | Gly | Gly | Asp | Lys | Leu | Lys | Arg | Ala | Val | Lys | 2820 |
| Lys | Pro | Tyr | Thr | Leu | Val | Asn | Asn | Tyr | Gly | Pro | Thr | Glu | Asn | Thr | 2835 |
| Val | Val | Ala | Thr | Ser | Ala | Glu | Ile | His | Pro | Glu | Glu | Gly | Ser | Leu | 2850 |
| Ser | Ile | Gly | Arg | Ala | Ile | Ala | Asn | Thr | Arg | Val | Tyr | Ile | Leu | Gly | 2865 |
| Glu | Gly | Asn | Gln | Val | Gln | Pro | Glu | Gly | Val | Ala | Gly | Glu | Leu | Cys | 2880 |

(Table 4 cont.)

| | | | | | | | | | | | | | | | |
|---|---|---|---|---|---|---|---|---|---|---|---|---|---|---|---|
| Val | Ala | Gly | Arg | Gly | Leu | Ala | Arg | Gly | Tyr | Leu | Asn | Arg | Glu | Asp | 2895 |
| Glu | Thr | Ala | Lys | Arg | Phe | Val | Ala | Asp | Pro | Phe | Val | Pro | Gly | Glu | 2910 |
| Arg | Met | Tyr | Arg | Thr | Gly | Asp | Leu | Val | Lys | Trp | Thr | Gly | Gly | Gly | 2925 |
| Ile | Glu | Tyr | Ile | Gly | Arg | Ile | Asp | Gln | Gln | Val | Lys | Val | Arg | Gly | 2940 |
| Tyr | Arg | Ile | Glu | Leu | Ser | Glu | Ile | Glu | Val | Gln | Leu | Ala | Gln | Leu | 2955 |
| Ser | Glu | Val | Gln | Asp | Ala | Ala | Val | Thr | Ala | Val | Lys | Asp | Lys | Gly | 2970 |
| Gly | Asn | Thr | Ala | Ile | Ala | Ala | Tyr | Val | Thr | Pro | Glu | Ser | Ala | Asp | 2985 |
| Ile | Glu | Ala | Leu | Lys | Ser | Ala | Leu | Lys | Glu | Thr | Leu | Pro | Asp | Tyr | 3000 |
| Met | Ile | Pro | Ala | Phe | Trp | Val | Thr | Leu | Asn | Glu | Leu | Pro | Val | Thr | 3015 |
| Ala | Asn | Gly | Lys | Val | Asp | Arg | Lys | Ala | Leu | Asn | Glu | Pro | Asp | Ile | 3030 |
| Glu | Ala | Gly | Ser | Gly | Glu | Tyr | Lys | Ala | Pro | Thr | Thr | Asp | Met | Glu | 3045 |
| Glu | Leu | Leu | Ala | Gly | Ile | Trp | Gln | Asp | Val | Leu | Gly | Met | Ser | Glu | 3060 |
| Val | Gly | Val | Thr | Asp | Asn | Phe | Phe | Ser | Leu | Gly | Gly | Asp | Ser | Ile | 3075 |
| Lys | Gly | Ile | Gln | Met | Ala | Ser | Arg | Leu | Asn | Gln | His | Gly | Trp | Lys | 3090 |
| Leu | Glu | Met | Lys | Asp | Leu | Asn | Gln | His | Pro | Thr | Ile | Glu | Glu | Leu | 3105 |
| Thr | Gln | Tyr | Val | Glu | Arg | Ala | Glu | Gly | Lys | Gln | Ala | Asp | Gln | Gly | 3120 |
| Pro | Val | Glu | Gly | Glu | Val | Ile | Leu | Thr | Pro | Ile | Gln | Arg | Trp | Phe | 3135 |
| Phe | Glu | Lys | Asn | Phe | Thr | Asn | Lys | His | His | Trp | Asn | Gln | Ser | Val | 3150 |
| Met | Leu | His | Ala | Lys | Lys | Gly | Phe | Asp | Pro | Glu | Arg | Val | Glu | Lys | 3165 |
| Thr | Leu | Gln | Ala | Leu | Ile | Glu | His | His | Asp | Ala | Leu | Arg | Met | Val | 3180 |
| Tyr | Arg | Glu | Gly | Gln | Glu | Asp | Val | Ile | Gln | Tyr | Asn | Arg | Gly | Leu | 3195 |
| Glu | Ala | Ala | Ser | Ala | Gln | Leu | Glu | Val | Ile | Gln | Ile | Glu | Gly | Gln | 3210 |
| Ala | Ala | Asp | Tyr | Glu | Asp | Arg | Ile | Glu | Arg | Glu | Ala | Glu | Arg | Leu | 3225 |
| Gln | Ser | Ser | Ile | Asp | Leu | Gln | Glu | Gly | Gly | Leu | Leu | Lys | Ala | Gly | 3240 |
| Leu | Phe | Gln | Ala | Glu | Asp | Gly | Asp | His | Leu | Leu | Leu | Ala | Ile | His | 3255 |
| His | Leu | Val | Val | Asp | Gly | Val | Ser | Trp | Arg | Ile | Leu | Leu | Glu | Asp | 3270 |
| Phe | Ser | Ala | Val | Tyr | Thr | Gln | Leu | Glu | Gln | Gly | Asn | Glu | Pro | Val | 3285 |
| Leu | Pro | Gln | Lys | Thr | His | Ser | Phe | Ala | Glu | Tyr | Ala | Glu | Arg | Leu | 3300 |
| Gln | Asp | Phe | Ala | Asn | Ser | Lys | Ala | Phe | Leu | Lys | Glu | Lys | Glu | Tyr | 3315 |
| Trp | Ser | Gln | Leu | Glu | Glu | Gln | Ala | Val | Ala | Ala | Lys | Leu | Pro | Lys | 3330 |
| Asp | Arg | Glu | Ser | Gly | Asp | Gln | Arg | Met | Lys | His | Thr | Lys | Thr | Ile | 3345 |
| Glu | Phe | Ser | Leu | Thr | Ala | Glu | Glu | Thr | Glu | Gln | Leu | Thr | Thr | Lys | 3360 |

(Table 4 cont.)

| | | | | | | | | | | | | | | | |
|---|---|---|---|---|---|---|---|---|---|---|---|---|---|---|---|
| Val | His | Glu | Ala | Tyr | His | Thr | Glu | Met | Asn | Asp | Ile | Leu | Leu | Thr | 3375 |
| Ala | Phe | Gly | Leu | Ala | Met | Lys | Glu | Trp | Thr | Gly | Gln | Asp | Arg | Val | 3390 |
| Ser | Val | His | Leu | Glu | Gly | His | Gly | Arg | Glu | Glu | Ile | Ile | Glu | Asp | 3405 |
| Leu | Thr | Ile | Ser | Arg | Thr | Val | Gly | Trp | Phe | Thr | Ser | Met | Tyr | Pro | 3420 |
| Met | Val | Leu | Asp | Met | Lys | His | Ala | Asp | Asp | Leu | Gly | Tyr | Gln | Leu | 3435 |
| Lys | Gln | Met | Lys | Glu | Asp | Ile | Arg | His | Val | Pro | Asn | Lys | Gly | Val | 3450 |
| Gly | Ser | Gly | Ile | Leu | Arg | Tyr | Leu | Thr | Ala | Pro | Glu | His | Lys | Glu | 3465 |
| Asp | Val | Ala | Phe | Ser | Ile | Gln | Pro | Asp | Val | Ser | Phe | Asn | Tyr | Leu | 3480 |
| Gly | Gln | Phe | His | Gln | Met | Ser | His | Pro | Pro | Phe | Phe | Thr | Thr | Ser | 3495 |
| His | Leu | Pro | Ser | Pro | His | Ser | Leu | Ser | Pro | Glu | Thr | Glu | Lys | Pro | 3510 |
| Asn | Ala | Leu | Asp | Val | Val | Gly | Tyr | Ile | Glu | Asn | Gly | Lys | Leu | Thr | 3525 |
| Met | Ser | Leu | Ala | Tyr | His | Ser | Leu | Glu | Phe | His | Glu | Lys | Thr | Val | 3540 |
| Gln | Thr | Phe | Ser | Asp | Ser | Phe | Lys | Ala | His | Leu | Leu | Arg | Ile | Ile | 3555 |
| Glu | His | Cys | Leu | Ser | Gln | Asp | Gly | Thr | Glu | Leu | Thr | Pro | Ser | Asp | 3570 |
| Leu | Gly | Asp | Asp | Asp | Leu | Thr | Leu | Asp | Glu | Leu | Asp | Lys | Leu | Met | 3585 |
| Glu | Ile | Phe | | | | | | | | | | | | | 3588 |

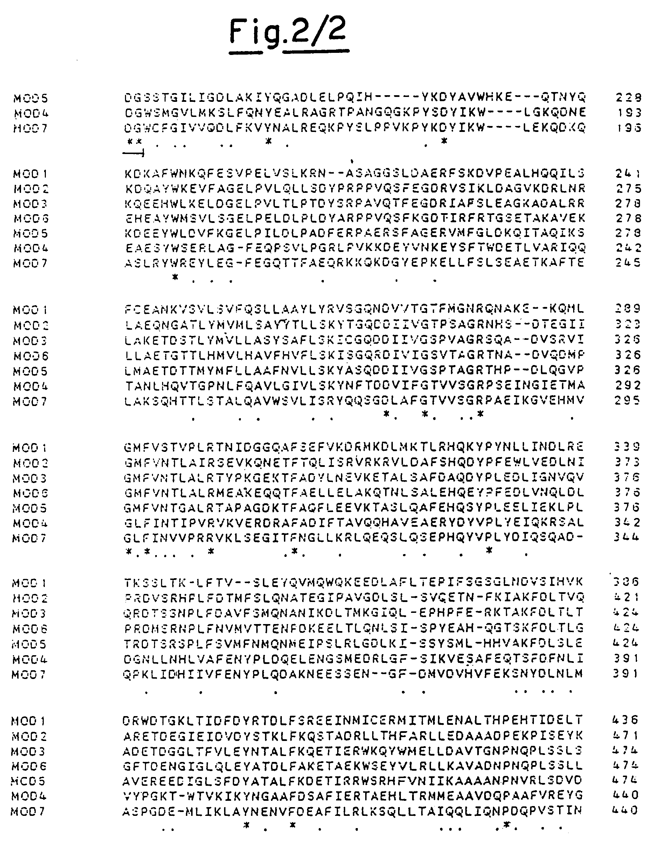

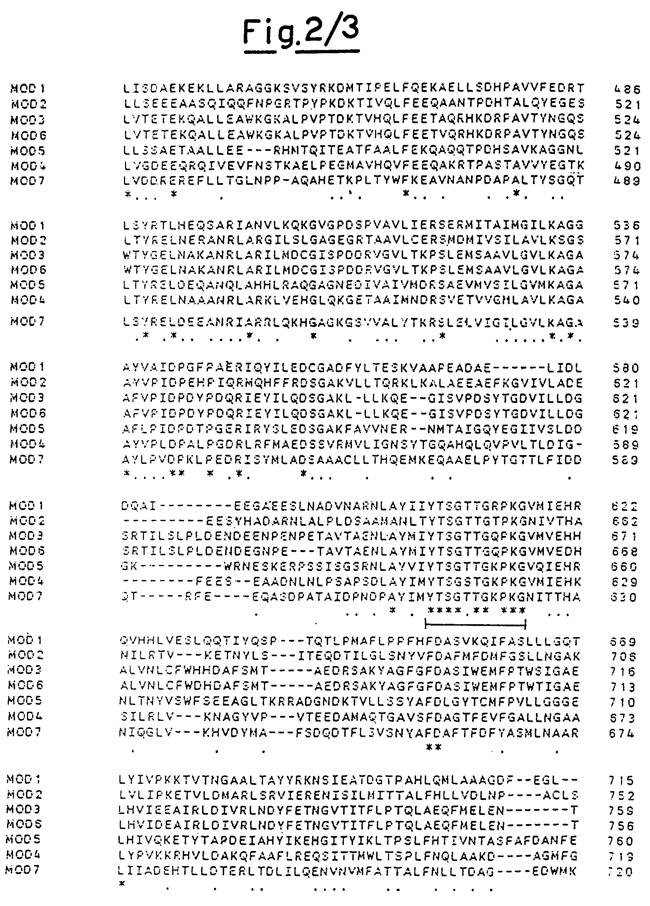

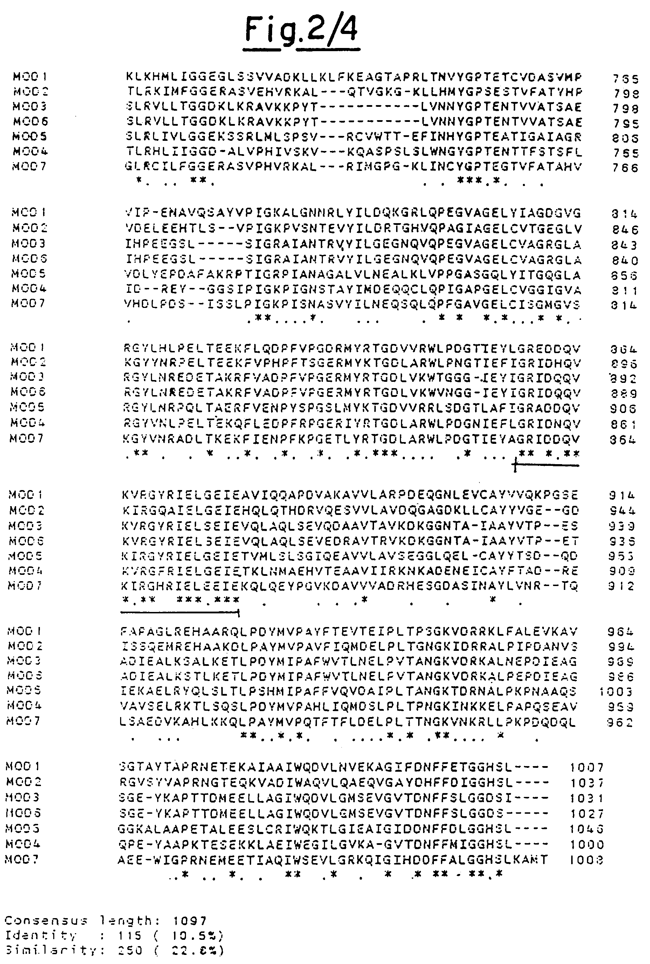

The entire amino acid sequence of the protein can be divided into 3 adjacent repeated regions (modules 1 – 3) with an internal homology of about 1000 bases, more marked in the second half of these regions (Figure 2).

At the C – terminal end of module 3 there is a region, module A (MOD A), of about 500 amino acids which has no homology with modules 1 – 3.

The second half of module 3 and module A have high homology with the synthetases coded by TycA and GrsA (tyrocidin and gramicidin synthetase subunits I) (G. Mittenhuber et al. (1989), J. Bacteriol., 171: 4881 – 4887; J. Kratzschmar et al. (1989), J. Bacteriol., 171: 5422 – 5429).

The sequence His – His – Val – Ile – Ser – Asp – Gly is repeated 4 times in the protein coded by ORF1 in position 139 – 145, 1184 – 1190, 2223 – 2229 and 3255 – 3261.

This sequence is highly conserved and shows homology with sequences found in enzymes, such as TycA and GrsA which catalyze amino acid activation.

The sequence Tyr – Thr – Ser – Gly – Tyr – Tyr – Gly – Arg – Pro – Lys – Gly is repeated three times in positions 606 – 616, 1653 – 1663 and 2698 – 2709.

Analogous homologies were found for the highly conserved sequence Gly – Arg – Glu – Asp – Asp – Gln – Val – Lys – Val – Arg – Gly – Tyr – Arg – Ile – Glu – Leu – Gly – Glu – Ile – Glu repeated three times in position 858 – 880, 1897 – 1919 and 2930 – 2949.

In addition, ORF1 has strong homology with the enzyme aminoadipyl cystein valine synthetase (ACV synthetase) which polymerizes and activates fatty acid with L – Cys and D – Val (D.J. Smith et al., (1990), EMBO J., 9: 2743 – 2750).

This enzyme also consists of three repeated units followed by a final region homologous with that of the tyrocidin and gramicidin subunits I.

The second ORE (ORF2), which is formed from 10764 bases (from nucleotide 10780 to nucleotide 21543), is separated from the ORF1 stop codon by an untranslated intergenic region of 12 bases containing a Shine – dalgarno with an AGAGGT sequence 5 bases distant from the ORF2 ATG codon (Table 3).

ORF2 codes for a protein of 3587 amino acids (molecular weight 408000 daltons) having the amino acid sequence given in Table 5.

TABLE 5

| | | | | | | | | | | | | | | | |
|---|---|---|---|---|---|---|---|---|---|---|---|---|---|---|---|
| Met | Ser | Lys | Lys | Ser | Ile | Gln | Lys | Val | Tyr | Ala | Leu | Thr | Pro | Met | 15 |
| Gln | Glu | Gly | Met | Leu | Tyr | His | Ala | Met | Leu | Asp | Pro | His | Ser | Ser | 30 |
| Ser | Tyr | Ser | Thr | Gln | Leu | Glu | Leu | Gly | Ile | His | Ala | Ala | Phe | Asp | 45 |
| Leu | Glu | Ile | Phe | Glu | Lys | Ser | Val | Asn | Glu | Leu | Ile | Arg | Ser | Tyr | 60 |
| Asp | Ile | Leu | Arg | Thr | Val | Phe | Val | His | Gln | Gln | Leu | Gln | Lys | Pro | 75 |
| Arg | Gln | Val | Val | Leu | Ala | Glu | Arg | Lys | Thr | Lys | Val | His | Tyr | Glu | 90 |
| Asp | Ile | Ser | His | Ala | Asp | Glu | Asn | Arg | Gln | Lys | Glu | His | Ile | Glu | 105 |
| Arg | Tyr | Lys | Gln | Gln | Val | Gln | Arg | Gln | Ala | Phe | Asn | Leu | Ala | Lys | 120 |
| Asp | Ile | Leu | Phe | Lys | Val | Ala | Val | Phe | Arg | Leu | Ala | Ala | Asp | Gln | 135 |
| Leu | Tyr | Leu | Ala | Trp | Ser | Asn | <u>His</u> | <u>His</u> | <u>Ile</u> | <u>Met</u> | <u>Met</u> | <u>Asp</u> | <u>Gly</u> | Trp | 150 |
| Ser | Met | Gly | Val | Leu | Met | Lys | Ser | Leu | Phe | Gln | Asn | Tyr | Glu | Ala | 165 |
| Leu | Arg | Ala | Gly | Arg | Thr | Pro | Ala | Asn | Gly | Gln | Gly | Lys | Pro | Tyr | 180 |
| Ser | Asp | Tyr | Ile | Lys | Trp | Leu | Gly | Lys | Gln | Asp | Asn | Glu | Glu | Ala | 195 |
| Glu | Ser | Tyr | Trp | Ser | Glu | Arg | Leu | Ala | Gly | Phe | Glu | Gln | Pro | Ser | 210 |
| Val | Leu | Pro | Gly | Arg | Leu | Pro | Val | Lys | Lys | Asp | Glu | Tyr | Val | Asn | 225 |
| Lys | Glu | Tyr | Ser | Phe | Thr | Trp | Asp | Glu | Thr | Leu | Val | Ala | Arg | Ile | 240 |
| Gln | Gln | Thr | Ala | Asn | Leu | His | Gln | Val | Thr | Gly | Pro | Asn | Leu | Phe | 255 |
| Gln | Ala | Val | Leu | Gly | Ile | Val | Leu | Ser | Lys | Tyr | Asn | Phe | Thr | Asp | 270 |
| Asp | Val | Ile | Phe | Gly | Thr | Val | Val | Ser | Gly | Arg | Pro | Ser | Glu | Ile | 285 |
| Asn | Gly | Ile | Glu | Thr | Met | Ala | Gly | Leu | Phe | Ile | Asn | Thr | Ile | Pro | 300 |
| Val | Arg | Val | Lys | Val | Glu | Arg | Asp | Arg | Ala | Phe | Ala | Asp | Ile | Phe | 315 |
| Thr | Ala | Val | Gln | Gln | His | Ala | Val | Glu | Ala | Glu | Arg | Tyr | Asp | Tyr | 330 |
| Val | Pro | Leu | Tyr | Glu | Ile | Gln | Lys | Arg | Ser | Ala | Leu | Asp | Gly | Asn | 345 |
| Leu | Leu | Asn | His | Leu | Val | Ala | Phe | Glu | Asn | Tyr | Pro | Leu | Asp | Gln | 360 |
| Glu | Leu | Glu | Asn | Gly | Ser | Met | Glu | Asp | Arg | Leu | Gly | Phe | Ser | Ile | 375 |
| Lys | Val | Glu | Ser | Ala | Phe | Glu | Gln | Thr | Ser | Phe | Asp | Phe | Asn | Leu | 390 |
| Ile | Val | Tyr | Pro | Gly | Lys | Thr | Trp | Thr | Val | Lys | Ile | Lys | Tyr | Asn | 405 |
| Gly | Ala | Ala | Phe | Asp | Ser | Ala | Phe | Ile | Glu | Arg | Thr | Ala | Glu | His | 420 |
| Leu | Thr | Arg | Met | Met | Glu | Ala | Ala | Val | Asp | Gln | Pro | Ala | Ala | Phe | 435 |
| Val | Arg | Glu | Tyr | Gly | Leu | Val | Gly | Asp | Glu | Glu | Gln | Arg | Gln | Ile | 450 |
| Val | Glu | Val | Phe | Asn | Ser | Thr | Lys | Ala | Glu | Leu | Pro | Glu | Gly | Met | 465 |
| Ala | Val | His | Gln | Val | Phe | Glu | Glu | Gln | Ala | Lys | Arg | Thr | Pro | Ala | 480 |

(Table 5 cont.)

| | | | | | | | | | | | | | | | |
|---|---|---|---|---|---|---|---|---|---|---|---|---|---|---|---|
| Ser | Thr | Ala | Val | Val | Tyr | Glu | Gly | Thr | Lys | Leu | Thr | Tyr | Arg | Glu | 495 |
| Leu | Asn | Ala | Ala | Ala | Asn | Arg | Leu | Ala | Arg | Lys | Leu | Val | Glu | His | 510 |
| Gly | Leu | Gln | Lys | Gly | Glu | Thr | Ala | Ala | Ile | Met | Asn | Asp | Arg | Ser | 525 |
| Val | Glu | Thr | Val | Val | Gly | Met | Leu | Ala | Val | Leu | Lys | Ala | Gly | Ala | 540 |
| Ala | Tyr | Val | Pro | Leu | Asp | Pro | Ala | Leu | Pro | Gly | Asp | Arg | Leu | Arg | 555 |
| Phe | Met | Ala | Glu | Asp | Ser | Ser | Val | Arg | Met | Val | Leu | Ile | Gly | Asn | 570 |
| Ser | Tyr | Thr | Gly | Gln | Ala | His | Gln | Leu | Gln | Val | Pro | Val | Leu | Thr | 585 |
| Leu | Asp | Ile | Gly | Phe | Glu | Glu | Ser | Glu | Ala | Ala | Asp | Asn | Leu | Asn | 600 |
| Leu | Pro | Ser | Ala | Pro | Ser | Asp | Leu | Ala | Tyr | Ile | Met | Tyr | Thr | Ser | 615 |
| Gly | Ser | Thr | Gly | Lys | Pro | Lys | Gly | Val | Met | Ile | Glu | His | Lys | Ser | 630 |
| Ile | Leu | Arg | Leu | Val | Lys | Asn | Ala | Gly | Tyr | Val | Pro | Val | Thr | Glu | 645 |
| Glu | Asp | Ala | Met | Ala | Gln | Thr | Gly | Ala | Val | Ser | Phe | Asp | Ala | Gly | 660 |
| Thr | Phe | Glu | Val | Phe | Gly | Ala | Leu | Leu | Asn | Gly | Ala | Ala | Leu | Tyr | 675 |
| Pro | Val | Lys | Lys | Arg | His | Val | Leu | Asp | Ala | Lys | Gln | Phe | Ala | Ala | 690 |
| Phe | Leu | Arg | Glu | Gln | Ser | Ile | Thr | Thr | Met | Trp | Leu | Thr | Ser | Pro | 705 |
| Leu | Phe | Asn | Gln | Leu | Ala | Ala | Lys | Asp | Ala | Gly | Met | Phe | Gly | Thr | 720 |
| Leu | Arg | His | Leu | Ile | Ile | Gly | Gly | Asp | Ala | Leu | Val | Pro | His | Ile | 735 |
| Val | Ser | Lys | Val | Lys | Gln | Ala | Ser | Pro | Ser | Leu | Ser | Leu | Trp | Asn | 750 |
| Gly | Tyr | Gly | Pro | Thr | Glu | Asn | Thr | Thr | Phe | Ser | Thr | Ser | Phe | Leu | 765 |
| Ile | Asp | Arg | Glu | Tyr | Gly | Gly | Ser | Ile | Pro | Ile | Gly | Lys | Pro | Ile | 780 |
| Gly | Asn | Ser | Thr | Ala | Tyr | Ile | Met | Asp | Glu | Gln | Gln | Cys | Leu | Gln | 795 |
| Pro | Ile | Gly | Ala | Pro | Gly | Glu | Leu | Cys | Val | Gly | Gly | Ile | Gly | Val | 810 |
| Ala | Arg | Gly | Tyr | Val | Asn | Leu | Pro | Glu | Leu | Thr | Glu | Lys | Gln | Phe | 825 |
| Leu | Glu | Asp | Pro | Phe | Arg | Pro | Gly | Glu | Arg | Ile | Tyr | Arg | Thr | Gly | 840 |
| Asp | Leu | Ala | Arg | Trp | Leu | Pro | Asp | Gly | Asn | Ile | Glu | Phe | Leu | Gly | 855 |
| Arg | Ile | Asp | Asn | Gln | Val | Lys | Val | Arg | Gly | Phe | Arg | Ile | Glu | Leu | 870 |
| Gly | Glu | Ile | Glu | Thr | Lys | Leu | Asn | Met | Ala | Glu | His | Val | Thr | Glu | 885 |
| Ala | Ala | Val | Ile | Ile | Arg | Lys | Asn | Lys | Ala | Asp | Glu | Asn | Glu | Ile | 900 |
| Cys | Ala | Tyr | Phe | Thr | Ala | Asp | Arg | Glu | Val | Ala | Val | Ser | Glu | Leu | 915 |
| Arg | Lys | Thr | Leu | Ser | Gln | Ser | Leu | Pro | Asp | Tyr | Met | Val | Pro | Ala | 930 |
| His | Leu | Ile | Gln | Met | Asp | Ser | Leu | Pro | Leu | Thr | Pro | Asn | Gly | Lys | 945 |
| Ile | Asn | Lys | Lys | Glu | Leu | Pro | Ala | Pro | Gln | Ser | Glu | Ala | Val | Gln | 960 |

(Table 5 cont.)

| | | | | | | | | | | | | | | | |
|---|---|---|---|---|---|---|---|---|---|---|---|---|---|---|---|
| Pro | Glu | Tyr | Ala | Ala | Pro | Lys | Thr | Glu | Ser | Glu | Lys | Lys | Leu | Ala | 975 |
| Glu | Ile | Trp | Glu | Gly | Ile | Leu | Gly | Val | Lys | Ala | Gly | Val | Thr | Asp | 990 |
| Asn | Phe | Phe | Met | Ile | Gly | Gly | His | Ser | Leu | Lys | Ala | Met | Met | Met | 1005 |
| Thr | Ala | Lys | Ile | Gln | Glu | His | Phe | His | Lys | Glu | Val | Pro | Ile | Lys | 1020 |
| Val | Leu | Phe | Glu | Lys | Pro | Thr | Ile | Gln | Glu | Leu | Ala | Leu | Tyr | Leu | 1035 |
| Glu | Glu | Asn | Glu | Ser | Lys | Glu | Glu | Gln | Thr | Phe | Glu | Pro | Ile | Arg | 1050 |
| Gln | Ala | Ser | Tyr | Gln | Gln | His | Tyr | Pro | Val | Ser | Pro | Ala | Gln | Arg | 1065 |
| Arg | Met | Tyr | Ile | Leu | Asn | Gln | Leu | Gly | Gln | Ala | Asn | Thr | Ser | Tyr | 1080 |
| Asn | Val | Pro | Ala | Val | Leu | Leu | Leu | Glu | Gly | Glu | Val | Asp | Lys | Asp | 1095 |
| Arg | Leu | Glu | Asn | Ala | Ile | Gln | Gln | Leu | Ile | Asn | Arg | His | Glu | Ile | 1110 |
| Leu | Arg | Thr | Ser | Phe | Asp | Met | Ile | Asp | Gly | Glu | Val | Val | Gln | Thr | 1125 |
| Val | His | Lys | Asn | Ile | Ser | Phe | His | Leu | Glu | Ala | Ala | Lys | Gly | Arg | 1140 |
| Glu | Glu | Asp | Ala | Glu | Glu | Ile | Ile | Lys | Ala | Phe | Val | Gln | Pro | Phe | 1155 |
| Glu | Leu | Asn | Arg | Ala | Pro | Leu | Val | Arg | Ser | Lys | Leu | Val | Gln | Leu | 1170 |
| Glu | Glu | Lys | Arg | His | Leu | Leu | Leu | Ile | Asp | Met | His | His | Ile | Ile | 1185 |
| Thr | Asp | Gly | Ser | Ser | Thr | Gly | Ile | Leu | Ile | Gly | Asp | Leu | Ala | Lys | 1200 |
| Ile | Tyr | Gln | Gly | Ala | Asp | Leu | Glu | Leu | Pro | Gln | Ile | His | Tyr | Lys | 1215 |
| Asp | Tyr | Ala | Val | Trp | His | Lys | Glu | Gln | Thr | Asn | Tyr | Gln | Lys | Asp | 1230 |
| Glu | Glu | Tyr | Trp | Leu | Asp | Val | Phe | Lys | Gly | Glu | Leu | Pro | Ile | Leu | 1245 |
| Asp | Leu | Pro | Ala | Asp | Phe | Glu | Arg | Pro | Ala | Glu | Arg | Ser | Phe | Ala | 1260 |
| Gly | Glu | Arg | Val | Met | Phe | Gly | Leu | Asp | Lys | Gln | Ile | Thr | Ala | Gln | 1275 |
| Ile | Lys | Ser | Leu | Met | Ala | Glu | Thr | Asp | Thr | Thr | Met | Tyr | Met | Phe | 1290 |
| Leu | Leu | Ala | Ala | Phe | Asn | Val | Leu | Leu | Ser | Lys | Tyr | Ala | Ser | Gln | 1305 |
| Asp | Asp | Ile | Ile | Val | Gly | Ser | Pro | Thr | Ala | Gly | Arg | Thr | His | Pro | 1320 |
| Asp | Leu | Gln | Gly | Val | Pro | Gly | Met | Phe | Val | Asn | Thr | Gly | Ala | Leu | 1335 |
| Arg | Thr | Ala | Pro | Ala | Gly | Asp | Lys | Thr | Phe | Ala | Gln | Phe | Leu | Glu | 1350 |
| Glu | Val | Lys | Thr | Ala | Ser | Leu | Gln | Ala | Phe | Glu | His | Gln | Ser | Tyr | 1365 |
| Pro | Leu | Glu | Glu | Leu | Ile | Glu | Lys | Leu | Pro | Leu | Thr | Arg | Asp | Thr | 1380 |
| Ser | Arg | Ser | Pro | Leu | Phe | Ser | Val | Met | Phe | Asn | Met | Gln | Asn | Met | 1395 |
| Glu | Ile | Pro | Ser | Leu | Arg | Leu | Gly | Asp | Leu | Lys | Ile | Ser | Ser | Tyr | 1410 |
| Ser | Met | Leu | His | His | Val | Ala | Lys | Phe | Asp | Leu | Ser | Leu | Glu | Ala | 1425 |
| Val | Glu | Arg | Glu | Glu | Asp | Ile | Gly | Leu | Ser | Phe | Asp | Tyr | Ala | Thr | 1440 |

(Table 5 cont.)

| | | | | | | | | | | | | | | | |
|---|---|---|---|---|---|---|---|---|---|---|---|---|---|---|---|
| Ala | Leu | Phe | Lys | Asp | Glu | Thr | Ile | Arg | Arg | Trp | Ser | Arg | His | Phe | 1455 |
| Val | Asn | Ile | Ile | Lys | Ala | Ala | Ala | Ala | Asn | Pro | Asn | Val | Arg | Leu | 1470 |
| Ser | Asp | Val | Asp | Leu | Leu | Ser | Ser | Ala | Glu | Thr | Ala | Ala | Leu | Leu | 1485 |
| Glu | Glu | Arg | His | Met | Thr | Gln | Ile | Thr | Glu | Ala | Thr | Phe | Ala | Ala | 1500 |
| Leu | Phe | Glu | Lys | Gln | Ala | Gln | Gln | Thr | Pro | Asp | His | Ser | Ala | Val | 1515 |
| Lys | Ala | Gly | Gly | Asn | Leu | Leu | Thr | Tyr | Arg | Glu | Leu | Asp | Glu | Gln | 1530 |
| Ala | Asn | Gln | Leu | Ala | His | His | Leu | Arg | Ala | Gln | Gly | Ala | Gly | Asn | 1545 |
| Glu | Asp | Ile | Val | Ala | Ile | Val | Met | Asp | Arg | Ser | Ala | Glu | Val | Met | 1560 |
| Val | Ser | Ile | Leu | Gly | Val | Met | Lys | Ala | Gly | Ala | Ala | Phe | Leu | Pro | 1575 |
| Ile | Asp | Pro | Asp | Thr | Pro | Gly | Glu | Arg | Ile | Arg | Tyr | Ser | Leu | Glu | 1590 |
| Asp | Ser | Gly | Ala | Lys | Phe | Ala | Val | Val | Asn | Glu | Arg | Asn | Met | Thr | 1605 |
| Ala | Ile | Gly | Gln | Tyr | Glu | Gly | Ile | Ile | Val | Ser | Leu | Asp | Asp | Gly | 1620 |
| Lys | Trp | Arg | Asn | Glu | Ser | Lys | Glu | Arg | Pro | Ser | Ser | Ile | Ser | Gly | 1635 |
| Ser | Arg | Asn | Leu | Ala | Tyr | Val | Ile | Tyr | Thr | Ser | Gly | Thr | Thr | Gly | 1650 |
| Lys | Pro | Lys | Gly | Val | Gln | Ile | Glu | His | Arg | Asn | Leu | Thr | Asn | Tyr | 1665 |
| Val | Ser | Trp | Phe | Ser | Glu | Glu | Ala | Gly | Leu | Thr | Lys | Arg | Arg | Ala | 1680 |
| Asp | Gly | Asn | Asp | Lys | Thr | Val | Leu | Leu | Ser | Ser | Tyr | Ala | Phe | Asp | 1695 |
| Leu | Gly | Tyr | Thr | Cys | Met | Phe | Pro | Val | Leu | Leu | Gly | Gly | Gly | Glu | 1710 |
| Leu | His | Ile | Val | Gln | Lys | Glu | Thr | Tyr | Thr | Ala | Pro | Asp | Glu | Ile | 1725 |
| Ala | His | Tyr | Ile | Lys | Glu | His | Gly | Ile | Thr | Tyr | Ile | Lys | Leu | Thr | 1740 |
| Pro | Ser | Leu | Phe | His | Thr | Ile | Val | Asn | Thr | Ala | Ser | Phe | Ala | Phe | 1755 |
| Asp | Ala | Asn | Phe | Glu | Ser | Leu | Arg | Leu | Ile | Val | Leu | Gly | Gly | Glu | 1770 |
| Lys | Ile | Ile | Pro | Thr | Asp | Val | Ile | Ala | Phe | Arg | Lys | Met | Tyr | Gly | 1785 |
| His | Thr | Glu | Phe | Ile | Asn | His | Tyr | Gly | Pro | Thr | Glu | Ala | Thr | Ile | 1800 |
| Gly | Ala | Ile | Ala | Gly | Arg | Val | Asp | Leu | Tyr | Glu | Pro | Asp | Ala | Phe | 1815 |
| Ala | Lys | Arg | Pro | Thr | Ile | Gly | Arg | Pro | Ile | Ala | Asn | Ala | Gly | Ala | 1830 |
| Leu | Val | Leu | Asn | Glu | Ala | Leu | Lys | Leu | Val | Pro | Pro | Gly | Ala | Ser | 1845 |
| Gly | Gln | Leu | Tyr | Ile | Thr | Gly | Gln | Gly | Leu | Ala | Arg | Gly | Tyr | Leu | 1860 |
| Asn | Arg | Pro | Gln | Leu | Thr | Ala | Glu | Arg | Phe | Val | Glu | Asn | Pro | Tyr | 1875 |
| Ser | Pro | Gly | Ser | Leu | Met | Tyr | Lys | Thr | Gly | Asp | Val | Val | Arg | Arg | 1890 |
| Leu | Ser | Asp | Gly | Thr | Leu | Ala | Phe | Ile | Gly | Arg | Ala | Asp | Asp | Gln | 1905 |
| Val | Lys | Ile | Arg | Gly | Tyr | Arg | Ile | Glu | Leu | Gly | Glu | Ile | Glu | Thr | 1920 |

(Table 5 cont.)

| | |
|---|---|
| Val Met Leu Ser Leu Ser Gly Ile Gln Glu Ala Val Val Leu Ala | 1935 |
| Val Ser Glu Gly Gly Leu Gln Glu Leu Cys Ala Tyr Tyr Thr Ser | 1950 |
| Asp Gln Asp Ile Glu Lys Ala Glu Leu Arg Tyr Gln Leu Ser Leu | 1965 |
| Thr Leu Pro Ser His Met Ile Pro Ala Phe Phe Val Gln Val Asp | 1980 |
| Ala Ile Pro Leu Thr Ala Asn Gly Lys Thr Asp Arg Asn Ala Leu | 1995 |
| Pro Lys Pro Asn Ala Ala Gln Ser Gly Gly Lys Ala Leu Ala Ala | 2010 |
| Pro Glu Thr Ala Leu Glu Glu Ser Leu Cys Arg Ile Trp Gln Lys | 2025 |
| Thr Leu Gly Ile Glu Ala Ile Gly Ile Asp Asp Asn Phe Phe Asp | 2040 |
| Leu Gly Gly His Ser Leu Lys Gly Met Met Leu Ile Ala Asn Ile | 2055 |
| Gln Ala Glu Leu Glu Lys Ser Val Pro Leu Lys Ala Leu Phe Glu | 2070 |
| Gln Pro Thr Val Arg Gln Leu Ala Ala Tyr Met Glu Ala Ser Ala | 2085 |
| Val Ser Gly Gly His Gln Val Leu Lys Pro Ala Asp Lys Gln Asp | 2100 |
| Met Tyr Pro Leu Ser Ser Ala Gln Lys Arg Met Tyr Val Leu Asn | 2115 |
| Gln Leu Asp Arg Gln Thr Ile Ser Tyr Asn Met Pro Ser Val Leu | 2130 |
| Leu Met Glu Gly Glu Leu Asp Ile Trp Pro Ala Arg Leu Thr Pro | 2145 |
| Gln Leu Val Asn Arg His Glu Ser Leu Arg Thr Ser Phe Met Glu | 2160 |
| Ala Asn Gly Glu Pro Val Gln Arg Ile Ile Glu Lys Ala Glu Val | 2175 |
| Asp Leu His Val Phe Glu Ala Lys Glu Asp Glu Ala Asp Gln Lys | 2190 |
| Ile Lys Glu Phe Ile Arg Pro Phe Asp Leu Asn Asp Ala Pro Leu | 2205 |
| Ile Arg Ala Ala Leu Leu Arg Ile Glu Ala Lys Lys His Leu Leu | 2220 |
| Leu Leu Asp Met His His Ile Ile Ala Asp Gly Val Ser Arg Gly | 2235 |
| Ile Phe Val Lys Glu Leu Ala Leu Leu Tyr Lys Gly Glu Gln Leu | 2250 |
| Pro Glu Pro Thr Leu His Tyr Lys Asp Phe Ala Val Trp Gln Asn | 2265 |
| Glu Ala Glu Gln Lys Glu Arg Met Lys Glu His Glu Ala Tyr Trp | 2280 |
| Met Ser Val Leu Ser Gly Glu Leu Pro Glu Leu Asp Leu Pro Leu | 2295 |
| Asp Tyr Ala Arg Pro Pro Val Gln Ser Phe Lys Gly Asp Thr Ile | 2310 |
| Arg Phe Arg Thr Gly Ser Glu Thr Ala Lys Ala Val Glu Lys Leu | 2325 |
| Leu Ala Glu Thr Gly Thr Thr Leu His Met Val Leu His Ala Val | 2340 |
| Phe His Val Phe Leu Ser Lys Ile Ser Gly Gln Arg Asp Ile Val | 2355 |
| Ile Gly Ser Val Thr Ala Gly Arg Thr Asn Ala Asp Val Gln Asp | 2370 |
| Met Pro Gly Met Phe Val Asn Thr Leu Ala Leu Arg Met Glu Ala | 2385 |
| Lys Glu Gln Gln Thr Phe Ala Glu Leu Leu Glu Leu Ala Lys Gln | 2400 |

(Table 5 cont.)

| | | | | | | | | | | | | | | | |
|---|---|---|---|---|---|---|---|---|---|---|---|---|---|---|---|
| Thr | Asn | Leu | Ser | Ala | Leu | Glu | His | Gln | Glu | Tyr | Pro | Phe | Glu | Asp | 2415 |
| Leu | Val | Asn | Gln | Leu | Asp | Leu | Pro | Arg | Asp | Met | Ser | Arg | Asn | Pro | 2430 |
| Leu | Phe | Asn | Val | Met | Val | Thr | Thr | Glu | Asn | Pro | Asp | Lys | Glu | Glu | 2445 |
| Leu | Thr | Leu | Gln | Asn | Leu | Ser | Ile | Ser | Pro | Tyr | Glu | Ala | His | Gln | 2460 |
| Gly | Thr | Ser | Lys | Phe | Asp | Leu | Thr | Leu | Gly | Gly | Phe | Thr | Asp | Glu | 2475 |
| Asn | Gly | Ile | Gly | Leu | Gln | Leu | Glu | Tyr | Ala | Thr | Asp | Leu | Phe | Ala | 2490 |
| Lys | Glu | Thr | Ala | Glu | Lys | Trp | Ser | Glu | Tyr | Val | Leu | Arg | Leu | Leu | 2505 |
| Lys | Ala | Val | Ala | Asp | Asn | Pro | Asn | Gln | Pro | Leu | Ser | Ser | Leu | Leu | 2520 |
| Leu | Val | Thr | Glu | Thr | Glu | Lys | Gln | Ala | Leu | Leu | Glu | Ala | Trp | Lys | 2535 |
| Gly | Lys | Ala | Leu | Pro | Val | Pro | Thr | Asp | Lys | Thr | Val | His | Gln | Leu | 2550 |
| Phe | Glu | Glu | Thr | Val | Gln | Arg | His | Lys | Asp | Arg | Pro | Ala | Val | Thr | 2565 |
| Tyr | Asn | Gly | Gln | Ser | Trp | Thr | Tyr | Gly | Glu | Leu | Asn | Ala | Lys | Ala | 2580 |
| Asn | Arg | Leu | Ala | Arg | Ile | Leu | Met | Asp | Cys | Gly | Ile | Ser | Pro | Asp | 2595 |
| Asp | Arg | Val | Gly | Val | Leu | Thr | Lys | Pro | Ser | Leu | Glu | Met | Ser | Ala | 2610 |
| Ala | Val | Leu | Gly | Val | Leu | Lys | Ala | Gly | Ala | Ala | Phe | Val | Pro | Ile | 2625 |
| Asp | Pro | Asp | Tyr | Pro | Asp | Gln | Arg | Ile | Glu | Tyr | Ile | Leu | Gln | Asp | 2640 |
| Ser | Gly | Ala | Lys | Leu | Leu | Leu | Lys | Gln | Glu | Gly | Ile | Ser | Val | Pro | 2655 |
| Asp | Ser | Tyr | Thr | Gly | Asp | Val | Ile | Leu | Leu | Asp | Gly | Ser | Arg | Thr | 2670 |
| Ile | Leu | Ser | Leu | Pro | Leu | Asp | Glu | Asn | Asp | Glu | Gly | Asn | Pro | Glu | 2685 |
| Thr | Ala | Val | Thr | Ala | Glu | Asn | Leu | Ala | Tyr | Met | Ile | Tyr | Thr | Ser | 2700 |
| Gly | Thr | Thr | Gly | Gln | Pro | Lys | Gly | Val | Met | Val | Glu | Asp | His | Ala | 2715 |
| Leu | Val | Asn | Leu | Cys | Phe | Trp | Asp | His | Asp | Ala | Phe | Ser | Met | Thr | 2730 |
| Ala | Glu | Asp | Arg | Ser | Ala | Lys | Tyr | Ala | Gly | Phe | Gly | Phe | Asp | Ala | 2745 |
| Ser | Ile | Trp | Glu | Met | Phe | Pro | Thr | Trp | Thr | Ile | Gly | Ala | Glu | Leu | 2760 |
| His | Val | Ile | Asp | Glu | Ala | Ile | Arg | Leu | Asp | Ile | Val | Arg | Leu | Asn | 2775 |
| Asp | Tyr | Phe | Glu | Thr | Asn | Gly | Val | Thr | Ile | Thr | Phe | Leu | Pro | Thr | 2790 |
| Gln | Leu | Ala | Glu | Gln | Phe | Met | Glu | Leu | Glu | Asn | Thr | Ser | Leu | Arg | 2805 |
| Val | Leu | Leu | Thr | Gly | Gly | Asp | Lys | Leu | Lys | Arg | Ala | Val | Lys | Lys | 2820 |
| Pro | Tyr | Thr | Leu | Val | Asn | Asn | Tyr | Gly | Pro | Thr | Glu | Asn | Thr | Val | 2835 |
| Val | Ala | Thr | Ser | Ala | Glu | Ile | His | Pro | Glu | Glu | Gly | Ser | Leu | Ser | 2850 |
| Ile | Gly | Arg | Ala | Ile | Ala | Asn | Thr | Arg | Val | Tyr | Ile | Leu | Gly | Glu | 2865 |
| Gly | Asn | Gln | Val | Gln | Pro | Glu | Gly | Val | Ala | Gly | Glu | Leu | Cys | Val | 2880 |

(Table 5 cont.)

Ala Gly Arg Gly Leu Ala Arg Gly Tyr Leu Asn Arg Glu Asp Glu 2895
Thr Ala Lys Arg Phe Val Ala Asp Pro Phe Val Pro Gly Glu Arg 2910
Met Tyr Arg Thr Gly Asp Leu Val Lys Trp Val Asn Gly Gly Ile 2925
Glu Tyr Ile Gly Arg Ile Asp Gln Gln Val Lys Val Arg Gly Tyr 2940
Arg Ile Glu Leu Ser Glu Ile Glu Val Gln Leu Ala Gln Leu Ser 2955
Glu Val Glu Asp Arg Ala Val Thr Arg Val Lys Asp Lys Gly Gly 2970
Asn Thr Ala Ile Ala Ala Tyr Val Thr Pro Glu Thr Ala Asp Ile 2985
Glu Ala Leu Lys Ser Thr Leu Lys Glu Thr Leu Pro Asp Tyr Met 3000
Ile Pro Ala Phe Trp Val Thr Leu Asn Glu Leu Pro Val Thr Ala 3015
Asn Gly Lys Val Asp Arg Lys Ala Leu Pro Glu Pro Asp Ile Glu 3030
Ala Gly Ser Gly Glu Tyr Lys Ala Pro Thr Thr Asp Met Glu Glu 3045
Leu Leu Ala Gly Ile Trp Gln Asp Val Leu Gly Met Ser Glu Val 3060
Gly Val Thr Asp Asn Phe Phe Ser Leu Gly Gly Asp Ser Ile Lys 3075
Gly Ile Gln Met Ala Ser Arg Leu Asn Gln His Gly Trp Lys Leu 3090
Glu Met Lys Asp Leu Phe Gln His Pro Thr Ile Glu Glu Leu Thr 3105
Gln Tyr Val Glu Arg Ala Glu Gly Lys Gln Ala Asp Gln Gly Pro 3120
Val Glu Gly Glu Val Ile Leu Thr Pro Ile Gln Arg Trp Phe Phe 3135
Glu Lys Asn Phe Thr Asn Lys His His Trp Asn Gln Ser Val Met 3150
Leu His Ala Lys Lys Gly Phe Asp Pro Glu Arg Val Glu Lys Thr 3165
Leu Gln Ala Leu Ile Glu His His Asp Ala Leu Arg Met Val Tyr 3180
Arg Glu Glu Asn Gly Asp Ile Val Gln Val Tyr Lys Pro Ile Gly 3195
Glu Ser Lys Val Ser Phe Glu Ile Val Asp Leu Tyr Gly Ser Asp 3210
Glu Glu Met Leu Arg Ser Gln Ile Lys Leu Leu Ala Asn Lys Leu 3225
Gln Ser Ser Leu Asp Leu Arg Asn Gly Pro Leu Leu Lys Ala Ala 3240
Glu Tyr Arg Thr Glu Ala Gly Asp His Leu Leu Ile Ala Val His 3255
His Leu Val Val Asp Gly Val Ser Trp Arg Ile Leu Leu Glu Asp 3270
Phe Ala Ser Gly Tyr Met Gln Ala Glu Lys Glu Glu Ser Leu Val 3285
Phe Pro Gln Lys Thr Asn Ser Phe Lys Asp Trp Ala Glu Glu Leu 3300
Ala Ala Phe Ser Gln Ser Ala His Leu Leu Gln Gln Ala Glu Tyr 3315
Trp Ser Gln Ile Ala Ala Glu Gln Val Ser Pro Leu Pro Lys Asp 3330
Cys Glu Thr Glu Gln Arg Ile Val Lys Asp Thr Ser Ser Val Leu 3345
Cys Glu Leu Thr Ala Glu Asp Thr Lys His Leu Leu Thr Asp Val 3360

(Table 5 cont.)

| | | | | | | | | | | | | | | | |
|---|---|---|---|---|---|---|---|---|---|---|---|---|---|---|---|
| His | Gln | Pro | Tyr | Gly | Thr | Glu | Ile | Asn | Asp | Ile | Leu | Leu | Ser | Ala | 3375 |
| Leu | Gly | Leu | Thr | Met | Lys | Glu | Trp | Thr | Lys | Gly | Ala | Lys | Ile | Gly | 3390 |
| Ile | Asn | Leu | Glu | Gly | His | Gly | Arg | Glu | Asp | Ile | Ile | Pro | Asn | Val | 3405 |
| Asn | Ile | Ser | Arg | Thr | Val | Gly | Trp | Phe | Thr | Ala | Gln | Tyr | Pro | Val | 3420 |
| Val | Leu | Asp | Ile | Ser | Asp | Ala | Asp | Ala | Ser | Ala | Val | Ile | Lys | Thr | 3435 |
| Val | Lys | Glu | Asn | Leu | Arg | Arg | Ile | Pro | Asp | Lys | Gly | Val | Gly | Tyr | 3450 |
| Gly | Ile | Leu | Arg | Tyr | Phe | Thr | Glu | Thr | Ala | Glu | Thr | Lys | Gly | Phe | 3465 |
| Thr | Pro | Glu | Ile | Ser | Phe | Asn | Tyr | Leu | Gly | Gln | Phe | Asp | Ser | Glu | 3480 |
| Val | Lys | Thr | Asp | Phe | Phe | Glu | Pro | Ser | Ala | Phe | Asp | Met | Gly | Arg | 3495 |
| Gln | Val | Ser | Gly | Glu | Ser | Glu | Ala | Leu | Tyr | Ala | Leu | Ser | Phe | Ser | 3510 |
| Gly | Met | Ile | Arg | Asn | Gly | Arg | Phe | Val | Leu | Ser | Cys | Ser | Tyr | Asn | 3525 |
| Glu | Lys | Glu | Phe | Glu | Arg | Ala | Thr | Val | Glu | Glu | Glu | Met | Glu | Arg | 3540 |
| Phe | Lys | Glu | Asn | Leu | Leu | Met | Leu | Ile | Arg | His | Cys | Thr | Glu | Lys | 3555 |
| Glu | Asp | Lys | Glu | Phe | Thr | Pro | Ser | Asp | Phe | Ser | Ala | Glu | Asp | Leu | 3570 |
| Glu | Met | Asp | Glu | Met | Gly | Asp | Ile | Phe | Asp | Met | Leu | Glu | Glu | Asn | 3585 |
| Leu | Lys | | | | | | | | | | | | | | 3587 |

This protein, which is structured in a similar manner to ORF1, contains three adjacent homology regions (modules 4 - 6) followed by a region, module B, of about 500 amino acids very similar to that of ORF1 and of gramicidin and tyrocidin synthetase I.

The third ORF (ORF3) is formed from 3825 bases (from nucleotide 21580 to nucleotide 25404).

The intergenic region between ORF2 and ORF3 is of 36 bases and contains a Shine - Dalgarno with a GGGAG sequence 8 bases distant from the ATG codon of ORF3 (Table 3).

ORF3 codes for a protein of 1274 amino acids (molecular weight 143816 daltons) having the amino acid sequence given in Table 6.

TABLE 6

| | | | | | | | | | | | | | | | |
|---|---|---|---|---|---|---|---|---|---|---|---|---|---|---|---|
| Met | Ser | Gln | Phe | Ser | Lys | Asp | Gln | Val | Gln | Asp | Met | Tyr | Tyr | Leu | 15 |
| Ser | Pro | Met | Gln | Glu | Gly | Met | Leu | Phe | His | Pro | Ile | Leu | Asn | Pro | 30 |
| Gly | Gln | Thr | Phe | Tyr | Leu | Glu | Gln | Ile | Thr | Met | Lys | Val | Lys | Gly | 45 |
| Ser | Leu | Asn | Ile | Lys | Cys | Leu | Glu | Glu | Ser | Met | Asn | Val | Ile | Met | 60 |
| Asp | Arg | Tyr | Asp | Val | Phe | Arg | Thr | Val | Phe | Ile | His | Glu | Lys | Val | 75 |
| Lys | Arg | Pro | Val | Gln | Val | Val | Leu | Lys | Lys | Arg | Gln | Phe | His | Ile | 90 |
| Glu | Glu | Ile | Asp | Leu | Thr | His | Leu | Thr | Gly | Ser | Glu | Gln | Thr | Ala | 105 |
| Lys | Ile | Asn | Glu | Tyr | Lys | Glu | Gln | Asp | Lys | Ile | Arg | Gly | Phe | Asp | 120 |
| Leu | Thr | Arg | Asp | Ile | Pro | Met | Arg | Ala | Ala | Ile | Phe | Lys | Lys | Ala | 135 |
| Glu | Glu | Ser | Phe | Glu | Trp | Val | Trp | Ser | Tyr | His | His | Ile | Ile | Leu | 150 |
| Asp | Gly | Trp | Cys | Phe | Gly | Ile | Val | Val | Gln | Asp | Leu | Phe | Lys | Val | 165 |
| Tyr | Asn | Ala | Leu | Arg | Glu | Gln | Lys | Pro | Tyr | Ser | Leu | Pro | Pro | Val | 180 |
| Lys | Pro | Tyr | Lys | Asp | Tyr | Ile | Lys | Trp | Leu | Glu | Lys | Gln | Asp | Lys | 195 |
| Gln | Ala | Ser | Leu | Arg | Tyr | Trp | Arg | Glu | Tyr | Leu | Glu | Gly | Phe | Glu | 210 |
| Gly | Gln | Thr | Thr | Phe | Ala | Glu | Gln | Arg | Lys | Lys | Gln | Lys | Asp | Gly | 225 |
| Tyr | Glu | Pro | Lys | Glu | Leu | Leu | Phe | Ser | Leu | Ser | Glu | Ala | Glu | Thr | 240 |
| Lys | Ala | Phe | Thr | Glu | Leu | Ala | Lys | Ser | Gln | His | Thr | Thr | Leu | Ser | 255 |
| Thr | Ala | Leu | Gln | Ala | Val | Trp | Ser | Val | Leu | Ile | Ser | Arg | Tyr | Gln | 270 |
| Gln | Ser | Gly | Asp | Leu | Ala | Phe | Gly | Thr | Val | Val | Ser | Gly | Arg | Pro | 285 |
| Ala | Glu | Ile | Lys | Gly | Val | Glu | His | Met | Val | Gly | Leu | Phe | Ile | Asn | 300 |
| Val | Val | Pro | Arg | Arg | Val | Lys | Leu | Ser | Glu | Gly | Ile | Thr | Phe | Asn | 315 |
| Gly | Leu | Leu | Lys | Arg | Leu | Gln | Glu | Gln | Ser | Leu | Gln | Ser | Glu | Pro | 330 |
| His | Gln | Tyr | Val | Pro | Leu | Tyr | Asp | Ile | Gln | Ser | Gln | Ala | Asp | Gln | 345 |
| Pro | Lys | Leu | Ile | Asp | His | Ile | Ile | Val | Phe | Glu | Asn | Tyr | Pro | Leu | 360 |
| Gln | Asp | Ala | Lys | Asn | Glu | Glu | Ser | Ser | Glu | Asn | Gly | Phe | Asp | Met | 375 |
| Val | Asp | Val | His | Val | Phe | Glu | Lys | Ser | Asn | Tyr | Asp | Leu | Asn | Leu | 390 |
| Met | Ala | Ser | Pro | Gly | Asp | Glu | Met | Leu | Ile | Lys | Leu | Ala | Tyr | Asn | 405 |
| Glu | Asn | Val | Phe | Asp | Glu | Ala | Phe | Ile | Leu | Arg | Leu | Lys | Ser | Gln | 420 |
| Leu | Leu | Thr | Ala | Ile | Gln | Gln | Leu | Ile | Gln | Asn | Pro | Asp | Gln | Pro | 435 |
| Val | Ser | Thr | Ile | Asn | Leu | Val | Asp | Asp | Arg | Glu | Arg | Glu | Phe | Leu | 450 |
| Leu | Thr | Gly | Leu | Asn | Pro | Pro | Ala | Gln | Ala | His | Glu | Thr | Lys | Pro | 465 |
| Leu | Thr | Tyr | Trp | Phe | Lys | Glu | Ala | Val | Asn | Ala | Asn | Pro | Asp | Ala | 480 |

(Table 6 cont.)

| | |
|---|---|
| Pro Ala Leu Thr Tyr Ser Gly Gln Thr Leu Ser Tyr Arg Glu Leu | 495 |
| Asp Glu Glu Ala Asn Arg Ile Ala Arg Arg Leu Gln Lys His Gly | 510 |
| Ala Gly Lys Gly Ser Val Val Ala Leu Tyr Thr Lys Arg Ser Leu | 525 |
| Glu Leu Val Ile Gly Ile Leu Gly Val Leu Lys Ala Gly Ala Ala | 540 |
| Tyr Leu Pro Val Asp Pro Lys Leu Pro Glu Asp Arg Ile Ser Tyr | 555 |
| Met Leu Ala Asp Ser Ala Ala Ala Cys Leu Leu Thr His Gln Glu | 570 |
| Met Lys Glu Gln Ala Ala Glu Leu Pro Tyr Thr Gly Thr Thr Leu | 585 |
| Phe Ile Asp Asp Gln Thr Arg Phe Glu Glu Gln Ala Ser Asp Pro | 600 |
| Ala Thr Ala Ile Asp Pro Asn Asp Pro Ala Tyr Ile Met Tyr Thr | 615 |
| Ser Gly Thr Thr Gly Lys Pro Lys Gly Asn Ile Thr Thr His Ala | 630 |
| Asn Ile Gln Gly Leu Val Lys His Val Asp Tyr Met Ala Phe Ser | 645 |
| Asp Gln Asp Thr Phe Leu Ser Val Ser Asn Tyr Ala Phe Asp Ala | 660 |
| Phe Thr Phe Asp Phe Tyr Ala Ser Met Leu Asn Ala Ala Arg Leu | 675 |
| Ile Ile Ala Asp Glu His Thr Leu Leu Asp Thr Glu Arg Leu Thr | 690 |
| Asp Leu Ile Leu Gln Glu Asn Val Asn Val Met Phe Ala Thr Thr | 705 |
| Ala Leu Phe Asn Leu Leu Thr Asp Ala Gly Glu Asp Trp Met Lys | 720 |
| Gly Leu Arg Cys Ile Leu Phe Gly Gly Glu Arg Ala Ser Val Pro | 735 |
| His Val Arg Lys Ala Leu Arg Ile Met Gly Pro Gly Lys Leu Ile | 750 |
| Asn Cys Tyr Gly Pro Thr Glu Gly Thr Val Phe Ala Thr Ala His | 765 |
| Val Val His Asp Leu Pro Asp Ser Ile Ser Ser Leu Pro Ile Gly | 780 |
| Lys Pro Ile Ser Asn Ala Ser Val Tyr Ile Leu Asn Glu Gln Ser | 795 |
| Gln Leu Gln Pro Phe Gly Ala Val Gly Glu Leu Cys Ile Ser Gly | 810 |
| Met Gly Val Ser Lys Gly Tyr Val Asn Arg Ala Asp Leu Thr Lys | 825 |
| Glu Lys Phe Ile Glu Asn Pro Phe Lys Pro Gly Glu Thr Leu Tyr | 840 |
| Arg Thr Gly Asp Leu Ala Arg Trp Leu Pro Asp Gly Thr Ile Glu | 855 |
| Tyr Ala Gly Arg Ile Asp Asp Gln Val Lys Ile Arg Gly His Arg | 870 |
| Ile Glu Leu Glu Glu Ile Glu Lys Gln Leu Gln Glu Tyr Pro Gly | 885 |
| Val Lys Asp Ala Val Val Val Ala Asp Arg His Glu Ser Gly Asp | 900 |
| Ala Ser Ile Asn Ala Tyr Leu Val Asn Arg Thr Gln Leu Ser Ala | 915 |
| Glu Asp Val Lys Ala His Leu Lys Lys Gln Leu Pro Ala Tyr Met | 930 |
| Val Pro Gln Thr Phe Thr Phe Leu Asp Glu Leu Pro Leu Thr Thr | 945 |
| Asn Gly Lys Val Asn Lys Arg Leu Leu Pro Lys Pro Asp Gln Asp | 960 |

(Table 6 cont.)

| | | | | | | | | | | | | | | | |
|---|---|---|---|---|---|---|---|---|---|---|---|---|---|---|---|
| Gln | Leu | Ala | Glu | Glu | Trp | Ile | Gly | Pro | Arg | Asn | Glu | Met | Glu | Glu | 975 |
| Thr | Ile | Ala | Gln | Ile | Trp | Ser | Glu | Val | Leu | Gly | Arg | Lys | Gln | Ile | 990 |
| Gly | Ile | His | Asp | Asp | Phe | Phe | Ala | Leu | Gly | Gly | His | Ser | Leu | Lys | 1005 |
| Ala | Met | Thr | Ala | Val | Pro | His | Gln | Gln | Glu | Leu | Gly | Ile | Asp | Leu | 1020 |
| Pro | Val | Lys | Leu | Leu | Phe | Glu | Ala | Pro | Thr | Ile | Ala | Gly | Ile | Ser | 1035 |
| Ala | Tyr | Leu | Lys | Asn | Gly | Gly | Ser | Asp | Gly | Leu | Gln | Asp | Val | Thr | 1050 |
| Ile | Met | Asn | Gln | Asp | Gln | Glu | Gln | Ile | Ile | Phe | Ala | Phe | Pro | Pro | 1065 |
| Val | Leu | Gly | Tyr | Gly | Leu | Met | Tyr | Gln | Asn | Leu | Ser | Ser | Arg | Leu | 1080 |
| Pro | Ser | Tyr | Lys | Leu | Cys | Ala | Phe | Asp | Phe | Ile | Glu | Glu | Glu | Asp | 1095 |
| Arg | Leu | Asp | Arg | Tyr | Ala | Asp | Leu | Ile | Gln | Lys | Leu | Gln | Pro | Glu | 1110 |
| Gly | Pro | Leu | Thr | Leu | Phe | Gly | Tyr | Ser | Ala | Gly | Cys | Ser | Leu | Ala | 1125 |
| Phe | Glu | Ala | Ala | Lys | Lys | Leu | Glu | Glu | Gln | Gly | Arg | Ile | Val | Gln | 1140 |
| Arg | Ile | Ile | Met | Val | Asp | Ser | Tyr | Lys | Lys | Gln | Gly | Val | Ser | Asp | 1155 |
| Leu | Asp | Gly | Arg | Thr | Val | Glu | Ser | Asp | Val | Glu | Ala | Leu | Met | Asn | 1170 |
| Val | Asn | Arg | Asp | Asn | Glu | Ala | Leu | Asn | Ser | Glu | Ala | Val | Lys | His | 1185 |
| Gly | Leu | Lys | Gln | Lys | Thr | His | Ala | Phe | Tyr | Ser | Tyr | Tyr | Val | Asn | 1200 |
| Leu | Ile | Ser | Thr | Gly | Gln | Val | Lys | Ala | Asp | Ile | Asp | Leu | Leu | Thr | 1215 |
| Ser | Gly | Ala | Asp | Phe | Asp | Met | Pro | Glu | Trp | Leu | Ala | Ser | Trp | Glu | 1230 |
| Glu | Ala | Thr | Thr | Gly | Val | Tyr | Arg | Val | Lys | Arg | Gly | Phe | Gly | Thr | 1245 |
| His | Ala | Glu | Met | Leu | Gln | Gly | Glu | Thr | Leu | Asp | Arg | Asn | Ala | Glu | 1260 |
| Ile | Leu | Leu | Glu | Phe | Leu | Asn | Thr | Gln | Thr | Val | Thr | Val | Ser | | 1274 |

Analysis of this protein swhows the presence of a region of 1008 amino acids (module 7) which is aligned for homology with modules 1 - 6 and contains the hyghly conserved sequences.

The last 243 amino acids of this protein show no homology with modules 1 - 6 or modules A or B nor with known sequences of antibiotic peptide synthetases.

The nucleotide sequence from 24577 to 24591 shows homology with the consensus sequence of the catalytic site of thioesterase. Finally, the fourth ORF (ORF4) is formed from 729 bases (from nucleotide 25433 to nucleotide 26161).

The intergenic region betweed ORF3 and ORF4 is of 30 bases and contains a Shine - Dalgarno with an AAAGGAGG sequence 9 bases distant from the ATG codon of ORF4 (Table 3).

ORF4 codes for a protein of 242 amino acids (molecular weight about 26000 daltons) with the amino acid sequence shown in Table 7.

TABLE 7

| | | | | | | | | | | | | | | | |
|---|---|---|---|---|---|---|---|---|---|---|---|---|---|---|---|
| | | | | | 5 | | | | | 10 | | | | | 15 |
| 1 | Met | Ser | Gln | Leu | Phe | Lys | Ser | Phe | Asp | Ala | Ser | Glu | Lys | Thr | Gln |
| 16 | Leu | Ile | Cys | Phe | Pro | Val | Ser | Gly | Gly | Tyr | Ser | Ala | Ser | Phe | Arg |
| 31 | Pro | Leu | His | Ala | Phe | Leu | Gln | Gly | Glu | Cys | Glu | Met | Leu | Ala | Ala |
| 46 | Glu | Pro | Pro | Gly | His | Gly | Thr | Asn | Gln | Thr | Ser | Ala | Ile | Glu | Asp |
| 61 | Leu | Glu | Glu | Leu | Thr | Asp | Leu | Tyr | Lys | Gln | Glu | Leu | Asn | Leu | Arg |
| 76 | Pro | Asp | Arg | Pro | Phe | Val | Leu | Phe | Gly | His | Ser | Met | Gly | Gly | Met |
| 91 | Ile | Thr | Phe | Ser | Trp | Arg | Lys | Ser | Leu | Ser | Val | Lys | Ala | Ser | Phe |
| 106 | Ala | Gly | Gly | Tyr | His | Phe | Ala | Ile | Gln | Pro | Pro | His | Ile | Gln | Arg |
| 121 | Lys | Lys | Val | Ser | His | Leu | Pro | Asp | Asp | Gln | Phe | Leu | Asp | His | Ile |
| 136 | Ile | Gln | Leu | Gly | Gly | Met | Pro | Ala | Glu | Leu | Val | Glu | Asn | Lys | Glu |
| 151 | Val | Met | Ser | Phe | Phe | Leu | Pro | Ser | Phe | Arg | Ser | Asp | Tyr | Arg | Ala |
| 166 | Leu | Glu | Gln | Phe | Glu | Leu | Tyr | Asp | Leu | Ala | Gln | Ile | Gln | Ser | Pro |
| 181 | Val | His | Val | Phe | Asn | Gly | Leu | Asp | Asp | Lys | Lys | Cys | Ile | Arg | Asp |
| 196 | Ala | Glu | Gly | Trp | Lys | Lys | Trp | Ala | Lys | Asp | Ile | Thr | Phe | His | Gln |
| 211 | Phe | Asp | Gly | Gly | His | Met | Phe | Leu | Leu | Ser | Gln | Thr | Glu | Glu | Val |
| 226 | Ala | Glu | Arg | Ile | Phe | Glu | | | | | | | | | |

Analysis of this protein shows the presence of the sequence Gly-His-Ser-Met-Gly, which is found in the active site of various bacterial and eukaryotic thioesterases. This resemblance and the sequence homologies with the gene grst indicate a probable thioesterase function for this subunit of the surfactin synthetase complex.

In conclusion the first three ORFs are divided into seven regions of internal homology which by analogy with other systems (grs, tyc and ACV) are associated with the enzymatic activities of recognition, activation and polymerization of the 7 component amino acids of surfactin (Glu, Leu, D-Leu, Val, Asp, D-Leu and Leu).

Finally, by analogy with TycA, GrsA and ACV synthetase, the presence of the A and B modules of ORF1 and ORF2 can be associated with racemase activity.

It has also been shown that the contiguity of the modules reflects the amino acid sequence in the peptide ring of surfactin, hence ORF1 catalyzes the activation of Glu, Leu and D-Leu and the synthesis of this tripeptide, ORF2 the elongation to the hexapeptide, and ORF3 the addition of the seventh amino acid.

Part of the first four homology modules is essential for establishing B.subtilis competence.

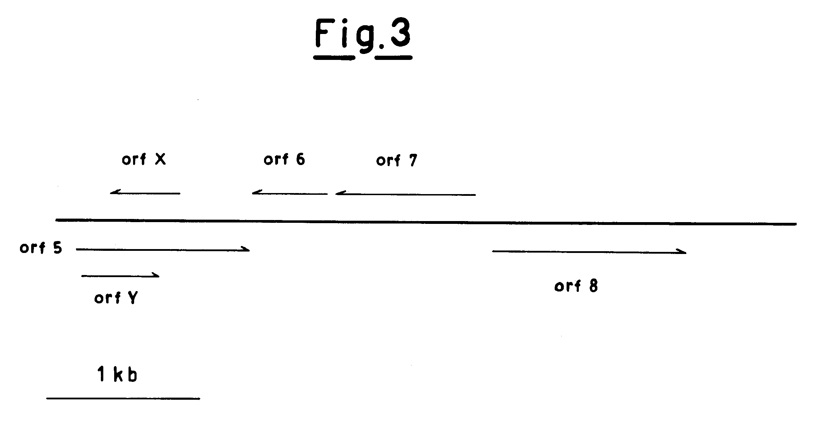

In accordance with the present invention that chromosomal DNA portion downstream of the ORF4 stop codon (Table 3 and Figure 3) was also sequenced.

Sequence analysis of this portion showed four regions (ORF5-ORF8) potentially coding for different products.

ORF5 comprises the nucleotides between positions 26262 and 27491 (Table 3) and codes for a protein of 408 amino acids with the sequence shown in Table 8.

TABLE 8

| | | | | | | | | | | | | | | | |
|---|---|---|---|---|---|---|---|---|---|---|---|---|---|---|---|
| | | | | | 5 | | | | | 10 | | | | | 15 |
| 1 | Met | Arg | Thr | Ser | Pro | Arg | Ser | Lys | Trp | Phe | Val | Leu | Leu | Phe | Thr |
| 16 | Phe | Val | Phe | Ala | Ile | Gly | Met | Asn | Ser | Phe | Arg | Asn | Ser | Phe | Gln |
| 31 | Phe | Phe | Met | Leu | Pro | Met | Ala | Asp | Ala | Phe | His | Ala | Asp | Arg | Ser |
| 46 | Leu | Ile | Ser | Val | Ser | Val | Ser | Ile | Phe | Met | Ile | Thr | Thr | Gly | Ile |
| 61 | Val | Gln | Phe | Phe | Val | Gly | Phe | Phe | Ile | Asp | Arg | Phe | Ser | Val | Arg |

76 Lys Ile Met Ala Leu Gly Ala Val Cys Ile Ser Ala Ser Phe Leu
91 Val Leu Pro Tyr Ser Pro Asn Val His Val Phe Ser Ala Ile Tyr
106 Gly Val Leu Gly Gly Ile Gly Tyr Ser Cys Ala Val Gly Val Thr
121 Thr Gln Tyr Phe Ile Ser Cys Trp Phe Asp Thr His Lys Gly Leu
136 Ala Leu Ala Ile Leu Thr Asn Ala Asn Ser Ala Gly Leu Leu Leu
151 Leu Ser Pro Ile Trp Ala Ala Ala Pro Tyr His Ala Gly Trp Gln
166 Ser Thr Tyr Thr Ile Leu Gly Ile Val Met Ala Ala Val Arg Leu
181 Pro Leu Leu Val Phe Gly Met Lys His Pro Pro His Ala Gln Ala
196 Glu Thr Val Lys Lys Ser Tyr Asp Trp Arg Gly Phe Trp Asn Val
211 Met Lys Gln Ser Arg Leu Ile His Ile Leu Tyr Phe Gly Val Phe
226 Thr Cys Gly Phe Thr Met Gly Ile Ile Asp Ala His Leu Val Pro
241 Ile Leu Lys Asp Ala His Val Ser His Val Asn Gly Met Met Ala
256 Ala Phe Gly Ala Phe Ile Ile Ile Gly Gly Leu Leu Ala Gly Trp
271 Leu Ser Asp Leu Leu Gly Ser Arg Ser Val Met Leu Ser Ile Leu
286 Phe Phe Ile Arg Leu Leu Ser Leu Ile Cys Leu Leu Ile Pro Ile
301 Leu Gly Ile His His Ser Asp Leu Trp Tyr Phe Gly Phe Ile Leu
316 Leu Phe Gly Leu Ser Tyr Thr Gly Val Ile Pro Leu Thr Ala Ala
331 Ser Ile Ser Glu Ser Tyr Gln Thr Gly Leu Ile Gly Ser Leu Leu
346 Gly Ile Asn Phe Phe Ile His Gln Val Ala Gly Ala Leu Ser Val
361 Tyr Ala Gly Gly Leu Phe Phe Asp Met Thr His Gly Tyr Leu Leu
376 Ile Val Ala Val Cys Ile Val Phe Val Gly Leu Ser Ala Val Ile
391 Glu Leu Val Pro Phe Leu Asp Lys Gln Lys Ala Lys Glu Thr His
406 His Ser Ile

ORF6 and ORF7 are contiguous and are potentially transcribed in the opposite direction to srfA from a single promoter situated between ORF7 and ORF8.

ORF6 comprises the nucleotides between positions 27505 and 28063 and codes for a protein of 185 amino acids with the amino acid sequence shown in Table 9.

TABLE 9

| | | | | | | | | | | | | | | | |
|---|---|---|---|---|---|---|---|---|---|---|---|---|---|---|---|
| | | | | | 5 | | | | | 10 | | | | | 15 |
| 1 | Met | Ile | Gln | Tyr | Ala | Ser | Glu | Ser | Ile | Asn | Leu | Pro | Gly | Glu | Ile |
| 16 | Thr | Phe | Lys | Asp | Val | Arg | Glu | Ile | Phe | Phe | Tyr | Gln | Ile | Ala | Lys |
| 31 | Ile | Ser | Cys | Phe | Tyr | Phe | Leu | Leu | Phe | Cys | Ala | Ile | Phe | Ala | Ala |
| 46 | Val | Asn | Phe | Ile | Asn | Gly | Trp | Pro | Arg | Ile | Val | Tyr | Gly | Ser | Asp |
| 61 | Ala | Leu | Asn | Leu | Phe | Met | Asn | Ser | Met | Leu | Ile | Ile | Val | Met | Ser |
| 76 | Val | Leu | Phe | Thr | Leu | Leu | Leu | Leu | Leu | Leu | Leu | Tyr | Val | Lys | Phe |
| 91 | Ser | Arg | Ala | Tyr | Lys | Lys | Asn | Glu | Arg | Met | Lys | Ser | Lys | Arg | Thr |
| 106 | Tyr | Thr | Leu | Asn | Gln | Glu | Gly | Ile | Arg | Ile | Cys | Ser | Lys | Lys | Tyr |
| 121 | Asp | Leu | Ile | Phe | Asn | Trp | Asp | Glu | Ile | Thr | Ala | Val | Phe | Glu | Tyr |
| 136 | Lys | Asn | Ile | Phe | Arg | Val | Asn | Thr | Ser | Ser | Gly | Gln | Tyr | Ile | Ala |
| 151 | Ile | Pro | Lys | His | Phe | Phe | His | Ser | Glu | Glu | Glu | Met | Asn | Arg | Phe |
| 166 | Lys | Glu | Ile | Ile | Leu | Lys | Asn | Thr | Glu | Thr | Lys | Lys | Leu | Lys | Phe |
| 181 | Lys | Lys | Asp | Gln | His | | | | | | | | | | |

ORF7 is located between nucleotides 28117 and 29055 and codes for a protein of 312 amino acids with the sequence shown in Table 10.

TABLE 10

| | | | | | | | | | | | | | | | |
|---|---|---|---|---|---|---|---|---|---|---|---|---|---|---|---|
| | | | | | 5 | | | | | 10 | | | | | 15 |
| 1 | Met | Val | Lys | His | Gln | Gln | Thr | Pro | Ala | Tyr | Ile | Ala | Ala | Ile | Leu |
| 16 | Tyr | Ser | Phe | Ile | Ile | Gly | Leu | Ser | Phe | Leu | Phe | Val | Lys | Ile | Ala |
| 31 | Leu | Gln | Thr | Ala | Glu | Pro | Phe | Asp | Ile | Leu | Ala | His | Arg | Phe | Thr |
| 46 | Ile | Ala | Phe | Ala | Ala | Ala | Thr | Val | Pro | Ile | Leu | Phe | Gly | Trp | Val |
| 61 | Lys | Leu | Ser | Ile | Leu | Val | Lys | Asp | Val | Ile | Asp | Ile | Leu | Pro | Leu |
| 76 | Ala | Leu | Leu | Tyr | Pro | Ala | Leu | Phe | Phe | Ser | Phe | Gln | Ala | Phe | Gly |

| | | | | | | | | | | | | | | | |
|---|---|---|---|---|---|---|---|---|---|---|---|---|---|---|---|
| 81 | Leu | Val | Tyr | Ser | Ser | Ser | Ser | Glu | Ala | Gly | Ile | Ile | Gln | Ala | Ala |
| 106 | Ile | Pro | Ile | Phe | Thr | Met | Val | Phe | Ala | Ala | Tyr | Val | Leu | Lys | Glu |
| 121 | Arg | Pro | Thr | Trp | Thr | Gln | Lys | Gly | Phe | Thr | Val | Leu | Ser | Val | Ala |
| 136 | Gly | Val | Met | Phe | Ile | Phe | Val | Met | Lys | Gly | Val | Asp | Val | Glu | Ser |
| 151 | Ala | Ser | Leu | Lys | Gly | Ser | Leu | Leu | Ile | Leu | Leu | Ser | Ala | Leu | Ser |
| 166 | Ser | Ala | Met | Tyr | Asn | Thr | Ala | Ala | Arg | Lys | Met | Thr | Gln | Arg | Phe |
| 181 | Lys | Leu | Thr | Glu | Leu | Thr | Tyr | Ile | Met | Ser | Ala | Ile | Gly | Phe | Val |
| 196 | Val | Phe | Asn | Ala | Ile | Ala | Leu | Val | Arg | His | Gly | Ala | Ala | Gly | Thr |
| 211 | Val | Gly | Thr | Tyr | Phe | Leu | Pro | Phe | Arg | Glu | Pro | Gly | Phe | Val | Leu |
| 226 | Ala | Ile | Val | Tyr | Leu | Gly | Val | Leu | Ser | Ser | Leu | Val | Thr | Ser | Phe |
| 241 | Leu | Ser | Asn | Tyr | Thr | Leu | Ser | Arg | Ile | Glu | Ala | Phe | Lys | Met | Ser |
| 256 | Ala | Phe | Asn | His | Val | Ser | Thr | Ile | Val | Thr | Met | Ile | Ala | Gly | Phe |
| 271 | Val | Ile | Leu | Asn | Glu | Ser | Leu | Ala | Trp | Tyr | His | Leu | Ala | Gly | Ala |
| 286 | Val | Cys | Ile | Met | Ile | Gly | Val | Val | Gly | Ser | Asn | Ile | Asn | Leu | Glu |
| 301 | Lys | Lys | Thr | Lys | Arg | Pro | Gly | Met | Pro | Ala | Lys | Lys | | | |

Finally ORF8, the mRNA of which is transcribed in the opposite direction to ORF6 and ORF7, is located between nucleotides 29179 and 30513 of the sequence of Table 3. This ORF codes for a protein of 444 amino acids with the sequence shown in Table 11.

TABLE 11

| | | | | | | | | | | | | | | | |
|---|---|---|---|---|---|---|---|---|---|---|---|---|---|---|---|
| | | | | | 5 | | | | | 10 | | | | | 15 |
| 1 | Met | Glu | Lys | Tyr | Met | Ser | Leu | Leu | Thr | Arg | Ile | Glu | Glu | Met | Met |
| 16 | Gln | Ser | Thr | Ser | Tyr | Gln | Glu | Gly | Asp | Arg | Leu | Pro | Ser | Ile | Arg |
| 31 | Gln | Leu | Ser | Ala | Arg | Tyr | Gln | Val | Ser | Lys | Ser | Thr | Val | Ile | Arg |
| 46 | Ala | Leu | Gln | Glu | Leu | Glu | Lys | Arg | His | Leu | Ile | Tyr | Ser | Val | Pro |
| 61 | Lys | Ser | Gly | Tyr | Tyr | Ile | Val | Lys | Lys | Ser | Gly | Lys | Ser | Lys | Ser |
| 76 | Gly | Gln | Pro | Gly | Pro | Ile | Asp | Phe | Ala | Thr | Ser | Ala | Pro | Asp | Pro |

91 Asp Val Phe Pro Tyr Leu Asp Phe Gln His Cys Ile Asn Lys Ala
106 Ile Asp Thr Tyr Lys Asn Asp Leu Phe Ile Tyr Gly Thr Pro Lys
121 Gly Leu Pro Ser Leu Ile Arg Val Leu Arg Lys Leu Leu Ala Thr
136 Gln Gln Val Phe Ala Asp Glu Arg His Ile Phe Ile Thr Ser Gly
151 Val Gln Gln Ala Leu Ser Leu Leu Cys Ala Met Pro Phe Pro Asn
166 Gly Lys Glu Lys Ile Ala Ile Glu Gln Pro Gly Tyr His Leu Met
181 Val Glu Gln Leu Glu Thr Leu Gly Ile Pro Ala Ile Gly Val Lys
196 Arg Thr Glu Glu Gly Leu Asp Ile Ala Lys Val Glu Arg Leu Phe
211 Gln Thr Glu Ser Ile Lys Phe Phe Tyr Thr Met Pro Arg Phe His
223 Asn Pro Leu Gly Cys Ser Leu Ser Glu Arg Asp Lys Gln Glu Leu
241 Val Arg Leu Ala Glu Ala Tyr Asp Val Tyr Leu Val Glu Asp Asp
256 Tyr Leu Gly Asp Leu Glu Glu Asn Lys Lys Ala Asp Pro Leu Tyr
271 Ala Tyr Asp Leu Ser Ser His Val Ile Tyr Leu Lys Ser Phe Ser
286 Lys Met Met Phe Pro Gly Leu Arg Val Gly Ala Ala Val Leu Pro
301 Glu Ala Leu Thr Asp Thr Phe Tyr Ala Tyr Lys Lys Leu Asn Asp
316 Ile Asp Cys Ser Met Ile Ser Gln Ala Ala Leu Glu Ile Tyr Leu
331 Lys Ser Gly Met Tyr Gly Arg His Lys Glu Lys Ile Arg Asp Ser
346 Tyr Lys Glu Arg Ser Leu Arg Leu His Gln Ala Ile Arg Thr His
361 Arg Gln Leu Gly Ser Gly Arg Phe Thr Phe Ser Ser Gly Gln Ala
376 Pro Cys Met His Thr His Leu Val Leu Pro Gln Asp Leu Pro Ala
391 Ser Arg Val Ile His Arg Leu Glu Lys Gln Gly Val Leu Leu Glu
406 Ala Ile Asp Arg His Tyr Leu Ser Asp Tyr Pro Lys Glu Asn Leu
421 Leu Lys Ile Asn Ile Ser Asn Val Lys Thr Glu Asp Ile Glu Arg
436 Gly Val Lys Leu Leu Met Ser His Leu

A further two potentially coding sequences were also identified in the region immediately downstream of ORF4, namely ORFx and ORFy.

ORFx, which is situated between nucleotides 26544 and 27011, is transcribed in the opposite direction to srfA.

This ORF codes for a protein of 155 amino acids with the sequence shown in table 12.

TABLE 12

| | | | | | 5 | | | | | 10 | | | | | 15 |
|---|---|---|---|---|---|---|---|---|---|---|---|---|---|---|---|
| 1 | Met | Arg | Asp | Met | Arg | Ile | Leu | Gln | Tyr | Arg | Asp | Glu | Val | Ser | Ile |
| 16 | Asn | Asn | Ser | His | Cys | Lys | Ser | Ala | Ser | Lys | His | Ala | Glu | Val | Gln |
| 31 | Asp | Met | Asn | Glu | Ala | Gly | Leu | Leu | His | His | Val | Pro | Lys | Pro | Ser |
| 46 | Pro | Ile | Ile | Arg | Phe | Phe | His | Ser | Phe | Arg | Leu | Arg | Met | Trp | Arg |
| 61 | Val | Leu | His | Pro | Lys | Asp | Glu | Glu | Arg | His | Glu | Asn | Ser | Arg | His |
| 76 | Asp | Asp | Ser | Gln | Asn | Gly | Ile | Gly | Ala | Leu | Pro | Ala | Gly | Met | Ile |
| 91 | Arg | Ser | Arg | Ser | Pro | Asn | Gly | Arg | Arg | Arg | Asp | Asp | Glu | Ala | Arg |
| 106 | Arg | Val | Gly | Ile | Gly | Gln | Asn | Ser | Lys | Arg | Gln | Thr | Phe | Val | Cys |
| 121 | Val | Lys | Pro | Thr | Ala | Asp | Glu | Ile | Leu | Gly | Arg | His | Ala | Asp | Arg |
| 136 | Ala | Gly | Ile | Pro | Asp | Ser | Ala | Lys | His | Thr | Val | Asn | Gly | Gly | Lys |
| 151 | His | Met | Asn | Ile | Arg | | | | | | | | | | |

ORFx presents homologies with some proteins pertaining to the transcription antiterminator class, in particular with B.subtilis sacY and with the protein regulating the transcription of levan sucrase in E.coli.

ORFy, which is situated between nucleotides 26285 ad 26650, is transcribed in the same direction as srfA and codes for a protein of 121 amino acids with the sequence shown in Table 13.

TABLE 13

| | | | | | 5 | | | | | 10 | | | | | 15 |
|---|---|---|---|---|---|---|---|---|---|---|---|---|---|---|---|
| 1 | Met | Val | Cys | Ile | Pro | Val | Tyr | Val | Cys | Phe | Arg | His | Arg | Asn | Glu |
| 16 | Leu | Ile | Gln | Lys | Phe | Leu | Ser | Ile | Phe | Tyr | Ala | Ala | Asn | Gly | Arg |
| 31 | Arg | Leu | Pro | Cys | Arg | Gln | Val | Ala | Asp | Phe | Gly | Phe | Cys | Gln | His |
| 46 | Phe | Tyr | Asp | His | Asn | Arg | His | Arg | Pro | Val | Phe | Cys | Arg | Phe | Phe |
| 61 | Tyr | Arg | Pro | Phe | Gln | Cys | Gln | Lys | Asn | Tyr | Gly | Ala | Gly | Ser | Cys |
| 76 | Leu | His | Gln | Arg | Lys | Leu | Phe | Gly | Ala | Ser | Leu | Phe | Thr | Glu | Cys |
| 91 | Ser | Cys | Val | Phe | Arg | His | Leu | Arg | Cys | Ala | Trp | Arg | Asn | Arg | Val |
| 106 | Phe | Leu | Arg | Gly | Arg | Arg | Asp | Asp | Pro | Val | Phe | His | Gln | Leu | Leu |
| 121 | Val | | | | | | | | | | | | | | |

The availability of these sequences and of the upstream sequence means that the srfA operon can be acted upon genetically to modify (i) the expression levels and/or (ii) the product characteristics by mutagenization of the surfactin amino acid sequence.

The expression of the surfactin or of at least one enzymatic protein of the surfactin synthetase complex or of fragments thereof can be obtained by cultivating in a culture medium containing a nitrogen source, a carbon source and microelements, a host micro-organism transformed with an expression vector containing the srfA operon or at least one coding sequence (ORF) of this operon or a region of this sequence.

Vectors suitable for the purpose can be chosen from plasmids, phages and cosmids available commercially or from collection centres.

The plasmids pSMSRF1, pSRSRF2, pSMSRF3, pSMSRF4, and lambda SRF5, which contain regions of the SRFA operon, were filed at the American type Culture Collection, where they have received the following espective filing numbers: ATCC 68632, ATCC 68633 ATCC 68634, ATCC 68635 and ATCC 75060.

The purpose of the following examples is merely to describe the present invention in greater detail and must in no way be interpreted as in any limiting the scope thereof.

EXAMPLE 1

Construction of surfactin producer strains by congression

Chromosomal DNA was extracted from the B.subtilis surfactin producer strain ATCC 21332 by the method described by Marmur (1961), J. molecular Biology 3: 208-218.

10 $\mu$g of DNA were then used to transform competent cells of the B.subtilis JH642 surfactin non-producer strain ($phe^-$, $trp^-$, $sfp^-$) (M.M. Nakano and P. Zuber (1989), J. Bacteriol., 171: 5347-5353) using the method described by Anagnostopoulos ad Spizizen ((1961), J. Bacteriol., 81: 741-746).

Transformant selection was made on plates of MT minimum medium [$(NH_4)_2SO_4$ 2 g/l, $K_2HPO_4$ 14 g/l, $kH_2PO_4$ 6 g/l, Na citrate.$2H_2O$ 1 g/l, $MgSO_4.7H_2O$ 0.2 g/l, pH 7.0] containing 4% glucose and all the amino acids required by the strain except for tryptophan (trp), to a final concentration of 25 $\mu$g/ml.

The resultant vital colonies, in which a extraneous fragment was incorporated, were transferred onto plates of TBAB medium containing defibrinated ram's blood (5% final) and assayed for the appearance of the hemolysis halo, which indicates surfactin production.

The bacterial colonies positive to screening have therefore co-transferred (following independent events of homologous recombination) the marker trp (which enables the cell to survive in a medium free of the amino acid tryptophan) and the unassociated marker which makes the cell able to produce surfactin.

Using this procedure, three B.subtilis surfactin producer strains derived from JH642 were isolated. One of these, known as JH642-4 $srf^+$, was subsequently characterised by genetic mapping.

EXAMPLE 2

characterisation of the strain JH642-4 $srf^+$ by genetic mapping

The object of the experiment was to locate the position of the DNA portion responsible for surfactin production on the B.subtilis JH642-4 $srf^+$ chromosome.

Genetic mapping was achieved by transduction with the PBS-1 bacteriophage (Hoch J.A. et al, (1967) J. Bacteriology, 93: 1925-1937).

a) Preparation of the PBS-1 lysate

The lysate of the PBS-1 phage was prepared on the strain JH642-4 $srf^+$.

20 ml of VY medium (25 g/l Veal infusion broth, 5 g/l yeast extract (DIFCO)) were inoculated with 0.5 ml of an overnight culture of the stated strains, the culture was followed until termination of the logarithmic growth phase, and then 1 ml was diluted in 9 ml of Y medium (NaCl 4 g/l, $K_2SO_4$ 5 g/l, $kH_2PO_4$ 1.5 g/l, $Na_2HPO_4$ 3 g/l, $MgSO_4.7H_2O$ 0.12 g/l, $CaCl_2$ 0.01 g/l, $FeCl_2$ 0.01 g/l, yeast extract 1 g/l, pH 7.0).

An aliquot (0.5 ml) of the bacterial suspension was infected with 1 ml of a $10^{-6}$ dilution of PBS-1 lysate in Y medium.

The mixture was incubated for 20 minutes at 37°C without agitation, 2 ml of TBAB soft agar were added (prepared by diluting TBAB in sterile water in a 65:40 w/v ratio), poured onto TBAB plates incubated overnight at 37°C.

The following morning 2 turbid plaques were withdrawn from the plate and inoculated into 40 ml of VY medium. After 2 hours of incubation at 37 °C with agitation, the culture was incubated at 37 °C overnight without agitation.

The lysate obtained in this manner was centrifuged for 10 minutes at 10,000 rpm. Dnasi (Worthington) and $MgCl_2$ were then added to the supernatant to a final concentration of 25 µg/ml and 0.05 M respectively. After 30 minutes of incubation at 37 °C the lysate was sterilized by filtration using Millipore 0.45 µm filters.

b) Transduction of B.subtilis PB5069 with the lysate PBS-1

The B.subtilis PB5069 strain (M. Perego and J. Hoch (1991), J. Bacteriol. 173: 2514-2520), auxotrophic by the markers aroI (requirement for aromatic amino acids), mtlB (inability to use mannitol as carbon source) and dalI (requirement for D-alanine) was transduced with the lysate PBS-1 prepared on the donor strain (D) JH642-4 $srf^+$.

In practice, a culture of the B.subtilis PB5069 receiving strain was inoculated into 10 ml of PY medium, and the growth followed under the microscope until the end of the logarithmic phase and the attainment of bacterial mobility.

Aliquots of the culture (0.5 ml) were then infected with 100 µl of the lysate PBS-1 prepared as under point a).

The phage was adsorbed onto the cells for 20 minutes at 37 °C.

After initial centrifuging (5 minutes at 7000 rpm), the cells were washed with 0.5 ml of MT minimum transformation medium, recentrifuged, resuspended in 100 µl of MT medium and selected by each of the auxotrophic markers.

This selection was effected by plating the cells on MT minimum medium containing 4% glucose and all the amino acids required by the strain except that for which the selection is made, to a final concentration of 25 µg/ml.

In transduction, the selection by the auxotrophic markers takes place as in the following table:

TABLE 1

| Marker | Nutritional requirement | Selective medium |
|---|---|---|
| aro1906 | trp, phe, tyr | MT + 0.5% glucose |
| mtlB1 | mtl | MT + 0.1% mtl |
| dal | D-ala | TBAB |

where:

aro1906 = requirement for aromatic amino acids
mtlB1 = inability to use mannitol as carbon source
dal = requirement for D-alanine
trp = tryptophan
phe = phenylalanine
tyr = tyrosine
mtl = mannitol
D-ala = D-alanine
MT = minimum transformation medium
TBAB = tryptose blood agar base

Surfactin production screening takes place on TBAB plates containing defibrinated ram's blood, 5% final.

After separate $aro^+$, $dal^+$ and $mtl^+$ selection, the transductant colonies obtained were analyzed for co-transfer of that DNA portion responsible for the production of surfactin and of the other auxotrophic markers ($aro^+$, $dal^+$ and $mtl^+$).

Data relative to the transduction experiments on the strain selecting for the three above markers are given below. The results obtained have been collected to show the recombination classes and the number of transductants obtained for each of them:

| dal$^+$ selection | |
|---|---|
| Recombination classes | No. transductants |
| dal$^+$ srf$^+$ mtl$^+$ | 50 |
| dal$^+$ srf$^+$ mtl$^-$ | 1 dal>srf 51% |
| dal$^+$ srf$^-$ mtl$^+$ | 31 dal>mtl 81% |
| dal$^+$ srf$^-$ mtl$^-$ | 18 |
| | 100 |

| aro$^+$ selection | |
|---|---|
| Recombination classes | No. transductants |
| aro$^+$ srf$^+$ mtl$^+$ | 13 |
| aro$^+$ srf$^+$ mtl$^-$ | 9 aro>srf 22% |
| aro$^+$ srf$^-$ mtl$^+$ | 7 aro>mtl 20% |
| aro$^+$ srf$^-$ mtl$^-$ | 71 |
| | 100 |

| mtl$^+$ selection | |
|---|---|
| Recombination classes | No. transductants |
| mtl$^+$ srf$^+$ dal$^+$ | 37 |
| mtl$^+$ srf$^+$ dal$^-$ | 18 mtl>srf 55% |
| mtl$^+$ srf$^-$ dal$^+$ | 26 mtl>dal 63% |
| mtl$^+$ srf$^-$ dal$^-$ | 19 |
| | 100 |

in accordance with the data obtained for the JH642-4 sfp$^+$ strain, it can be seen that the least frequent recombinant class (ie that requiring the largest number of cross-overs for it to occur) is the class (dal$^+$ srf$^+$ mtl$^-$), suggesting that the marker order on the chromosome has to be srf-mtl-dal.

To establish the distance of the markers (srf and mtl) from the selected marker (dal), the number of dal$^+$ srf$^+$ and dal$^+$ mtl$^+$ colonies are added respectively to obtain the co-transfer percentage, indicating in what percentage the marker srf or mtl is transferred simultaneously to the marker dal. The more frequently two markers are co-transferred, the closer they are on the chromosome.

The map distance is then obtained by subtracting the percentage co-transfer from 100%. In the described case the co-transfer percentages are:

dal ---> srf 51%

dal ---> mtl 81%

From which the following map distances are obtained:

dal ---> srf 49

dal ---> mtl 19

By proceeding in the same manner for the aro$^+$ and mtl$^+$ selections, the following genetic map was constructed:

```
aro            srf           mtl           dal

----------------------------------------------------------

:              80:            :    19       :
:--------------:--------------><-----------:
:    78        :      49      :             :
:--------------><---------------------------:
:              :      73      :             :
:<-------------:--------------:-------------:
:              :      75      :             :
:--------------:--------------:------------>:
:              :      45      :    37       :
:              <--------------:------------>:
:  62          :              :             :
:<-------------:--------------:             :
```

Even though the map distances measured with the three markers are not always congruent in absolute terms, it has however always been confirmed that the order in which srf, aro, dal and mtl are arranged on the chromosome is: aro – – – srf – – – mtl – – – dal.

This zone is at about 30° on the B.subtilis circular chromosome.

EXAMPLE 3

Cloning of the srfA operon

a) Construction of the PstI integration gene bank

The chromosomal DNA of B.subtilis JH642 was prepared by the method described by Marmur (–(1961), J. Molecular Biology 3: 208–218).

An aliquot (1 µg) of the chromosomal DNA was totally digested for 1 hour at 37°C with the restriction enzyme PstI (Biolabs).

On termination of the digestion, the DNA was purified with 1 volume of phenol, 1 volume of phenol/chloroform (1:1 v/v), and 1 volume of chloroform. The DNA was then precipitated with 2 volumes of ethanol (99%) in 0.5 M tris–HCl pH 8.0 for 10 minutes at –80°C. After centrifuging for 10 minutes at 12,000 rpm in a microcentrifuge (Eppendorf), the DNA was dried in a lyophilizer and then resuspended in 20 µl of sterile $H_2O$.

An aliquot (6 µl) of the suspension was then ligated in a ligation mixture with the integration pJM103 plasmid (M. Perego and J. Hoch (1991), J. Bacteriol. 173: 2514–2520), (200 ng), previously linearized with the restriction enzyme PstI.

The resultant ligation mixture, containing 1 unit of the enzyme T4–DNA ligase, was incubated for 16 hours at 14°C.

1 µl of a 1/10 dilution of said mixture was used for transforming 100 µl of competent cells of E.coli JM109 (BRL) (ampicillin–sensitive).

The transformants were plated on LB plates containing ampicillin (100 µg/ml), IPTG (isopropyl–β–D–thiogalactopyranoside) (0.5 mM) and X–Gal (5–bromo–4–chloro–3–indolyl–D–galactopyranoside) (20

μg/ml).

Transforming colonies with a white or pale blue coloration, indicating the insertion of a B.subtilis JH642 chromosomal DNA fragment into the vector pJM103 were transferred in groups of one hundred onto selective LB plates containing ampicillin (100 μg/ml), IPTG (0.5 mM) and X-Gal (20 μg/ml).

The plasmid DNA was extracted from each group of one hundred colonies by the fast extraction method described by Holmes and Quigley ((1981) Anal. Biochem., 114: 193-197).

in practice, the cells were collected from the plates, resuspended in 3 ml of LB medium (DIFCO) and then used (1.5 ml) for plasmid extraction. In this manner 22 samples of plasmid DNA were prepared for the PstI gene bank, each composed of plasmids present in 100 individual colonies.

The plasmid DNA was extracted from 10 individual colonies of the gene bank to check the insertion efficiency and the average dimensions of the cloned fragments.

About 60% of the tested colonies contained an insert of dimensions between 1500 and 4000 bp.

b) Analysis of the PstI gene bank

The gene bank was tested for inactivation of surfactin production.

The plasmid DNA extracted from the E.coli JM109 colonies was used to transform competent cells of B.subtilis JH642-4 $srf^+$.

The transformants were then selected on Shaeffer medium with 5 μg/ml of chloramphenicol added.

The transformants which were chloramphenicol-resistant ($cm^r$),indicating integration of the plasmid DNA, were replicated on selective TBAB plates of TBAB containing 5% defibrinated ram's blood and assayed for disappearance of the hemolysis halo.

Of 1200 colonies analyzed 23 were inactive in surfactin production.

To check the position of the marker $cm^r$ relative to the markers aroI and mtl1, the 23 colonies were mapped by genetic transduction as described in Example 2.

The chromosomal DNA extracted from these colonies was digested with PstI and analyzed by hybridization using the Southern method (T. Maniatis et al. "Molecular cloning: a laboratory manual" Cold spring Harbor laboratory 1982) and using as probe the integration vector pJM103 label led with $^{32}P$.

The colonies positive to hybridization were divided into six groups differing by dimensions and/or structure of the chromosomal DNA fragment which hybridized with the probe.

The six selected groups were known as P2, P8, P17, P26, P27, P29.

The chromosomal DNA was isolated from each of these clones, digested with EcoRI and then circularized in the presence of T4 DNA ligase.

An aliquot (1 μl) of the ligase mixtures was used for transforming competent cells of E.coli DH5α - (BRL).

The transformants were then selected on LB medium with added ampicillin.

The plasmids were isolated from the colonies which were ampicillin-resistant ($amp^r$), and consisted of a portion of the integrative starting plasmid JM103 and a region of the chromosomal DNA adjacent to the plasmid insertion site.

The plasmids p2E1, p8E9, p17E17, p26E25, p27E33 and p29E41 were obtained in this manner.

The dimensions of the chromosomal fragments (inserts) contained in each plasmid are given in Table 2.

TABLE 2

| | |
|---|---|
| P2E1 | 1.4 Kb |
| P8E9 | 1.7 Kb |
| P17E17 | 3.5 Kb |
| P26E25 | 3.0 Kb |
| P27E33 | 6.5 Kb |
| P29E41 | 3.1 Kb |

Subsequent analysis of the restriction and hybridization maps of each plasmid with the DNA of the other plasmids showed that the plasmids p2E1 and p26E25 contained part of the fragments carried by p17E17 and p27E33 respectively.

All these plasmids, if integrated into the chromosome of the B.subtilis JH642-4 $srf^+$ producer strain caused inactivation of surfactin production and in addition mapped in the region between aroI and mtlB.

c) Cloning of srfA by chromosome walking

As the fragments originating from the plasmids p8E9, p17E17. p27E33 and p29E41 do not represent the entire srfA operon, in order to extend the cloned gene zone mini-gene banks were constructed from chromosomal DNA of B.subtilis JH642-4 $srf^+$ by the known chromosome walking method (Strauss E.C. et al. (1987) Anal. Biochem. 154: 353).

The chromosomal DNA inserted into p27E33 was isolated by digestion with PstI and labelled radioactively with $^{32}P$ for use as the probe on the chromosomal DNA of the B.subtilis JH642-4 $srf^+$ producer strain previously digested with the enzyme Bg1II.

This experiment showed that the p27E33 insert hybridized with a chromosomal DNA and of about 8 Kb.

A mini-gene bank of Bg1II-Bg1II fragments of about 8 Kb was then created as follows:

10 $\mu$g of B.subtilis JH642-4 $srf^+$ DNA were digested with Bg1II as specified by the supplier. The digestion mixture was then loaded onto agarose gel, and after electrophoresis the fragments of about 8Kb dimensions were electroeluted and cloned in the pJM103 plasmid previously digested with BamHI. The BamHI and BglII extremities are cohesive.

After transforming competent cells of E.coli DH5$\alpha$ with the ligase mixture, the transformants were selected on LB medium with added ampicillin.

About 500 colonies of the mini-gene bank were analyzed by hybridization with the p27E33 insert. The pSRF3 plasmid containing the 5' end of the chromosomal DNA insert was isolated from one of the positive colonies (Figure 1).

Likewise, to obtain clones containing sequences upstream of pSRF3, a PstI-Bc1I fragment of about 500 bases, derived from the 5' end of pSRF3, was used as the probe to select a second mini-gene bank of Bc1I fragments of 1.7 Kb which had given a positive southern blot indication from chromosomal DNA with the same fragment used as probe.

This gene bank was constructed in the same manner as the first, starting from 10 $\mu$g of pJHC642-4 $srf^+$ DNA.

The pSRF2 plasmid was isolated by operating in this manner (Figure 1).

To obtain clones containing sequences upstream of pSRF2, a BclI-BglII fragment of 500 bp derived from the 5' end of pSRF2 was then used as probe to select a mini-gene bank of Bg1II fragments of 4.0 Kb which had given a positive southern blot indication from chromosomal DNA with the same fragment used as probe.

The resultant plasmid was known as pSRF1 (Figure 1).

The 3.5 Kb p17E17 insert was used to isolate the pSRF4 plasmid containing the 5.5 Kb EcoRI-NsiI fragment adjacent to pSRF3 (Figure 1).

Finally, the 1.7 Kb p8E9 insert was used as hybridization probe for analyzing a B.subtilis 168 (BGSC 1A1) gene bank constructed in the bacteriophage lambda FIXII (STRATAGENE, La Jolla, CA) by the method described by Maniatis et al. ("Molecular cloning: a laboratory manual", Cold Spring Harbor laboratory, 1982) using the E.coli LE392 strain (STRATAGENE, La Jolla, CA) as receiving strain.

Following this analysis it was possible to isolate a phage, given the name lambda SRF5, which from an analysis of the restriction map and comparison of the southern blot data was found to contain the srfA operon fragment from the nucleotide 6842 to the nucleotide 26125.

The pSRF1, pSRF2, pSRF3, pSRF4 and lambda SRF5 inserts were sequenced by the method of Sanger et al. (1977), PNAS, 74: 5463-5467) and using the sequence strategy described by Strauss et al. ((1986), Analytical biochem., 154: 353-360).

EXAMPLE 4

Sequence analysis of the srfA operon

Analysis of that sequence of the chromosomal zone identified genetically as essential for surfactin production showed four regions potentially coding for proteins (open reading frame - ORF), a zone upstream of the first ORE containing the srfA operon promoter (from -610 to +1) and a presumed terminator positioned downstream of the stop codon of the fourth ORE (Table 3).

A detailed analysis of the various sequenced regions will now be given.

a) Analysis of the ORF1 sequence

The first ORE (ORF1) is formed from 10767 bases (from nucleotide +1 to nucleotide 10767).

The sequence codes for a polypeptide of 3588 amino acids of molecular weight 402091 daltons having the amino acid sequence given in Table 4. The ATG start codon which encodes the first methionine is preceded by a typical ribosomal attachment sequence (Shine-Dalgarno) with a GGGAGG sequence 9 bases distant from the ATG codon.

The entire polypeptide amino acid sequence can be divided into three contiguous repeated regions (modules 1 to 3) with an internal homology of about 1000 bases, more marked in the second half of these regions (Figure 2).

At the C-terminal end of module 3 there is a region, module A (MOD A), of about 500 amino acids which has no homology with modules 1-3.

The second half of module 3 and the module A have high homology with the synthetases coded by TycA and GrsA (tyrocidin and gramicidin synthetase subunits I) (G. Mittenhuber et al. (1989), J. Bacteriol., 171: 4881-4887; J. Kratzschmar et al. (1989), J.
Bacteriol. 171: 5422-5429).

The sequence His-His-Val-Ile-Ser-Asp-Gly is repeated 4 times in the polypeptide coded by ORF1 in positions 139-145, 1184-1190, 2223-2229 and 3255-3261.

This sequence is highly conserved and shows homology with sequences found in enzymes, such as TycA and GrsA which catalyze amino acid activation.

The sequence Tyr-Thr-Ser-Gly-Tyr-Tyr-Gly-Arg-Pro-Lys-Gly is repeated three times in positions 606-616, 1653-1663 and 2698-2709.

Analogous homologies were found for the highly conserved sequence Gly-Arg-Glu-Asp-Asp-Gln-Val-Lys-Val-Arg-Gly-Tyr-Arg-Ile-Glu-Leu-Gly-Glu-Ile-Glu repeated three times in position 858-880, 1897-1919 and 2930-2949.

It is interesting to note the strong homology between ORF1 and the enzyme aminoadipyl cystein valine synthetase (ACV synthetase) which polymerizes and activates fatty acid with L-Cys and D-Val (D.J. Smith et al., (1990), EMBO J., 9: 2743-2750).

This enzyme also consists of three repeated units followed by a final region homologous with that of subunits I of the tyrocidin and gramacidin synthetase.

b) Analysis of the ORF2 sequence

The second ORF (ORF2) is formed from 10764 bases (from nucleotide 10780 to nucleotide 21543) (Table 3). This open reading phase is separated from the ORF1 stop codon by an untranslated intergenic region of 12 bases containing a Shine-dalgarno with an AGAGGT sequence 5 bases distant from the ORF2 ATG codon.

The ORF2 sequence codes for a polypeptide of 3587 amino acids with a molecular weight of 408000 daltons structured similarly to ORF1 (Table 5).

In this respect, the polypeptide contains three adjacent homology regions (modules 4-6) followed by a region, module B, of about 500 amino acids very similar to that of ORF1 and of gramicidin and tyrocidin synthetase I.

The first ORF2 module (MOD4) is also homologous to the first part of the sequences of the subunits II of tyrocidin and gramicidin synthetase.

As with ORF1, the sequences in the following positions are highly conserved:

- His-His-Val-Ile-Ser-Asp-Gly in positions 143-149, 1182-1188, 2225-2231, 3255-3261.
- Tyr-Thr-Ser-Gly-Tyr-Tyr-Gly-Arg-Pro-Lys-Gly in positions 613-623, 1644-1654, 2698-2708;
- Gly-Arg-Glu-Asp-Asp-Gln-Val-Lys-Val-Arg-Gly-Tyr-Arg-Ile-Glu-Leu-Gly-Glu-Ile-Glu in position 855-874, 1900-1919 e 2928-2947.

c) Analysis of the ORF3 sequence

The third ORF (ORF3) is formed from 3825 bases (from nucleotide 21580 to nucleotide 25404) (Table 3).

The intergenic region between ORF2 and ORF3 is of 36 bases and contains a Shine-Dalgarno with a GGGAG sequence 8 bases distant from the ATG codon of ORF3.

The sequence ORF3 codes for a polypeptide of 1274 amino acids with a molecular weight of 143816 daltons (Table 6).

Analysis of this polypeptide shows the presence of a region of 1008 amino acids (module 7) which is aligned for homology with modules 1–6 and contains the highly conserved sequences in the following positions:

- His–His–Val–Ile–Ser–Asp–Gly in position 146–152;
- Tyr–Thr–Ser–Gly–Tyr–Tyr–Gly–Arg–Pro–Lys–Gly in position 614–624;
- Gly–Arg–Glu–Asp–Asp–Gln–Val–Lys–Val–Arg–Gly–Tyr–Arg–Ile–Glu–Leu–Gly–Glu–Ile–Glu in position 858–877.

The last 243 amino acids of this polypeptide show no homology with modules 1–6 or modules A or B nor with known sequences of antibiotic peptide synthetases.

The nucleotide sequence from 24577 to 24591 shows homology with the consensus sequence of the catalytic site of thioesterase.

d) Analysis of the ORF4 sequence

The fourth ORF (ORF4), which is formed from 729 bases (from nucleotide 25433 to nucleotide 26161), is located 30 bases from the ORF3 stop codon (Table 3).

ORF4 codes for a protein of 242 amino acids with the amino acid sequence shown in Table 7.

In the intergenic region there is a Shine–Dalgarno AAAGGAGG sequence 9 bases distant from the ATG codon of ORF4.

By homology with the enzyme gramicidin synthetase, coded by the grst gene, a molecular weight of about 25 kilodaltons can be supposed.

It is interesting to note the presence of the sequence Gly–His–Ser–Met–Gly which is found in the active site of various bacterial and eukaryotic thioesterases. This resemblance and the sequence homologies with the grst gene indicate a probable thioesterase function for this subunit of the surfactin synthetase complex.

EXAMPLE 5

Cloning and sequencing of the region downstream of the srfA operon

Operating as in the preceding Examples 3 and 4, the zone downstream of the ORF4 stop codon was cloned and sequenced.

Analysis of the sequence of this portion shows four regions (ORF5–ORF8) potentially cloning for different products.

Specifically, ORF5 consists of the nucleotides between positions 21262 and 27491 (Table 3) and codes for a protein of 408 amino acids with the sequence shown in Table 8.

ORF6 and ORF7 are contiguous and transcribed in the opposite direction to srfA from a single promoter situated between ORF7 and ORF8.

ORF6 is located between nucleotides 27506 and 28063 and codes for a protein of 185 amino acids with the amino acid sequence shown in Table 9.

ORF7 is located between nucleotides 28119 and 20055 and codes for a protein of 312 amino acids with the sequence shown in Table 10.

Finally, ORF8, the mRNA of which is transcribed in the opposite direction to ORF6 and ORF7, is located between nucleotides 29181 and 30513 of the sequence of Table 3. This ORF codes for a protein of 444 amino acids with the sequence shown in Table 11.

A further two potentially coding sequences were also identified in the region immediately downstream of ORF4, namely ORFx and ORFy.

ORFx, which is located between nucleotides 26544 and 27011, is transcribed in the opposite direction to srfA. This ORF codes for a protein of 155 amino acids with the sequence shown in Table 12.

ORFx presents homologies with some proteins pertaining to the transcription antiterminator class, in particular with B.subtilis sacY and with a protein regulating the transcription of levan sucrase in E.coli.

OREy, which is situated between nucleotides 26285 and 26650, is transcribed in the same direction as srfA and codes for a protein of 121 amino acids with the sequence shown in Table 13.

These latter ORFs could be involved in the regulation and synthesis of surfactin.

## Claims

1. A Bacillus subtilis chromosomal DNA region comprising the srfA operon which encodes the multienzymatic complex surfactin synthetase, wherein said region is characterised by the nucleotide sequence shown in Table 3.

2. A DNA sequence comprising at least one coding sequence (ORF) of the Bacillus subtilis srfA operon which codes for an enzymatic protein of the multienzymatic complex surfacting synthetase.

3. A DNA sequence as claimed in claim 2, wherein the coding sequence is ORF 1 characterised by the nucleotide sequence shown in Table 3.

4. A DNA sequence as claimed in claim 3, wherein ORF1 codes for an enzymatic protein with the amino acid sequence shown in Table 4.

5. A DNA sequence as claimed in claim 2, wherein the coding sequence is ORF2 characterised by the nucleotide sequence shown in Table 3.

6. A DNA sequence as claimed in claim 5, wherein ORF2 codes for an enzymatic protein with the amino acid sequence shown in Table 5.

7. A DNA sequence as claimed in claim 2, wherein the coding sequence is ORF3 characterised by the nucleotide sequence shown in Table 3.

8. A DNA sequence as claimed in claim 7, wherein ORF3 codes for an enzymatic protein with the amino acid sequence shown in Table 6.

9. A DNA sequence as claimed in claim 2, wherein the coding sequence is ORF4 characterised by the nucleotide sequence shown in Table 3.

10. A DNA sequence as claimed in claim 9, wherein ORF4 codes for an enzymatic protein with the amino acid sequence shown in Table 7.

11. An expression cloning vector containing a DNA sequence in accordance with each of claims 1 to 10.

12. A micro-organism transformed by a vector in accordance with claim 11, wherein said micro-organism is chosen from prokaryotic or eukaryotic cells.

13. A method for producing surfactin comprising cultivating in a culture medium containing a nitrogen source, a carbon source and microelements a micro-organism transformed with an expression vector containing the Bacillus subtilis srfA operon which encodes the multienzymatic complex surfactin synthetase.

14. A method for producing at least one enzymatic protein of the multienzymatic complex surfactin synthetase or fragments thereof, comprising cultivating in a culture medium containing a nitrogen source, a carbon source and microelements a micro-organism transformed with an expression vector containing the DNA sequence comprising at least one ORF coding sequence of the Bacillus subtilis srfA operon or fragments thereof.

15. An enzymatic protein of the multienzymatic complex surfactin synthetase or fragments thereof.

16. The use of an enzymatic protein or fragments thereof in accordance with claim 15 for the in vitro or in vivo synthesis of peptides.

17. The plasmid vector pSMSRF1 ATCC 68632.

18. The plasmid vector pSMSRF2 ATCC 68633.

**19.** The plasmid vector pSMSRF3 ATCC 68634.

**20.** The plasmid vector pSMSRF4 ATCC 68635.

**21.** The phagic vector lambda SRF5 ATCC 75060.

Fig.1

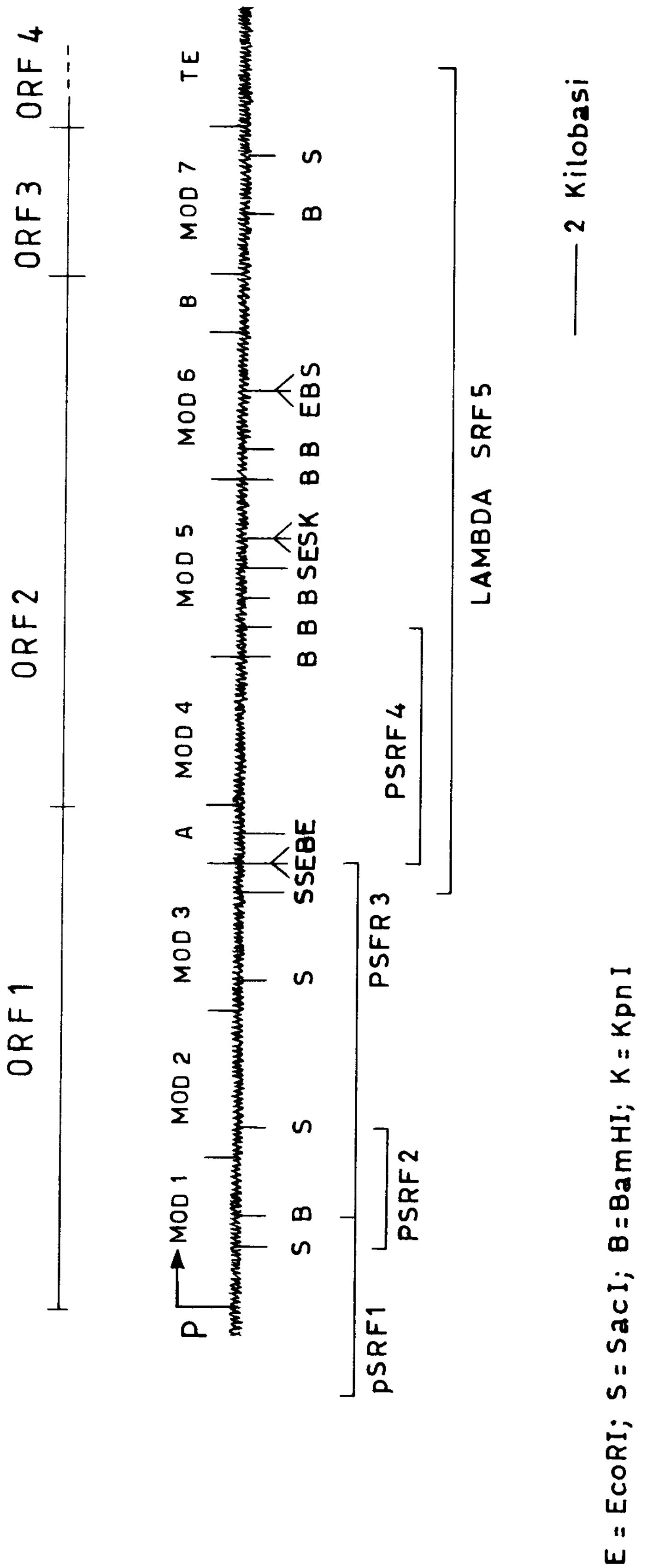
ORF 1
ORF 2
ORF 3
ORF 4
P
MOD 1
MOD 2
MOD 3
A
MOD 4
MOD 5
MOD 6
B
MOD 7
TE
S B
S
S
SSEBE
B B B B SESK
B B EBS
B S
pSRF1
PSRF2
PSFR3
PSRF4
LAMBDA SRF5
2 Kilobasi
E = EcoRI; S = SacI; B = BamHI; K = KpnI

# Fig.2/1

```
MOD1    MEITF---------------------------------------------     5
MOD2    KAMTLLTKIHKETGIEIPQQFLFEHPTITALA---EEADHRESKAFAVIE    47
MOD3    AGMKMPALVHQELGVELSLKDLFQSPTVEGLAQVI-ASAEKGTA--ASIS    47
MOD6    KGMMLIANIQAELEKSVPLKALFEQPTVRQLAAYMEASAVSGGH--QVLK    48
MOD5    KAMMMTAKIQEHFHKEVPIKVLFEKPTIQELALYLEENESKEEQTFEPIR    50
MOD4    MS----------------------KKSI----------------------     6
MOD7    MSQ-------------------FSKDQV----------------------     9
         .

MOD1    -------YPLTDAQKRIWYTEKFYPHTSISNLAGIGKLVSADAIDYVLVE    48
MOD2    PAEKQEHYPLHWHSS-----EHISSASSRMRESAI--HASSSNSEGFRYS    90
MOD3    PAEKQDTYPVSSPQKRMYVLQQLEDAQTSYNMPAV--LRLTGELDVERLN    95
MOD6    PADKQDMYPLSSAQKRMYVLNQLDRQTISYNMPSV--LLMEGELDI-WPA    95
MOD5    QASYQQHYPVSPAQRRMYILNQLGQANTSYNVPAV--LLLEGEVDKDRLE    98
MOD4    ----QKVYALTPMQEGMLYHAMLDPHSSSYSTQLE--LGIHAAFDLEIFE    50
MOD7    ----QDMYYLSPMQEGMLFHPILNPGQTFYLEQIT--MKVKGSLNIKCLE    63
               * .           .                  .. .    .

MOD1    QAIQEFIRRNDAMRL---RLRLDENGEPVQYISEYRPVDIKHTDTT--ED    93
MOD2    KAGARISGINPTPRVIETSFVLENST-PRQKIHVCVDFNIEMIERG---G   136
MOD3    SVMQQLMQRHEALR---TTFEIKDGE-TVQRIWEEAECEIAYFEAP---E   138
MOD6    RLTPQLVNRHESLR---TSFMEANGE-PVQRIIEKAEVDLHVFEAK---E   138
MOD5    NAIQQLINRHEILR---TSFDMIDGE-VVQTVHKNISFHLEAAKGR---E   141
MOD4    KSVNELIRSYDILRTV---FVHQQLQKPRQVVLAERKTKVHYEDISHADE    97
MOD7    ESMNVIMDRYDVFRTV---FIHEKVKRPVQVVLKKRQFHIEEIDLTHLTG   100
             .       *     .   .     * .      ..   .     .

MOD1    PNAIEFISQWSREETKKPLPLYDCDLFRFSLFTIKENEVWFYANVHHVIS   143
MOD2    RSDEAIMASF-----VRTFDLAKAPLFRIGLLGLEENRHMLLFDMHHLIS   181
MOD3    EETERIVSEF-----IKPFKIDQLPLFRIGLIKHSDTEQVLLFDMHHIIS   183
MOD6    DEADQKIKEF-----IRPFDLNDAPLIRAALLRIEAKKHLLLLDMHHIIA   183
MOD5    EDAEEIIKAF-----VQPFELNRAPLVRSKLVQLEEKRHLLLIDMHHIIT   186
MOD4    NRQKEHIERYKQQVQRQAFNLAKDILFKVAVFRLAADQLYLAWSNHHIMM   147
MOD7    SEQTAKINEYKEQDKIRGFDLTRDIPMRAAIFKKAEESFEWVWSYHHIIL   150
              .  .      . . .      .  .    ..      .**..
                                                    |-----

MOD1    DGMSMNIVGNAIMHIYLELASGSETKEGISHSFIDHVLSEQEYAQSKRFE   193
MOD2    DGVSIGIMLEELARIYKGEQLPDLRLQ-----YKDYAVWQSR-QAAEGYK   225
MOD3    DGASVGVLIEELSKLYDGETLEFLRIQ-----YKDYAVWQHRFIQSELYK   228
MOD6    DGVSRGIFVKELALLYKGEQLPEPTLH-----YKDFAVWQNEAEQKERMK   228
                 .        '
```

# Fig.2/2

```
MOO5    DGSSTGILIGDLAKIYQGADLELPQIH-----YKDYAVWHKE---QTNYQ    228
MOO4    DGWSMGVLMKSLFQNYEALRAGRTPANGQGKPYSDYIKW----LGKQONE    193
MOO7    DGWCFGIVVQDLFKVYNALREQKPYSLPPVKPYKDYIKW----LEKQDKQ    196
        **  .  ..    ..    * .                 . *

MOO1    KDKAFWNKQFESVPELVSLKRN--ASAGGSLDAERFSKDVPEALHQQILS    241
MOO2    KDQAYWKEVFAGELPVLQLLSDYPRPPVQSFEGDRVSIKLDAGVKDRLNR    275
MOO3    KQEEHWLKELDGELPVLTLPTDYSRPAVQTFEGDRIAFSLEAGKADALRR    278
MOO6    EHEAYWMSVLSGELPELDLPLDYARPPVQSFKGDTIRFRTGSETAKAVEK    278
MOO5    KDEEYWLDVFKGELPILDLPADFERPAERSFAGERVMFGLDKQITAQIKS    278
MOO4    EAESYWSERLAG-FEQPSVLPGRLPVKKDEYVNKEYSFTWDETLVARIQQ    242
MOO7    ASLRYWREYLEG-FEGQTTFAEQRKKQKDGYEPKELLFSLSEAETKAFTE    245
             * . . .          .          .   .

MOO1    FCEANKVSVLSVFQSLLAAYLYRVSGQNDVVTGTFMGNRQNAKE--KQML    289
MOO2    LAEQNGATLYMVMLSAYYTLLSKYTGQCDIIVGTPSAGRNHS--DTEGII    323
MOO3    LAKETDSTLYMVLLASYSAFLSKICGQDDIIVGSPVAGRSQA--DVSRVI    326
MOO6    LLAETGTTLHMVLHAVFHVFLSKISGQRDIVIGSVTAGRTNA--DVQDMP    326
MOO5    LMAETDTTMYMFLLAAFNVLLSKYASQDDIIVGSPTAGRTHP--DLQGVP    326
MOO4    TANLHQVTGPNLFQAVLGIVLSKYNFTDDVIFGTVVSGRPSEINGIETMA    292
MOO7    LAKSQHTTLSTALQAVWSVLISRYQQSGDLAFGTVVSGRPAEIKGVEHMV    295
                .    . .    . .       *.   *.  ..*      .

MOO1    GMFVSTVPLRTNIDGGQAFSEFVKDRMKDLMKTLRHQKYPYNLLINDLRE    339
MOO2    GMFVNTLAIRSEVKQNETFTQLISRVRKRVLDAFSHQDYPFEWLVEDLNI    373
MOO3    GMFVNTLALRTYPKGEKTFADYLNEVKETALSAFDAQDYPLEDLIGNVQV    376
MOO6    GMFVNTLALRMEAKEQQTFAELLELAKQTNLSALEHQEYPFEDLVNQLDL    376
MOO5    GMFVNTGALRTAPAGDKTFAQFLEEVKTASLQAFEHQSYPLEELIEKLPL    376
MOO4    GLFINTIPVRVKVERDRAFADIFTAVQQHAVEAERYDYVPLYEIQKRSAL    342
MOO7    GLFINVVPRRVKLSEGITFNGLLKRLQEQSLQSEPHQYVPLYDIQSQAD-    344
        *.*... . *        .*.   .       . .  .  *   .

MOO1    TKSSLTK-LFTV--SLEYQVMQWQKEEDLAFLTEPIFSGSGLNDVSIHVK    386
MOO2    PRDVSRHPLFDTMFSLQNATEGIPAVGDLSL-SVQETN-FKIAKFDLTVQ    421
MOO3    QRDTSSNPLFDAVFSMQNANIKDLTMKGIQL-EPHPFE-RKTAKFDLTLT    424
MOO6    PRDMSRNPLFNVMVTTENFDKEELTLQNLSI-SPYEAH-QGTSKFDLTLG    424
MOO5    TRDTSRSPLFSVMFNMQNMEIPSLRLGDLKI-SSYSML-HHVAKFDLSLE    424
MOO4    DGNLLNHLVAFENYPLDQELENGSMEDRLGF-SIKVESAFEQTSFDFNLI    391
MOO7    QPKLIDHIIVFENYPLQDAKNEESSEN--GF-DMVDVHVFEKSNYDLNLM    391
          .    .     .              . .           .. .. .

MOO1    DRWDTGKLTIDFDYRTDLFSREEINMICERMITMLENALTHPEHTIDELT    436
MOO2    ARETDEGIEIDVDYSTKLFKQSTADRLLTHFARLLEDAAADPEKPISEYK    471
MOO3    ADETDGGLTFVLEYNTALFKQETIERWKQYWMELLDAVTGNPNQPLSSLS    474
MOO6    GFTDENGIGLQLEYATDLFAKETAEKWSEYVLRLLKAVADNPNQPLSSLL    474
MOO5    AVEREEDIGLSFDYATALFKDETIRRWSRHFVNIIKAAAANPNVRLSDVD    474
MOO4    VYPGKT-WTVKIKYNGAAFDSAFIERTAEHLTRMMEAAVDQPAAFVREYG    440
MOO7    ASPGDE-MLIKLAYNENVFDEAFILRLKSQLLTAIQQLIQNPDQPVSTIN    440
         ..        *  .  *   .    .        ...    .*.  . .
```

# Fig.2/3

```
MOD1      LISDAEKEKLLARAGGKSVSYRKDMTIPELFQEKAELLSDHPAVVFEDRT   486
MOD2      LLSEEEAASQIQQFNPGRTPYPKDKTIVQLFEEQAANTPDHTALQYEGES   521
MOD3      LVTETEKQALLEAWKGKALPVPTDKTVHQLFEETAQRHKDRPAVTYNGQS   524
MOD6      LVTETEKQALLEAWKGKALPVPTDKTVHQLFEETVQRHKDRPAVTYNGQS   524
MOD5      LLSSAETAALLEE---RHMTQITEATFAALFEKQAQQTPDHSAVKAGGNL   521
MOD4      LVGDEEQRQIVEVFNSTKAELPEGMAVHQVFEEQAKRTPASTAVVYEGTK   490
MOD7      LVDDREREFLLTGLNPP-AQAHETKPLTYWFKEAVNANPDAPALTYSGQT   489
          *... *    .           ..'.    *.. .    . .*.  ..

MOD1      LSYRTLHEQSARIANVLKQKGVGPDSPVAVLIERSERMITAIMGILKAGG   536
MOD2      LTYRELNERANRLARGILSLGAGEGRTAAVLCERSMDMIVSILAVLKSGS   571
MOD3      WTYGELNAKANRLARILMDCGISPDDRVGVLTKPSLEMSAAVLGVLKAGA   574
MOD6      WTYGELNAKANRLARILMDCGISPDDRVGVLTKPSLEMSAAVLGVLKAGA   574
MOD5      LTYRSLDEQANQLAHHLRAQGAGNEDIVAIVMDRSAEVMVSILGVMKAGA   571
MOD4      LTYRELNAAANRLARKLVEHGLQKGETAAIMNDRSVETVVGMLAVLKAGA   540
MOD7      LSYRELDEEANRIARRLQKHGAGKGSVVALYTKRSLELVIGILGVLKAGA   539
           .* .*.. ....*. .   *   .  ...  ..*     ......*.*.

MOD1      AYVAIDPGFPAERIQYILEDCGADFYLTESKVAAPEADAE------LIDL   580
MOD2      AYVPIDPEHPIQRMQHFFRDSGAKVLLTQRKLKALAEEAEFKGVIVLADE   621
MOD3      AFVPIDPDYPDQRIEYILQDSGAKL-LLKQE--GISVPDSYTGDVILLDG   621
MOD6      AFVPIDPDYPDQRIEYILQDSGAKL-LLKQE--GISVPDSYTGDVILLDG   621
MOD5      AFLPIDPDTPGERIRYSLEDSGAKFAVVNER--NMTAIGQYEGIIVSLDD   619
MOD4      AYVPLDPALPGDRLRFMAEDSSVRMVLIGNSYTGQAHQLQVPVLTLDIG-   589
MOD7      AYLPVDPKLPEDRISYMLADSAAACLLTHQEMKEQAAELPYTGTTLFIDD   589
          *....**  * .*.     *...   .      . .            .

MOD1      DQAI---------EEGAEESLNADVNARNLAYIIYTSGTTGRPKGVMIEHR   622
MOD2      ----------EESYHADARNLALPLDSAAMANLTYTSGTTGTPKGNIVTHA   662
MOD3      SRTILSLPLDENDEENPENPETAVTAENLAYMIYTSGTTGQPKGVMVEHH   671
MOD6      SRTILSLPLDENDEGNPE---TAVTAENLAYMIYTSGTTGQPKGVMVEDH   668
MOD5      GK----------WRNESKERPSSISGSRNLAYVIYTSGTTGKPKGVQIEHR   660
MOD4      ---------FEES--EAADNLNLPSAPSDLAYIMYTSGSTGKPKGVMIEHK   629
MOD7      QT-----RFE----EQASDPATAIDPNDPAYIMYTSGTTGKPKGNITTHA   630
                        .         .. . * . ****.** ***  ...
                               .
                                           |---------|

MOD1      QVHHLVESLQQTIYQSP---TQTLPMAFLPPFHFDASVKQIFASLLLGQT   669
MOD2      NILRTV---KETNYLS---ITEQDTILGLSNYVFDAFMFDMFGSLLNGAK   706
MOD3      ALVNLCFWHHDAFSMT-----AEDRSAKYAGFGFDASIWEMFPTWSIGAE   716
MOD6      ALVNLCFWDHDAFSMT-----AEDRSAKYAGFGFDASIWEMFPTWTIGAE   713
MOD5      NLTNYVSWFSEEAGLTKRRADGNDKTVLLSSYAFDLGYTCMFPVLLGGGE   710
MOD4      SILRLV---KNAGYVP---VTEEDAMAQTGAVSFDAGTFEVFGALLNGAA   673
MOD7      NIQGLV---KHVDYMA---FSDQDTFLSVSNYAFDAFTFDFYASMLNAAR   674
           .        .    .                 **     ..     .

MOD1      LYIVPKKTVTNGAALTAYYRKNSIEATDGTPAHLQMLAAAGDF--EGL--   715
MOD2      LVLIPKETVLDMARLSRVIERENISILMITTALFHLLVDLNP----ACLS   752
MOD3      LHVIEEAIRLDIVRLNDYFETNGVTITFLPTQLAEQFMELEN-------T   759
MOD6      LHVIDEAIRLDIVRLNDYFETNGVTITFLPTQLAEQFMELEN-------T   756
MOD5      LHIVQKETYTAPDEIAHYIKEHGITYIKLTPSLFHTIVNTASFAFDANFE   760
MOD4      LYPVKKRHVLDAKQFAAFLREQSITTMWLTSPLFNQLAAKD----AGMFG   719
MOD7      LIIADEHTLLDTERLTDLILQENVNVMFATTALFNLLTDAG----EDWMK   720
          *    .    .   ..     ....    .. . . . .
```

# Fig.2/4

```
MOD1   KLKHMLIGGEGLSSVVADKLLKLFKEAGTAPRLTNVYGPTETCVDASVMP   765
MOD2   TLRKIMFGGERASVEHVRKAL---QTVGKG-KLLHMYGPSESTVFATYHP   798
MOD3   SLRVLLTGGDKLKRAVKKPYT-----------LVNNYGPTENTVVATSAE   798
MOD6   SLRVLLTGGDKLKRAVKKPYT-----------LVNNYGPTENTVVATSAE   795
MOD5   SLRLIVLGGEKSSRLMLSPSV---RCVWTT-EFINHYGPTEATIGAIAGR   805
MOD4   TLRHLIIGGD-ALVPHIVSKV---KQASPSLSLWNGYGPTENTTFSTSFL   755
MOD7   GLRCILFGGERASVPHVRKAL---RIMGPG-KLINCYGPTEGTVFATAHV   766
        *. .. **.                      . . ***.*. . .

MOD1   VIP-EMAVQSAYVPIGKALGNNRLYILDQKGRLQPEGVAGELYIAGDGVG   814
MOD2   VDELEEHTLS--VPIGKPVSNTEVYILDRTGHVQPAGIAGELCVTGEGLV   846
MOD3   IHPEEGSL-----SIGRAIANTRVYILGEGNQVQPEGVAGELCVAGRGLA   843
MOD6   IHPEEGSL-----SIGRAIANTRVYILGEGNQVQPEGVAGELCVAGRGLA   840
MOD5   VDLYEPDAFAKRPTIGRPIANAGALVLNEALKLVPPGASGQLYITGQGLA   856
MOD4   ID--REY--GGSIPIGKPIGNSTAYIMDEQQCLQPIGAPGELCVGGIGVA   811
MOD7   VHDLPDS--ISSLPIGKPISNASVYILNEQSQLQPFGAVGELCISGMGVS   814
       .            .**....*.   ...    . * *  *.*...* *..

MOD1   RGYLHLPELTEEKFLQDPFVPGDRMYRTGDVVRWLPDGTIEYLGREDDQV   864
MOD2   KGYYNRPELTEEKFVPHPFTSGERMYKTGDLARWLPNGTIEFIGRIDHQV   896
MOD3   RGYLNREDETAKRFVADPFVPGERMYRTGDLVKWTGGG-IEYIGRIDQQV   892
MOD6   RGYLNREDETAKRFVADPFVPGERMYRTGDLVKWVNGG-IEYIGRIDQQV   889
MOD5   RGYLNRPQLTAERFVENPYSPGSLMYKTGDVVRRLSDGTLAFIGRADDQV   906
MOD4   RGYVNLPELTEKQFLEDPFRPGERIYRTGDLARWLPDGNIEFLGRIDNQV   861
MOD7   KGYVNRADLTKEKFIENPFKPGETLYRTGDLARWLPDGTIEYAGRIDDQV   864
       .** .  . * ..*. .*. .*. .*.***....  .* ... ** *.**

MOD1   KVRGYRIELGEIEAVIQQAPDVAKAVVLARPDEQGNLEVCAYVVQKPGSE   914
MOD2   KIRGQAIELGEIEHQLQTHDRVQESVVLAVDQGAGDKLLCAYYVGE--GD   944
MOD3   KVRGYRIELSEIEVQLAQLSEVQDAAVTAVKDKGGNTA-IAAYVTP--ES   939
MOD6   KVRGYRIELSEIEVQLAQLSEVEDRAVTRVKDKGGNTA-IAAYVTP--ET   936
MOD5   KIRGYRIELGEIETVMLSLSGIQEAVVLAVSEGGLQEL-CAYYTSD--QD   953
MOD4   KVRGFRIELGEIETKLNMAEHVTEAAVIIRKNKADENEICAYFTAD--RE   909
MOD7   KIRGHRIELEEIEKQLQEYPGVKDAVVVADRHESGDASINAYLVNR--TQ   912
       *.**  ***.***  .     ...*      .   .    *  .

MOD1   FAPAGLREHAARQLPDYMVPAYFTEVTEIPLTPSGKVDRRKLFALEVKAV   964
MOD2   ISSQEMREHAAKDLPAYMVPAVFIQMDELPLTGNGKIDRRALPIPDANVS   994
MOD3   ADIEALKSALKETLPDYMIPAFWVTLNELPVTANGKVDRKALNEPDIEAG   989
MOD6   ADIEALKSTLKETLPDYMIPAFWVTLNELPVTANGKVDRKALPEPDIEAG   986
MOD5   IEKAELRYQLSLTLPSHMIPAFFVQVDAIPLTANGKTDRNALPKPNAAQS  1003
MOD4   VAVSELRKTLSQSLPDYMVPAHLIQMDSLPLTPNGKINKKELPAPQSEAV   959
MOD7   LSAEDVKAHLKKQLPAYMVPQTFTFLDELPLTTNGKVNKRLLPKPDQDQL   962
        .  ...      **..*.*.  . ....*.* .**.... *   .

MOD1   SGTAYTAPRNETEKAIAAIWQDVLNVEKAGIFDNFFETGGHSL----  1007
MOD2   RGVSYVAPRNGTEQKVADIWAQVLQAEQVGAYDHFFDIGGHSL----  1037
MOD3   SGE-YKAPTTDMEELLAGIWQDVLGMSEVGVTDNFFSLGGDSI----  1031
MOD6   SGE-YKAPTTDMEELLAGIWQDVLGMSEVGVTDNFFSLGGDS-----  1027
MOD5   GGKALAAPETALEESLCRIWQKTLGIEAIGIDDNFFDLGGHSL----  1046
MOD4   QPE-YAAPKTESEKKLAEIWEGILGVKA-GVTDNFFMIGGHSL----  1000
MOD7   AEE-WIGPRNEMEETIAQIWSEVLGRKQIGIHDDFFALGGHSLKAMT  1008
            ..* .. *. .  **  .*   . *  .* **-**.*
```

Consensus length: 1097
Identity   : 115 ( 10.5%)
Similarity: 250 ( 22.8%)

Fig.3

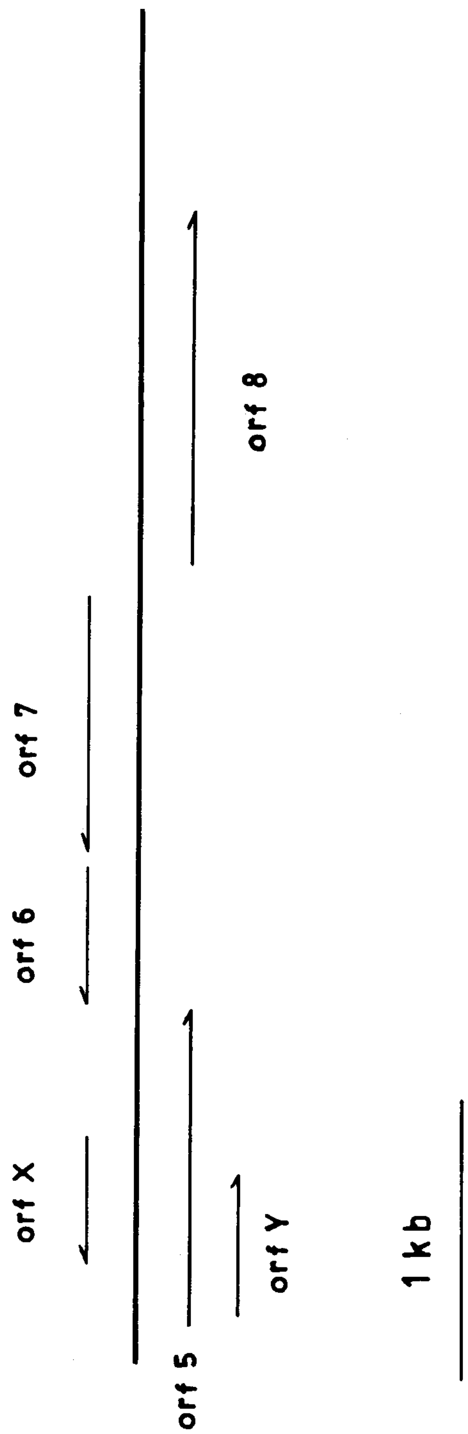

European Patent Office

# EUROPEAN SEARCH REPORT

Application Number

EP 92 20 3037
PAGE1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | JOURNAL OF BACTERIOLOGY vol. 173, no. 5, March 1991, BALTIMORE, US pages 1770 - 1778 M.M. NAKANO ET AL. 'srfA is an operon required for surfactin production, competence development, and efficient sporulation in Bacillus-subtilis' * the whole document * | 1-4, 11-16 | C12N15/52 C07K7/06 C12P21/04 // (C12N15/52, C12R1:125) |
| D,Y | --- | 5-10 | |
| Y | CRITICAL REVIEWS IN BIOTECHNOLOGY vol. 10, no. 3, 1990, pages 223 - 240 M.M. NAKANO ET AL. 'Molecular biology of antibiotic production in Bacillus' * the whole document * --- | 5-10 | |
| X | JOURNAL OF BACTERIOLOGY vol. 173, no. 17, September 1991, BALTIMORE, US pages 5487 - 5493 M.M. NAKANO ET AL. 'Transcription initiation region of the SrfA operon, which is controlled by the ComP-ComA signal transduction system in Bacillus-subtilis' * the whole document * --- | 1-4, 11-16 | TECHNICAL FIELDS SEARCHED (Int. Cl.5) C07K C12P C12N |
| X | BIOCHEMISTRY. vol. 30, no. 26, 1991, EASTON, PA US pages 6503 - 6508 C. ULLRICH ET AL. 'Cell-free biosynthesis of surfactin, a cyclic lipopeptide produced by Bacillus subtilis' * the whole document * --- -/-- | 15-16 | |

The present search report has been drawn up for all claims

| Place of search | Date of completion of the search | Examiner |
|---|---|---|
| BERLIN | 03 FEBRUARY 1993 | JULIA P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 92 20 3037
PAGE2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | FEBS LETTERS vol. 231, no. 1, 11 April 1988, AMSTERDAM, NL pages 107 - 110 B. KLUGE ET AL. 'Studies on the biosynthesis of surfactin, a lipopeptide antibiotic from Bacillus subtilis ATCC 21332' * the whole document * --- | 15-16 | |
| P,X | FEMS MICROBIOLOGY LETTERS vol. 92, no. 2, 15 April 1992, NL pages 175 - 180 S. BORCHERT ET AL. 'Identification of putative multifunctional peptide synthetase genes using highly conserved oligonucleotide sequences derived from known synthetases' * the whole document * ----- | 1-16 | |

TECHNICAL FIELDS SEARCHED (Int. Cl.5)

The present search report has been drawn up for all claims

| Place of search | Date of completion of the search | Examiner |
|---|---|---|
| BERLIN | 03 FEBRUARY 1993 | JULIA P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document